# EUROPEAN PATENT APPLICATION

(11) **EP 4 446 307 A1**
(43) Date of publication of application: **16.10.2024**
(21) Application number: 22892084.9
(22) Date of filing: 11.11.2022
(51) Int. Cl.: C07C 219/08, C07K 5/062, A61K 31/223, A61P 1/06

(54) **DERIVATIVE OF ASPARTIC ACID AND USE THEREOF IN TREATMENT OF METABOLIC DISEASES SUCH AS HEPATIC FIBROSIS AND NON-ALCOHOLIC HEPATITIS**

(30) Priority: 12.11.2021 CN 202111341140; 13.06.2022 CN 202210661248
(71) Applicant: SHENZHEN JINGTAI TECHNOLOGY CO., LTD., Shenzhen Guangdong 518017 (CN)
(72) Inventor: GAO, Jie, SHENZHEN, Guangdong 518000 (CN); CHENG, Huimin, SHENZHEN, Guangdong 518000 (CN); CHENG, Jun, SHENZHEN, Guangdong 518000 (CN); ZHANG, Peiyu, SHENZHEN, Guangdong 518000 (CN)
(74) Representative: Kluin Patent
(86) International application number: PCT/CN2022/131311
(87) International publication number: WO 2023/083291

(57) **Abstract**

The present invention relates to compounds of Formula I and Formula II and the use thereof in the treatment of metabolic diseases such as liver fibrosis and non-alcoholic hepatitis. Specifically, provided are the compounds as shown, and pharmaceutically acceptable salts or esters, prodrug, stereoisomers, hydrates, solvates, crystalline forms and metabolite forms thereof, or any combination or mixture thereof in the preparation of a drug or reagent, the drug being used for at least one of the following: A-(L₁)ₓ-A' (Formula I); A₁-(L₁)ₓ-A₁' (Formula II).

## Description

### Technical Field

The present invention relates to the field of biomedicine, specifically to a derivative of aspartic acid and use thereof in the treatment of liver fibrosis, non-alcoholic hepatitis and other metabolic diseases.

### Background Art

Fibrosis can occur in a variety of tissues and organs, its main pathological changes are the increase of fibrous connective tissue, the decrease of parenchymal cells in organ tissues, and its continued progression can cause organ structural damage and functional decline, or even failure, thereby seriously threatening human health and life. Worldwide, tissue fibrosis is the main cause of disability and death in many diseases. According to relevant statistics of the United States, nearly 45% of patients who die from various diseases in the United States can be attributed to diseases associated with tissue fibrogenesis.

Liver fibrosis is especially common in patients with tissue fibrosis. It is a reversible pathological phenomenon in which fibrous connective tissue is excessively deposited in the liver tissue during the body's repair process after liver damage. There are many causes of liver fibrosis. Patients with various chronic viral liver diseases are at high risk of developing liver fibrosis and cirrhosis. Alcoholics or long-term drinkers develop fatty liver in the early stage and can develop liver fibrosis and liver cirrhosis in the later stage, while other fatty livers caused by non-alcoholic factors such as obesity can also develop into liver fibrosis and liver cirrhosis; in addition, repeated infection with schistosomiasis can easily cause portal liver fibrosis; chronic cholestasis can produce biliary liver fibrosis; hepatolenticular degeneration and hemoglobin deposition can produce metabolic liver fibrosis; various toxic substances can cause toxic liver fibrosis; people who like low-protein diets and fatty fried foods can develop malnutrition-induced liver fibrosis; patients with chronic congestive heart failure can develop cardiogenic liver fibrosis. In addition, liver fibrosis is also an important pathological diagnostic indicator of non-alcoholic fatty liver disease (NAFLD) and non-alcoholic steatohepatitis (NASH).

The mechanism of occurrence and development of liver fibrosis is very complex. Current research mainly focuses on the activation and transformation of hepatic stellate cells. The possible pathways are the activation of signal transduction pathways mediated by transforming growth factor beta (TGF-β), platelet-derived growth factor (PDGF), tumor necrosis factor α (TNF-α), etc., due to various chronic stimuli, and the activation of hepatic stellate cells by prostaglandin cyclooxygenase-2 (COX-2), diffuse extracellular matrix (ECM) and oxidative stress, so that they are converted into myofibroblasts and fibroblasts, resulting in increased secretion or decreased degradation of extracellular matrix, thereby forming liver fibrosis. Since the mechanism of occurrence and development of liver fibrosis is not yet clear, the development of drugs to treat liver fibrosis has been relatively slow.

Currently, in the clinical treatment of liver fibrosis or cirrhosis caused by viral hepatitis (mainly hepatitis B or hepatitis C), nucleoside (acid) analogues or interferons are mainly used for antiviral treatment. By inhibiting viral replication, the response of inflammatory factors is controlled, and the progression of liver fibrosis or cirrhosis is slowed down. However, there is no effective treatment for liver fibrosis or cirrhosis caused by other factors such as alcohol, metabolism, drugs, etc., to which auxiliary treatment with traditional Chinese medicine or ready-prepared Chinese medicines is mainly applied.

Through suppression of subtractive hybridization and bioinformatics methods, NS3TP1 (HCV nonstructural protein 3-transactivated protein 1, NS3TP1) was screened and cloned for the first time, which is also named ASNSD1 (asparagine synthetase domain containing 1, ASNSD1), has registration number AY11696 in GenBank, and locates on human chromosome 2q32.2. The total length of the coding sequence of this gene is 1,932 nucleotides, and the coding product consists of 643 amino acid residues. NS3TP1 is widely distributed in the body, mainly in hepatocytes and gallbladder gland epithelial cells in the liver. Janine Meienberg et al. found through MLPA analysis that complete COL3A1 (encoding collagen III, a major component of liver fibrosis extracellular matrix deposition) hemizygous deletion affects the expression of ASNSD1, indicating that the expression of ASNSD1 may interact with COL3A1. Therefore, the development of NS3TP1 modulators can be a direction for the development of new drugs for NAFLD.

### Contents of the present invention

The purpose of the present invention is to obtain a compound having regulatory effect on NS3TP1, and to obtain a compound that having therapeutic effect on liver fibrosis and non-alcoholic fatty liver disease. The inventors have discovered through creative research that the aspartic acid derivatives of the present invention have therapeutic effects on liver fibrosis and non-alcoholic fatty liver disease, and these molecules have good pharmaceutical properties (e.g., solubility, AUC and/or oral bioavailability and other properties) and safety.

To this end, in the first aspect of the present invention, the present invention provides a use of a compound represented by the following Formula I, or pharmaceutically acceptable salt, ester, prodrug, stereoisomer, hydrate, solvate, isotopic compound, crystalline form, metabolite form thereof, or any combination or mixture thereof, in the manufacture of a medicament or agent, and the medicament is used for at least one of the following:
1) treating, improving, or preventing a liver disease (e.g., NAFLD, NASH, liver fibrosis);
2) alleviating a mammalian liver fibrosis, liver steatosis, or inflammation;
3) inhibiting the activation of a stellate cell;
4) regulating the expression of NS3TP1;

A-(L₁)ₓ-A' (Formula I)

wherein, A is 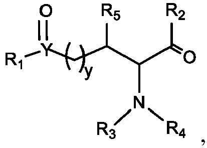 A' is 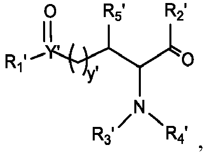 A and A' are the same or different;
wherein:
   x is selected from the group consisting of 0, 1 and 2;

1) when x is 0, A' is absent; for A, wherein:
   R₁ and R₂ are each independently selected from the group consisting of: C1-C6 alkyl, -O-R^{a1}, -NR^{a2}R^{a3}.
   R₃ and R₄ are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, -C(R^{h})₂-R^{d1}, -(CO)-(L)ₙ-R^{d2}, -(CO)O-(L)ₙ-R^{d3}, -(SO₂)-(L)ₙ-R^{d4}, -P(O)(OR^{e})₂, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), heterocyclyl;
   R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-O-(CO)-O-R^{b4}, -(C1-C6 alkyl)ₙ-(CO)-NR^{b5}R^{b6}, -(C1-C6 alkyl)ₙ-NR^{b7}-(CO)-R^{b8}, -(C1-C6 alkyl)ₙ-NR^{b9}-(CO)-O-R^{b10}, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
   R^{b1}, R^{b2}, R^{b3}, R^{b4}, R^{b5}, R^{b6}, R^{b7}, R^{b8}, R^{b9}, R^{b10} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-O-R^{c1}, -(C1-C6 alkyl)ₙ-O-(CO)-R^{c2}, -(C1-C6 alkyl)ₙ-O-(CO)-O-R^{c3}, the alkyl, cycloalkyl, aryl, heterocyclyl are optionally substituted with one or more groups independently selected from the group consisting of halogen, amino, hydroxyl, carboxyl, C1-C6 alkyl, C6-C10 aryl;
   R^{c1}, R^{c2}, and R^{c3} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, 3- to 15-membered heterocyclyl;
   L is each independently selected from the group consisting of: absence, O, NR^{d5}, C1-C6 alkyl, C2-C6 alkenyl, C2-C6 alkynyl;
   R^{e} is each independently selected from the group consisting of hydrogen, deuterium, Na, K, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl and heterocyclyl are optionally substituted with one or more groups independently selected from the group consisting of halogen, C1-C6 alkyl, halogenated C1-C6 alkyl;
   R^{h} is each independently selected from the group consisting of: C1-C6 alkyl, -NHC(O)R^{g1}, -OC(O)R^{g2}, -OP(O)(ONa)₂, -NHC(O)OR^{d6}, -OC(O)OR^{d7}, -OC(O)NHR^{d8};
   R^{d1}, R^{d2}, R^{d3}, R^{d4}, R^{d5}, R^{d6}, R^{d7}, R^{d8} are each independently selected from the group consisting of: hydrogen, deuterium, amino, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more R^{f} groups;
   R^{f} is each independently selected from the group consisting of halogen, C1-C6 alkyl, halogenated C1-C6 alkyl, C2-C6 alkenyl, C2-C6 alkynyl, C6-C10 aryl, halogenated C6-C10 aryl, C3-C7 cycloalkyl, 3- to 15-membered heterocyclyl, -NR^{g3}R^{g4}, -OR^{g5}, -NHC(O)R^{g6}, -OC(O)R^{g7};
   R^{g1}, R^{g2}, R^{g3}, R^{g4}, R^{g5}, R^{g6}, and R^{g7} are each independently selected from the group consisting of: hydrogen, C1-C6 alkyl, C2-C6 alkenyl, C2-C6 alkynyl, C3-C7 cycloalkyl, C6-C10 aryl, 3- to 15-membered heterocyclyl;
   R₅ is selected from the group consisting of: hydrogen, deuterium, C1-C6 alkyl, halogen, amino; or, R₅ is selected from the group consisting of hydrogen, amino;
   Y is C or S;
   y is an integer of 0 or above; or y is selected from the group consisting of 0, 1, 2, and 3; or y is 0 or 1;
   n is 0 or 1;
2) when x is 1 or 2, A and A' are the same or different, wherein:
   any one of R₁, R₂, R₃, and R₄ of a unit A is connected to any one of R₁', R₂', R₃', and R₄' of an adjacent unit A' through L₁;
   each L₁ is independently a unit-linking group selected from the group consisting of: absence, O, S, carbonyl, alkyl (or C1-C6 alkyl), alkenyl (or C2-C6 alkenyl), alkynyl (or C2-C6 alkynyl), -O-alkenyl-O- (or -O-C2-C6 alkenyl-O-), -O-alkynyl-O- (or -O-C2-C6 alkynyl-O-), cycloalkyl (or C3-C7 cycloalkyl), heteroalkyl (or 3- to 7-membered heteroalkyl; for example, -O-alkyl-O-(or -O-C1-C6 alkyl-O-)), wherein the alkyl, cycloalkyl, heteroalkyl are optionally substituted with one or more groups independently selected from the group consisting of Z;
   Z is independently selected from the group consisting of: halogen, amino, alkyl (or C1-C6 alkyl), alkenyl (or C2-C6 alkenyl), alkynyl (or C2-C6 alkynyl), haloalkyl (or halogenated C1-C6 alkyl), cycloalkyl (or C3-C7 cycloalkyl), aryl (or C6-C12 aryl), heterocyclyl;
   or, L₁ is selected from the group consisting of: absence, -CR'R", wherein R' and R" are each independently H, C1-C6 alkyl (or C1-C3 alkyl);
   or, L₁ is selected from the group consisting of: absence, -CH₂-, -CH(CH₃)-, -C-(CH₃)₂;
   for A, wherein:
      R₁ and R₂ are each independently selected from the group consisting of: C1-C6 alkyl, -O-, -O-R^{a1}, -NR^{a2}R^{a3};
      R₃ and R₄ are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, -C(R^{h})₂-R^{d1}, -(CO)-(L)ₙ-R^{d2}, -(CO)O-(L)ₙ-R^{d3}, -(SO₂)-(L)ₙ-R^{d4}, -P(O)(OR^{e})₂, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), heterocyclyl;
      R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-O-(CO)-O-R^{b4}, -(C1-C6 alkyl)ₙ-(CO)-NR^{b5}R^{b6}, -(C1-C6 alkyl)ₙ-NR^{b7}-(CO)-R^{b8}, -(C1-C6 alkyl)ₙ-NR^{b9}-(CO)-O-R^{b10}, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
      R^{b1}, R^{b2}, R^{b3}, R^{b4}, R^{b5}, R^{b6}, R^{b7}, R^{b8}, R^{b9}, R^{b10} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-O-R^{c1}, -(C1-C6 alkyl)ₙ-O-(CO)-R^{c2}, -(C1-C6 alkyl)ₙ-O-(CO)-O-R^{c3}, the alkyl, cycloalkyl, aryl, heterocyclyl are optionally substituted with one or more groups independently selected from the group consisting of halogen, amino, hydroxyl, carboxyl, C1-C6 alkyl, C6-C10 aryl;
      R^{c1}, R^{c2}, and R^{c3} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, 3- to 15-membered heterocyclyl;
      L is each independently selected from the group consisting of: absence, O, NR^{d5}, C1-C6 alkyl, C2-C6 alkenyl, C2-C6 alkynyl;
      R^{e} is each independently selected from the group consisting of hydrogen, deuterium, Na, K, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, and heterocyclyl are optionally substituted with one or more groups independently selected from the group consisting of halogen, C1-C6 alkyl, and halogenated C1-C6 alkyl;
      R^{h} is each independently selected from the group consisting of: C1-C6 alkyl, -NHC(O)R^{g1}, -OC(O)R^{g2}, -OP(O)(ONa)₂, -NHC(O)OR^{d6}, -OC(O)OR^{d7}, -OC(O)NHR^{d8};
      R^{d1}, R^{d2}, R^{d3}, R^{d4}, R^{d5}, R^{d6}, R^{d7}, R^{d8} are each independently selected from the group consisting of: hydrogen, deuterium, amino, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more R^{f} groups;
      R^{f} is each independently selected from the group consisting of halogen, C1-C6 alkyl, halogenated C1-C6 alkyl, C2-C6 alkenyl, C2-C6 alkynyl, C6-C10 aryl, halogenated C6-C10 aryl, C3-C7 cycloalkyl, 3- to 15-membered heterocyclyl, -NR^{g3}R^{g4}, -OR^{g5}, -NHC(O)R^{g6}, -OC(O)R^{g7};
      R^{g1}, R^{g2}, R^{g3}, R^{g4}, R^{g5}, R^{g6}, and R^{g7} are each independently selected from the group consisting of: hydrogen, C1-C6 alkyl, C2-C6 alkenyl, C2-C6 alkynyl, C3-C7 cycloalkyl, C6-C10 aryl, 3- to 15-membered heterocyclyl;
      R₅ is selected from the group consisting of: hydrogen, deuterium, C1-C6 alkyl, halogen, amino; or, R₅ is selected from the group consisting of hydrogen, amino;
      Y is C or S;
      y is an integer of 0 or above; or y is selected from the group consisting of 0, 1, 2, and 3; or y is 0 or 1;
      n is 0 or 1;
      for A', wherein:
         R₁' and R₂' are each independently selected from the group consisting of: C1-C6 alkyl, -O-, -O-R^{a1}, -NR^{a2}R^{a3}.
         R₃' and R₄' are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, -C(R^{h})₂-R^{d1}, -(CO)-(L)ₙ-R^{d2}, -(CO)O-(L)ₙ-R^{d3}, -(SO₂)-(L)ₙ-R^{d4}, -P(O)(OR^{e})₂, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), heterocyclyl;
         R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-O-(CO)-O-R^{b4}, -(C1-C6 alkyl)ₙ-(CO)-NR^{b5}R^{b6}, -(C1-C6 alkyl)ₙ-NR^{b7}-(CO)-R^{b8}, -(C1-C6 alkyl)ₙ-NR^{b9}-(CO)-O-R^{b10}, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
         R^{b1}, R^{b2}, R^{b3}, R^{b4}, R^{b5}, R^{b6}, R^{b7}, R^{b8}, R^{b9}, R^{b10} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-O-R^{c1}, -(C1-C6 alkyl)ₙ-O-(CO)-R^{c2}, -(C1-C6 alkyl)ₙ-O-(CO)-O-R^{c3}, the alkyl, cycloalkyl, aryl, heterocyclyl are optionally substituted with one or more groups independently selected from the group consisting of halogen, amino, hydroxyl, carboxyl, C1-C6 alkyl, C6-C10 aryl;
         R^{c1}, R^{c2}, and R^{c3} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, 3- to 15-membered heterocyclyl;
         L is each independently selected from the group consisting of: absence, O, NR^{d5}, C1-C6 alkyl, C2-C6 alkenyl, C2-C6 alkynyl;
         R^{e} is each independently selected from the group consisting of hydrogen, deuterium, Na, K, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, and heterocyclyl are optionally substituted with one or more groups independently selected from the group consisting of halogen, C1-C6 alkyl, and halogenated C1-C6 alkyl;
         R^{h} is each independently selected from the group consisting of: C1-C6 alkyl, -NHC(O)R^{g1}, -OC(O)R^{g2}, -OP(O)(ONa)₂, -NHC(O)OR^{d6}, -OC(O)OR^{d7}, -OC(O)NHR^{d8};
         R^{d1}, R^{d2}, R^{d3}, R^{d4}, R^{d5}, R^{d6}, R^{d7}, R^{d8} are each independently selected from the group consisting of: hydrogen, deuterium, amino, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more R^{f} groups;
         R^{f} is each independently selected from the group consisting of halogen, C1-C6 alkyl, halogenated C1-C6 alkyl, C2-C6 alkenyl, C2-C6 alkynyl, C6-C10 aryl, halogenated C6-C10 aryl, C3-C7 cycloalkyl, 3- to 15-membered heterocyclyl, -NR^{g3}R^{g4}, -OR^{g5}, -NHC(O)R^{g6}, -OC(O)R^{g7};
         R^{g1}, R^{g2}, R^{g3}, R^{g4}, R^{g5}, R^{g6}, and R^{g7} are each independently selected from the group consisting of: hydrogen, C1-C6 alkyl, C2-C6 alkenyl, C2-C6 alkynyl, C3-C7 cycloalkyl, C6-C10 aryl, 3- to 15-membered heterocyclyl;
         R₅' is selected from the group consisting of: hydrogen, deuterium, C1-C6 alkyl, halogen, amino; or, R₅ is selected from the group consisting of hydrogen and amino;
         Y' is C or S;
         y' is an integer of 0 and above; or y' is selected from the group consisting of 0, 1, 2, and 3; or y' is 0 or 1;
         n is 0 or 1;
         or, Formula I is:

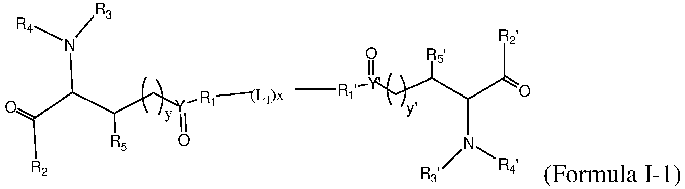

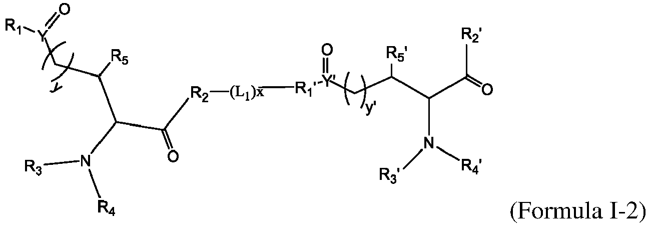

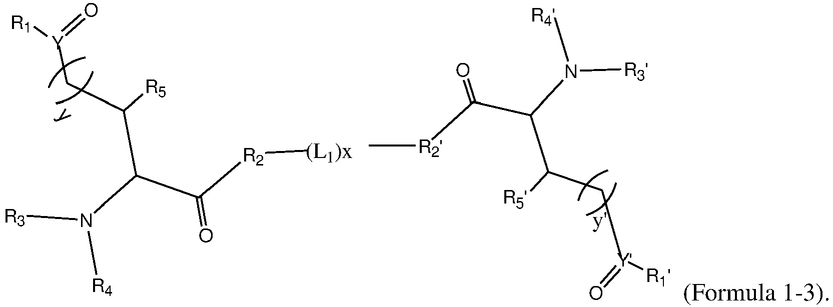

In some embodiments, in the above Formula I, when x is 0, A' is absent; for A, wherein:
R₁ and R₂ are each independently selected from the group consisting of:
C1-C6 alkyl,
-O-R^{a1}, wherein R^{a1} is selected from the group consisting of: hydrogen, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-O-(CO)-O-R^{b4}, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
-NR^{a2}R^{a3}, wherein R^{a2} and R^{a3} are each independently selected from the group consisting of: hydrogen, C1-C6 alkyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-O-(CO)-O-R^{b4}, -(C1-C6 alkyl)ₙ-(CO)-NR^{b5}R^{b6}, the alkyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
R^{b1}, R^{b2}, R^{b3}, R^{b4}, R^{b5} and R^{b6} are each independently selected from the group consisting of: hydrogen, hydroxyl, amino, C1-C6 alkyl, C3-C7 cycloalkyl, C6-C10 aryl, 3- to 15-membered heterocyclyl, -(C1-C6 alkyl)ₙ-(CO)-O-R^{c1}, -(C1-C6 alkyl)ₙ-O-(CO)-R^{c2}, -(C1-C6 alkyl)ₙ-O-(CO)-O-R^{c3}, the alkyl, cycloalkyl, aryl, heterocyclyl are optionally substituted with one or more groups independently selected from the group consisting of halogen, amino, hydroxyl, carboxyl, C1-C6 alkyl, C6-C10 aryl;
R^{c1}, R^{c2} and R^{c3} are each independently selected from the group consisting of: hydrogen, hydroxyl, amino, C1-C6 alkyl, C3-C7 cycloalkyl.

In some embodiments, in the above Formula I, when x is 0, A' is absent; for A, wherein:
R₁ and R₂ are each independently selected from the group consisting of:
hydroxyl,
C1-C6 alkyl,
-O-R^{a1}, wherein, R^{a1} is selected from the group consisting of: C1-C6 alkyl, C3-C7 cycloalkyl, C2-C6 alkenyl, -(C1-C6 alkyl)ₙ-O-(CO)-O-R^{b4}, which are optionally further substituted with one or more C6-C10 aryl groups, wherein R^{b4} is selected from the group consisting of C1-C6 alkyl, n is 1;
-NR^{a2}R^{a3}, wherein R^{a2} and R^{a3} are each independently selected from the group consisting of:
   hydrogen,
   C1-C6 alkyl, wherein C1-C6 alkyl is optionally further substituted with one or more C6-C10 aryl groups;
   -(C1-C6 alkyl)ₙ-O-(CO)-R^{b3}, wherein R^{b3} is C1-C6 alkyl, which is further substituted with C6-C10 aryl;
   -(C1-C6 alkyl)ₙ-(CO)-R^{b1}, wherein R^{b1} is hydroxyl, n or 1;
   -(C1-C6 alkyl)ₙ-(CO)-NR^{b5}R^{b6}, wherein n is 1, R^{b5} and R^{b6} are each independently hydrogen, or a 3- to 15-membered heterocyclyl, and the heterocyclic ring is further substituted with one or more C1-C6 alkyl groups;
   -(C1-C6 alkyl)ₙ-(CO)-O-R^{b2}, wherein the alkyl is substituted with one or more C6-C10 aryl groups, n is 1, R^{b2} is C1-C6 alkyl or -(C1-C6 alkyl)ₙ-O-(CO)-O-R^{c}, R^{c} is C1-C6 alkyl.

In some embodiments, in the above Formula I, when x is 0, A' is absent; for A, wherein:
R₃ and R₄ are each independently selected from the group consisting of: hydrogen, hydroxyl, C1-C6 alkyl, -(CO)-(L)ₙ-R^{d2}, -(CO)O-(L)ₙ-R^{d3}, the alkyl, aryl and heterocyclyl are optionally substituted with one or more groups independently selected from the group consisting of halogen, C1-C6 alkyl, halogenated C1-C6 alkyl, aryl (or C6-C12 aryl), and heterocyclyl;
each L is independently selected from the group consisting of: absence, O, NR^{d5}, C1-C6 alkyl;
R^{d5}, R^{d2} and R^{d3} are each independently selected from the group consisting of: hydrogen, amino, C1-C6 alkyl, C3-C7 cycloalkyl, C6-C10 aryl, 3- to 15-membered heterocyclyl, the alkyl, cycloalkyl, aryl, and heterocyclyl are optionally substituted with one or more R^{f} groups;
R^{f} is each independently selected from the group consisting of amino, halogen, C1-C6 alkyl, halogenated C1-C6 alkyl, C6-C10 aryl, halogenated C6-C10 aryl, C3-C7 cycloalkyl, 3- to 15-membered heterocyclyl.

In some embodiments, in the above Formula I, when x is 0, A' is absent; for A, wherein:
R₃ and R₄ are each independently selected from the group consisting of:
hydrogen,
hydroxyl,
C1-C6 alkyl,
-(CO)-(L)ₙ-R^{d2}: wherein, the combination option of (L)ₙ and R^{d2} is selected from the group consisting of the following combinations:
   1) L is absent, R^{d2} is C1-C6 alkyl that is further substituted with one or more R^{f} groups, R^{f} is amino;
   2) L is absent, R^{d2} is H;
   3) L is absent, R^{d2} is C1-C6 alkyl;
   4) L is absent, R^{d2} is C1-C6 alkyl that is further substituted with one or more R^{f} groups, R^{f} is selected from the group consisting of C1-C6 alkyl and amino;
   5) L is absent, R^{d2} is 3- to 15-membered heterocyclyl;
   6) L is NR^{d5}, wherein R^{d5} is C1-C6 alkyl, n is 1, R^{d2} is H;
-(CO)O-(L)ₙ-R^{d3}: wherein, the combination option of (L)ₙ and R^{d3} is selected from the group consisting of the following combinations: L is absent, R^{d3} is C1-C6 alkyl, which is optionally further substituted with one or more R^{f} groups, and R^{f} is selected from the group consisting of C1-C6 alkyl, C6-C10 aryl, polycyclic aromatic hydrocarbonyl (e.g., fluorenyl).

In some embodiments, in the above Formula I, when x is 1 or 2, A is the same as or different from A', wherein
for A, wherein:
R₁ and R₂ are each independently selected from the group consisting of: C1-C6 alkyl, -O-, -O-R^{a1}, -NR^{a2}R^{a3};
R₃ and R₄ are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
R₅ is selected from the group consisting of: hydrogen, deuterium, C1-C6 alkyl, halogen, amino; or, R₅ is selected from the group consisting of hydrogen, amino; or, R₅ is selected from the group consisting of hydrogen;
Y is C;
y is selected from the group consisting of 0, 1, 2, 3; or y is 0 or 1.

In some embodiments, in the above Formula I, when x is 1 or 2 (or x is 1), A is the same as or different from A', wherein
for A', wherein:
R₁' and R₂' are each independently selected from the group consisting of: C1-C6 alkyl, -O-, -O-R^{a1}, -NR^{a2}R^{a3};
R₃' and R₄' are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogen, C1-C6 alkyl, aryl (or C6-C12 aryl) or heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
R₅' is selected from the group consisting of: hydrogen, deuterium, C1-C6 alkyl, halogen, amino; or, R₅ is selected from the group consisting of hydrogen, amino; or, R₅ is selected from the group consisting of hydrogen;
Y' is C;
y' is selected from the group consisting of 0, 1, 2, 3; or y is 0 or 1.

In some embodiments, in the above Formula I, when x is 0, A' is absent; for A, wherein:
R₁ is selected from the group consisting of:
   C1-C6 alkyl,
   -O-R^{a1}, wherein, R^{a1} is selected from the group consisting of: hydrogen, C1-C6 alkyl, C2-C6 alkenyl, C3-C7 cycloalkyl, C6-C10 aryl, 3- to 15-membered heterocyclyl, -(C1-C6 alkyl)ₙ-O-(CO)-R^{b3}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
   -NR^{a2}R^{a3}, wherein, R^{a2} and R^{a3} are each independently selected from the group consisting of: hydrogen, C1-C6 alkyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-O-(CO)-R^{b3}, the alkyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl) -(3- to 15-membered heterocyclyl);
R^{b1} and R^{b3} are each independently selected from the group consisting of: hydrogen, hydroxyl, amino, C1-C6 alkyl, C6-C10 aryl, the alkyl, aryl, heterocyclyl are optionally substituted with one or more groups independently selected from the group consisting of halogen, amino, hydroxyl, carboxyl, C1-C6 alkyl, C6-C10 aryl.

In some embodiments, in the above Formula I, when x is 0, A' is absent; for A, wherein:
R₁ is selected from the group consisting of:
hydroxyl,
C1-C6 alkyl,
-O-R^{a}, wherein R^{a1} is selected from the group consisting of: C1-C6 alkyl, C3-C7 cycloalkyl, C2-C6 alkenyl, -(C1-C6 alkyl)ₙ-O-(CO)-O-R^{b4}, which are optionally further substituted with one or more C6-C10 aryl groups, wherein R^{b4} is selected from the group consisting of C1-C6 alkyl, n is 1;
-NR^{a2}R^{a3}, wherein R^{a2} and R^{a3} are each independently selected from the group consisting of: hydrogen; C1-C6 alkyl, which is optionally further substituted with one or more C6-C10 aryl groups; -(C1-C6 alkyl)ₙ-O-(CO)-R^{b3}, R^{b3} is C1-C6 alkyl, which is further substituted with C6-C10 aryl; -(C1-C6 alkyl)ₙ-(CO)-R^{b1}, R^{b1} is hydroxyl, n is 1.

In some embodiments, in the above Formula I, when x is 0, A' is absent; for A, wherein:
R₁ is selected from the group consisting of: hydroxyl, methoxy, -OCH₂CH=CH₂, ethoxy, -OC(CH₃)₃, -OCH₂C₆H₅, -OC₆H₁₁, Trt-NH-, 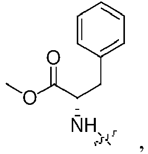 CH₃-NH-, HOCOCH₂NH-, -OCH(CH₃)₂, -CH₃, 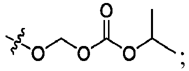 wherein, Trt represents trityl.

In some embodiments, in the above Formula I, when x is 0, A' is absent; for A, wherein:
R₂ is selected from the group consisting of:
C1-C6 alkyl,
-O-R^{a1}, wherein, R^{a1} is selected from the group consisting of: hydrogen, C1-C6 alkyl, C2-C6 alkenyl, C6-C10 aryl, -(C1-C6 alkyl)ₙ-O-(CO)-O-R^{b4}, and the alkyl, alkenyl, aryl are optionally substituted with one or more groups independently selected from the group consisting of halogen, amino, hydroxyl, C1-C6 alkyl, C6-C10 aryl, -(C1-C6 alkyl)-(C6-C10 aryl);
-NR^{a2}R^{a3}, wherein, R^{a2} and R^{a3} are each independently selected from the group consisting of: hydrogen, C1-C6 alkyl, -(C1-C6 alkyl)ₙ-(CO)-R^{b1}, -(C1-C6 alkyl)ₙ-(CO)-O-R^{b2}, -(C1-C6 alkyl)ₙ-(CO)-NR^{b5}R^{b6}, 3- to 15-membered heterocyclyl, the alkyl and heterocyclyl are optionally substituted with one or more groups independently selected from the group consisting of halogen, amino, hydroxyl, C1-C6 alkyl;
R^{b1}, R^{b2}, R^{b4}, R^{b5} and R^{b6} are each independently selected from the group consisting of: hydrogen, hydroxyl, C1-C6 alkyl, C3-C7 cycloalkyl, -(C1-C6 alkyl)ₙ-O-(CO)-O-R^{c3}, the cycloalkyl is optionally substituted with one or more groups independently selected from the group consisting of halogen, amino, hydroxyl, carboxyl, C1-C6 alkyl, C6-C10 aryl;
R^{c3} is each independently selected from the group consisting of: hydrogen, hydroxyl, amino, C1-C6 alkyl, C3-C7 cycloalkyl.

In some embodiments, in the above Formula I, when x is 0, A' is absent; for A, wherein:
R₂ is selected from the group consisting of:
hydroxyl,
-O-R^{a1}: wherein, R^{a1} is selected from the group consisting of: C1-C6 alkyl, C2-C6 alkenyl, -(C1-C6 alkyl)ₙ-O-(CO)-O-R^{b4}, which are optionally further substituted with one or more C6-C10 aryl groups, wherein R^{b4} is selected from the group consisting of C1-C6 alkyl, n is 1;
-NR^{a2}R^{a3}: R^{a2} and R^{a3} are each independently selected from the group consisting of: hydrogen; -(C1-C6 alkyl)ₙ-(CO)-NR^{b5}R^{b6}, n is 1, R^{b5} and R^{b6} are each independently hydrogen, or 3- to 15-membered heterocyclyl, the heterocyclyl is further substituted with one or more C1-C6 alkyl groups; -(C1-C6 alkyl)ₙ-(CO)-R^{b1}, n is 1, R^{b1} is hydroxyl; -(C1-C6 alkyl)ₙ-(CO)-O-R^{b2}, the alkyl is substituted with one or more C6-C10 aryl groups, n is 1, R^{b2} is C1-C6 alkyl or -(C1-C6 alkyl)ₙ-O-(CO) -O-R^{c3}, R^{c3} is C1-C6 alkyl.

In some embodiments, in the above Formula I, when x is 0, A' is absent; for A, wherein:
R₂ is selected from the group consisting of: hydroxyl, methoxy, -OCH₂CH=CH₂, ethoxy, -OC(CH₃)₃, -OCH₂C₆H₅, 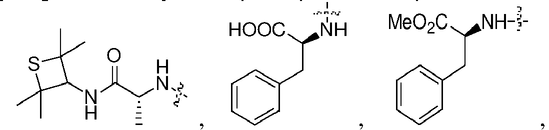 -OCH(CH₃)₂, 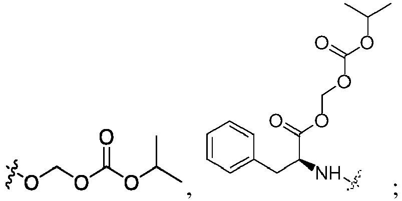
R₃ and R₄ are each independently selected from the group consisting of: hydrogen, hydroxyl, Boc, Cbz, -CH₃, Fmoc, -COOCH₂C₆H₅, -COCH₂NH₂, -CHO, -COCH₃, 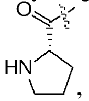 -COCH₂CH₃, -COCH(CH₃)₂, -COCH(CH₂)₂, -COOCH₃, -CON(CH₃)₂, -COCH(CH₃)(NH₂); wherein, Boc represents tert-butoxycarbonyl, Cbz represents benzyloxycarbonyl, and Fmoc represents fluorenylmethoxycarbonyl.

In some embodiments, in the above Formula I, when x is 1, A is the same as or different from A', wherein
for A, wherein:
R₁ is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
or, R₁ is selected from the group consisting of: -O-, hydroxyl, -O-R^{a}; R^{a} is C1-C6 alkyl;
or, R₁ is selected from the group consisting of: -O-, hydroxyl, methoxy, ethoxy, -OCH(CH₃)₂;
Y is C;
y is 0;
R₂ is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
or, R₂ is selected from the group consisting of: hydroxyl, -O-, -O-R^{a}: R^{a} is C1-C6 alkyl;
or, R₂ is selected from the group consisting of: hydroxyl, -O-, methoxy;
R₃ and R₄ are each independently selected from the group consisting of: hydrogen, hydroxyl, C1-C6 alkyl;
or, R₃ and R₄ are each independently hydrogen;
R₅ is selected from the group consisting of: hydrogen, deuterium, C1-C6 alkyl, halogen, amino; or, R₅ is selected from the group consisting of hydrogen, amino; or, R₅ is selected from the group consisting of hydrogen.

In some embodiments, in the above Formula I, when x is 1, A is the same as or different from A', wherein
for A', wherein:
R₁' is selected from the group consisting of: -O-, C1-C6 alkyl, -O-R^{a1}, wherein, R^{a1} is selected from the group consisting of: hydrogen, C1-C6 alkyl, C2-C6 alkenyl, C3-C7 cycloalkyl, C6-C10 aryl, 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
or, R₁ is selected from the group consisting of: -O-, hydroxyl, -O-R^{a}: R^{a} is C1-C6 alkyl;
or, R₁' is selected from the group consisting of: -O-, hydroxyl, methoxy, ethoxy, -OCH(CH₃)₂;
Y' is C;
y' is 0;
R₂' is selected from the group consisting of: -O-, C1-C6 alkyl, -O-R^{a1}, wherein, R^{a1} is selected from the group consisting of: hydrogen, C1-C6 alkyl, C2-C6 alkenyl, C3-C7 cycloalkyl, C6-C10 aryl, 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
or, R₂' is selected from the group consisting of: hydroxyl, -O-, -O-R^{a}: R^{a} is C1-C6 alkyl;
or, R₂' is selected from the group consisting of: hydroxyl, -O-, methoxy;
R₃' and R₄' are each independently selected from the group consisting of: hydrogen, hydroxyl, C1-C6 alkyl;
or, R₃' and R₄' are each independently hydrogen;
R₅' is selected from the group consisting of: hydrogen, deuterium, C1-C6 alkyl, halogen, and amino;
or, R₅' is selected from the group consisting of hydrogen and amino;
or, R₅' is selected from the group consisting of hydrogen.

In some embodiments, in the above Formula I, when x is 2, A is the same as or different from A', wherein
any one of R₁, R₂, R₃, and R₄ of a unit A is connected to any one of R₁', R₂', R₃', and R₄' of an adjacent unit A' through L₁;
each L₁ is independently a unit-linking group selected from the group consisting of: absence, O, S, carbonyl, alkyl (or C1-C6 alkyl), alkenyl (or C2-C6 alkenyl), alkynyl (or C2-C6 alkynyl), -O-alkenyl-O- (or -O-C2-C6 alkenyl-O-), -O-alkynyl-O- (or -O-C2-C6 alkynyl-O-), cycloalkyl (or C3-C7 cycloalkyl), heteroalkyl (or 3- to 7-membered heteroalkyl; for example, -O-alkyl-O-(or -O-C1-C6 alkyl-O-)), wherein the alkyl, cycloalkyl, heteroalkyl are optionally substituted with one or more groups independently selected from the group consisting of Z;
Z is independently selected from the group consisting of: halogen, amino, alkyl (or C1-C6 alkyl), alkenyl (or C2-C6 alkenyl), alkynyl (or C2-C6 alkynyl), haloalkyl (or halogenated C1-C6 alkyl), cycloalkyl (or C3-C7 cycloalkyl), aryl (or C6-C12 aryl), heterocyclyl;
or, L₁ is selected from the group consisting of: absence, -CR'R", wherein R' and R" are each independently H, C1-C6 alkyl (or C1-C3 alkyl);
or, L₁ is selected from the group consisting of: absence, -CH₂-, -CH(CH₃)-, -C-(CH₃)₂;
or, L₁ is selected from the group consisting of: -CH₂-.

In some embodiments, in the above Formula I, when x is 2, A is the same as or different from A', wherein
for A, wherein:
R₁ is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
or, R₁ is selected from the group consisting of: -O-, hydroxyl, -O-R^{a}: R^{a} is C1-C6 alkyl;
or, R₁ is selected from the group consisting of: -O-, hydroxyl, methoxy, ethoxy, -OCH(CH₃)₂;
or, R₁ is selected from the group consisting of: methoxy;
Y is C;
y is 0;
R₂ is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
or, R₂ is selected from the group consisting of: hydroxyl, -O-, -O-R^{a}: R^{a} is C1-C6 alkyl;
or, R₂ is selected from the group consisting of: hydroxyl, -O-, methoxy;
or, R₂ is selected from the group consisting of: -O-;
R₃ and R₄ are each independently selected from the group consisting of: hydrogen, hydroxyl, C1-C6 alkyl;
or, R₃ and R₄ are each independently hydrogen;
R₅ is selected from the group consisting of: hydrogen, deuterium, C1-C6 alkyl, halogen, amino; or, R₅ is selected from the group consisting of hydrogen, amino; or, R₅ is selected from the group consisting of hydrogen.

In some embodiments, in the above Formula I, when x is 2, A is the same as or different from A', wherein
For A', wherein:
R₁' is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
or, R₁ is selected from the group consisting of: -O-, hydroxyl, -O-R^{a}: R^{a} is C1-C6 alkyl;
or, R₁' is selected from the group consisting of: -O-, hydroxyl, methoxy, ethoxy, -OCH(CH₃)₂;
or, R₁' is selected from the group consisting of: methoxy;
Y' is C;
y' is 0;
R₂' is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
or, R₂' is selected from the group consisting of: hydroxyl, -O-, -O-R^{a}: R^{a} is C1-C6 alkyl;
or, R₂' is selected from the group consisting of: hydroxyl, -O-, methoxy;
more furthermore, R₂' is selected from the group consisting of: -O-;
R₃' and R₄' are each independently selected from the group consisting of: hydrogen, hydroxyl, C1-C6 alkyl;
or, R₃' and R₄' are each independently hydrogen;
R₅' is selected from the group consisting of: hydrogen, deuterium, C1-C6 alkyl, halogen, and amino;
or, R₅' is selected from the group consisting of hydrogen and amino;
or, R₅' is selected from the group consisting of hydrogen.

In some embodiments, the present invention provides a use of a compound represented by the following Formula I, or pharmaceutically acceptable salt, ester, prodrug, stereoisomer, hydrate, solvate, isotopic compound, crystalline form, metabolite form thereof, or any combination or mixture thereof, in the manufacture of a medicament or agent, and the medicament is used for at least one of the following:
1) treating, improving, or preventing a liver disease (e.g., NAFLD, NASH, liver fibrosis);
2) alleviating a mammalian liver fibrosis, liver steatosis, or inflammation;
3) inhibiting the activation of a stellate cell;
4) regulating the expression of NS3TP1;

A-(L₁)ₓ-A' (Formula I)

wherein, A is 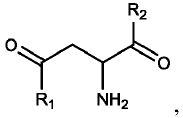 A' is 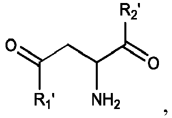 A and A' are the same or different;
wherein:
   x is selected from the group consisting of 0, 1, 2;
   1) when x is 0, A' is absent;
      for A, wherein:
      R₁ and R₂ are each independently selected from the group consisting of: -O-R^{a1}, -NR^{a2}R^{a3};
      R^{a1}, R^{a2}, and R^{a3} are each independently selected from the group consisting of: hydrogen, deuterium, C1-C3 alkyl, C2-C4 alkenyl, C2-C4 alkynyl, C3-C7 cycloalkyl, C6-C10 aryl, 3- to 15-membered heterocyclyl;
   2) when x is 1 or 2, A and A' are the same or different, wherein:
      any one of R₁ and R₂ of a unit A is connected to any one of R₁' and R₂' of an adjacent unit A' through L₁;
      each L₁ is independently a unit-connecting group selected from the group consisting of: absence, O, alkyl (or C1-C6 alkyl), alkenyl (or C2-C6 alkenyl), alkynyl (or C2-C6 alkynyl), -O-alkyl-O-(or -O-C1-C6 alkyl-O-), -O-alkenyl-O- (or -O-C2-C6 alkenyl-O-), -O-alkynyl-O- (or -O-C2-C6 alkynyl-O-);
      for A, wherein:
         R₁ and R₂ are each independently selected from the group consisting of: -O-R^{a1}, -NR^{a2}R^{a3};
         R^{a1}, R^{a2}, and R^{a3} are each independently selected from the group consisting of: hydrogen, deuterium, C1-C3 alkyl, C2-C4 alkenyl, C2-C4 alkynyl, C3-C7 cycloalkyl, C6-C10 aryl, 3- to 15-membered heterocyclyl;
         for A', wherein:
            R₁' and R₂' are each independently selected from the group consisting of: -O-R^{a1}, -NR^{a2}R^{a3};
            R^{a1}, R^{a2}, and R^{a3} are each independently selected from the group consisting of: hydrogen, deuterium, C1-C3 alkyl, C2-C4 alkenyl, C2-C4 alkynyl, C3-C7 cycloalkyl, C6-C10 aryl, 3- to 15-membered heterocyclyl.

In some embodiments, in the above Formula I, when x is 0, A' is absent; for A, wherein:
R₁ is each independently selected from the group consisting of: -O-R^{a1}, -NR^{a2}R^{a3};
R^{a1}, R^{a2}, and R^{a3} are each independently selected from the group consisting of: hydrogen, deuterium, C1-C3 alkyl, C2-C4 alkenyl, C2-C4 alkynyl, C3-C7 cycloalkyl, C6-C10 aryl, 3- to 15-membered heterocyclyl;
or, R₁ is each independently selected from the group consisting of: -OH, -O-(C1-C3 alkyl), -O-(C1-C3 alkenyl), -NH₂, -NH(C1-C3 alkyl), -N(C1-C3 alkyl)₂;
or, R₁ is each independently selected from the group consisting of: -OH, methoxy, ethoxy, -OCH(CH₃)₂, -NH₂;
R₂ is each independently selected from the group consisting of: -O-R^{a1}, -NR^{a2}R^{a3};
R^{a1}, R^{a2}, and R^{a3} are each independently selected from the group consisting of: hydrogen, deuterium, C1-C3 alkyl, C2-C4 alkenyl, C2-C4 alkynyl, C3-C7 cycloalkyl, C6-C10 aryl, 3- to 15-membered heterocyclyl;
or, R₂ is each independently selected from the group consisting of: -OH, -O-(C1-C3 alkyl), -O-(C1-C3 alkenyl), -NH₂, -NH(C1-C3 alkyl), -N(C1-C3 alkyl)₂;
or, R₂ is each independently selected from the group consisting of: -OH, methoxy, ethoxy, -OCH(CH₃)₂, -NH₂;

In some embodiments, in the above Formula I, when x is 1, A is the same as or different from A', wherein:
any one of R₁ and R₂ of a unit A is connected to any one of R₁' and R₂' of an adjacent unit A' through L₁;
each L₁ is independently a unit-connecting group selected from the group consisting of: absence, O, alkyl (or C1-C6 alkyl), alkenyl (or C2-C6 alkenyl), alkynyl (or C2-C6 alkynyl), -O-alkyl-O-(or -O-C1-C6 alkyl-O-), -O-alkenyl-O- (or -O-C2-C6 alkenyl-O-), -O-alkynyl-O- (or -O-C2-C6 alkynyl-O-);
or, L₁ is selected from the group consisting of: absence, alkyl (or C1-C3 alkyl), alkenyl (or C2-C3 alkenyl);
or, L₁ is selected from the group consisting of: absence, -CH₂-, -CH(CH₃)-, -C-(CH₃)₂;
or, L₁ is selected from the group consisting of: -CH₂-.

In some embodiments, in the above Formula I, when x is 1, A is the same as or different from A', wherein:
for A, wherein:
R₁ is selected from the group consisting of: -O-, -O-R^{a1}, wherein R^{a1} is selected from the group consisting of: hydrogen, C1-C3 alkyl, C2-C4 alkenyl, C3-C7 cycloalkyl, C6-C10 aryl, 3- to 15-membered heterocyclyl;
or, R₁ is selected from the group consisting of: -O-, hydroxyl, -O-(C1-C3 alkyl);
or, R₁ is selected from the group consisting of: -O-, hydroxyl, methoxy, ethoxy, -OCH(CH₃)₂;
R₂ is selected from the group consisting of: -O-, -O-R^{a1}, wherein R^{a1} is selected from the group consisting of: hydrogen, C1-C3 alkyl, C2-C4 alkenyl, C3-C7 cycloalkyl, C6-C10 aryl, 3- to 15-membered heterocyclyl;
or, R₂ is selected from the group consisting of: -O-, hydroxyl, -O-(C1-C3 alkyl);
or, R₂ is selected from the group consisting of: -O-, hydroxyl, methoxy, ethoxy, -OCH(CH₃)₂.

In some embodiments, in the above Formula I, when x is 1, A is the same as or different from A', wherein:
for A', wherein:
R₁ is selected from the group consisting of: -O-, -O-R^{a1}, wherein R^{a1} is selected from the group consisting of: hydrogen, C1-C3 alkyl, C2-C4 alkenyl, C3-C7 cycloalkyl, C6-C10 aryl, 3- to 15-membered heterocyclyl;
or, R₁ is selected from the group consisting of: -O-, hydroxyl, -O-(C1-C3 alkyl);
or, R₁ is selected from the group consisting of: -O-, hydroxyl, methoxy, ethoxy, -OCH(CH₃)₂;
R₂ is selected from the group consisting of: -O-, -O-R^{a1}, wherein R^{a1} is selected from the group consisting of: hydrogen, C1-C3 alkyl, C2-C4 alkenyl, C3-C7 cycloalkyl, C6-C10 aryl, 3- to 15-membered heterocyclyl;
or, R₂ is selected from the group consisting of: -O-, hydroxyl, -O-(C1-C3 alkyl);
or, R₂ is selected from the group consisting of: -O-, hydroxyl, methoxy, ethoxy, -OCH(CH₃)₂.

In some embodiments, in the above Formula I, when x is 2, A is the same as or different from A', wherein:
any one of R₁ and R₂ of a unit A is connected to any one of R₁' and R₂' of an adjacent unit A' through L₁;
each L₁ is independently a unit-connecting group selected from the group consisting of: absence, O, alkyl (or C1-C6 alkyl), alkenyl (or C2-C6 alkenyl), alkynyl (or C2-C6 alkynyl), -O-alkyl-O-(or -O-C1-C6 alkyl-O-), -O-alkenyl-O- (or -O-C2-C6 alkenyl-O-), -O-alkynyl-O- (or -O-C2-C6 alkynyl-O-);
or, L₁ is selected from the group consisting of: absence, alkyl (or C1-C3 alkyl), alkenyl (or C2-C3 alkenyl);
or, L₁ is selected from the group consisting of: absence, -CH₂-, -CH(CH₃)-, -C-(CH₃)₂;
or, L₁ is selected from the group consisting of: -CH₂-.

In some embodiments, in the above Formula I, when x is 2, A is the same as or different from A', wherein:
for A, wherein:
R₁ is selected from the group consisting of: -O-, -O-R^{a1}, wherein R^{a1} is selected from the group consisting of: hydrogen, C1-C3 alkyl, C2-C4 alkenyl, C3-C7 cycloalkyl, C6-C10 aryl, 3- to 15-membered heterocyclyl;
or, R₁ is selected from the group consisting of: -O-, hydroxyl, -O-(C1-C3 alkyl);
or, R₁ is selected from the group consisting of: -O-, hydroxyl, methoxy, ethoxy, -OCH(CH₃)₂;
R₂ is selected from the group consisting of: -O-, -O-R^{a1}, wherein R^{a1} is selected from the group consisting of: hydrogen, C1-C3 alkyl, C2-C4 alkenyl, C3-C7 cycloalkyl, C6-C10 aryl, 3- to 15-membered heterocyclyl;
or, R₂ is selected from the group consisting of: -O-, hydroxyl, -O-(C1-C3 alkyl);
or, R₂ is selected from the group consisting of: -O-, hydroxyl, methoxy, ethoxy, -OCH(CH₃)₂;

In some embodiments, in the above Formula I, when x is 2, A is the same as or different from A', wherein:
for A', wherein:
R₁ is selected from the group consisting of: -O-, -O-R^{a1}, wherein R^{a1} is selected from the group consisting of: hydrogen, C1-C3 alkyl, C2-C4 alkenyl, C3-C7 cycloalkyl, C6-C10 aryl, 3- to 15-membered heterocyclyl;
or, R₁ is selected from the group consisting of: -O-, hydroxyl, -O-(C1-C3 alkyl);
or, R₁ is selected from the group consisting of: -O-, hydroxyl, methoxy, ethoxy, -OCH(CH₃)₂;
R₂ is selected from the group consisting of: -O-, -O-R^{a1}, wherein R^{a1} is selected from the group consisting of: hydrogen, C1-C3 alkyl, C2-C4 alkenyl, C3-C7 cycloalkyl, C6-C10 aryl, 3- to 15-membered heterocyclyl;
or, R₂ is selected from the group consisting of: -O-, hydroxyl, -O-(C1-C3 alkyl);
or, R₂ is selected from the group consisting of: -O-, hydroxyl, methoxy, ethoxy, -OCH(CH₃)₂.

In the second aspect of the present invention, the present invention provides a use of a compound represented by the following Formula 1-4, or pharmaceutically acceptable salt, ester, prodrug, stereoisomer, hydrate, solvate, isotopic compound, crystalline form, metabolite form thereof, or any combination or mixture thereof, in the manufacture of a medicament or agent, and the medicament is used for at least one of the following:
1) treating, improving, or preventing a liver disease (e.g., NAFLD, NASH, liver fibrosis);
2) alleviating a mammalian liver fibrosis, liver steatosis, or inflammation;
3) inhibiting the activation of a stellate cell;
4) regulating the expression of NS3TP1; 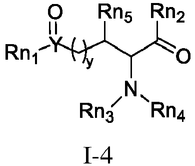
wherein:
Rn₁ and Rn₂ are each independently selected from the group consisting of: C1-C6 alkyl, -O-R^{a4}, -O-L₂-O-R^{a4}, -NR^{a5}R^{a6};
each R^{a4} is independently selected from the group consisting of: hydrogen, deuterium, C1-C6 alkyl, C2-C6 alkenyl, C2-C6 alkynyl, C3-C7 cycloalkyl, C3-C7 cycloalkyl-C1-C6 alkyl, C6-C10 aryl, C6-C10 aryl-C1-C6 alkyl, 3- to 15-membered heterocyclyl, 3- to 15-membered heterocyclyl-C1-C6 alkyl, C1-C6 alkoxyacyl, -(O=)C-CH₂-CH(NH₂)-COORₘ, -(O=)C-CH(NH₂)-CH₂-COORₘ, Ph-CH2-CH(NH2)-C(=O)-, NH₂-CH₂-C(=O)-, CH3-CH(NH2)-C(=O)-, Rₘ,OOC-(CH₂)₂-CH(NH₂)-C(=O)-, -C(=O)-(CH₂)₂-CH(NH₂)-COORₘ, RₘOOC-CH(NH₂)-CH₂-S-S-CH₂-CH(NH₂)-C=O-, H2N-CO-(CH2)2-CH(NH2)-C(=O)-, *N=CH-NH-CH=*C-CH₂-CH(NH₂)-C(=O)- (or 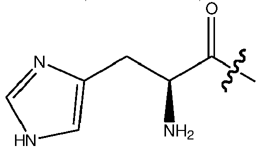 ), HO-p-Ph-CH₂-CH(NH₂)-C(=O)-, HO-CH₂-CH(NH₂)-C(=O)-, CH₃-S-(CH₂)₂-CH(NH₂)-C(=O)-, HN=C(NH₂)-NH-(CH₂)₃-CH(NH₂)-C(=O)-, (CH₃)₂CH-CH₂-CH(NH₂)-C(=O)-; wherein Ph represents phenyl, HO-p-Ph-CH₂-CH(NH₂)-C(=O)- represents p-hydroxybenzylaminomethylcarbonyl;
R^{a5} and R^{a6} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, 3- to 15-membered heterocyclylaminoacyl-C1-C6 alkyl, Ph-CH₂-CH(COORₘ)-, -CH₂-COORₘ, -CH(COORₘ)-CH₂-COORₘ, -CH(CH₃)-COORₘ, RₘOOC-(CH₂)₂-CH(COORₘ)-, -CH(COORₘ)-CH₂-S-S-CH₂-CH(NH₂)-COORₘ, H₂N-CO-(CH₂)₂-CH(COORₘ)-, *N=CH-NH-CH=*C-CH₂-CH(COORₘ)- (or 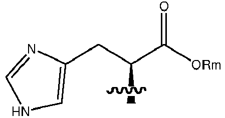 ), HO-p-Ph-CH₂-CH(COORₘ)-, HO-CH₂-CH(COORₘ)-, CH₃-S-(CH₂)₂-CH(COORₘ)-, HN=C(NH₂)-NH-(CH₂)₃-CH(COORₘ)-, (CH₃)₂CH-CH₂-CH(COORₘ)-;
each Rₘ is independently selected from the group consisting of: hydrogen, C1-C6 alkyl, C1-C6 alkoxyacyl-O-L₃-;
L₂ and L₃ are each independently selected from the group consisting of: C1-C6 alkyl, C2-C6 alkenyl, C2-C6 alkynyl, C3-C7 cycloalkyl, 3- to 7-membered heteroalkyl, L₂ and L₃ are optionally substituted with one or more groups independently selected from the group consisting of halogen, amino, and hydroxyl;
Rn₃ and Rn₄ are each independently selected from the group consisting of: hydrogen, deuterium, hydroxyl, H(C=O)-, C1-C6 alkyl, C2-C6 alkenyl, C2-C6 alkynyl, C3-C7 cycloalkyl, C6-C10 aryl, 3- to 15-membered heterocyclyl, aminoacyl, C1-C6 alkylacyl, C1-C6 alkoxyacyl, C1-C6 alkylaminoacyl, amino-C1-C6 alkanoyl, 3- to 15-membered heterocyclylacyl, C3-C7 cycloalkylacyl, C6-C10 aryl-C1-C6 alkoxyacyl;
Y is C or S;
y is 0, 1, 2 or 3.

In some embodiments, in the above Formula 1-4, Rn₁ and Rn₂ are each independently selected from the group consisting of: C1-C6 alkyl, -O-R^{a4}, -O-L₂-O-R^{a4}, -NR^{a5}R^{a6};
each R^{a4} is independently selected from the group consisting of: hydrogen, C1-C6 alkyl, C2-C6 alkenyl, C3-C7 cycloalkyl, C3-C7 cycloalkyl-C1-C6 alkyl, C6-C10 aryl, C6-C10 aryl-C1-C6 alkyl, 3- to 15-membered heterocyclyl, 3- to 15-membered heterocyclyl-C1-C6 alkyl, C1-C6 alkoxyacyl, -(O=)C-CH₂-CH(NH₂)-COORₘ, Ph-CH₂-CH(NH₂)-C(=O)-, NH₂-CH₂-C(=O)-, CH₃-CH(NH₂)-C(=O)-, RₘOOC-(CH₂)₂-CH(NH₂)-C(=O)-, RₘOOC-CH(NH₂)-CH₂-S-S-CH₂-CH(NH₂)-C(=O)-, H₂N-CO-(CH₂)₂-CH(NH₂)-C(=O)-, *N=CH-NH-CH=*C-CH₂-CH(NH₂)-C(=O)- (or 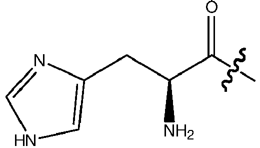 ), HO-p-Ph-CH₂-CH(NH₂)-C(=O)-, HO-CH₂-CH(NH₂)-C(=O)-, CH₃-S-(CH₂)₂-CH(NH₂)-C(=O)-, HN=C(NH₂)-NH-(CH₂)₃-CH(NH₂)-C(=O)-, (CH₃)₂CH-CH₂-CH(NH₂)-C(=O)-;
R^{a5} and R^{a6} are each independently selected from the group consisting of: hydrogen, C1-C6 alkyl, C2-C6 alkenyl, C3-C7 cycloalkyl, C6-C10 aryl, 3- to 15-membered heterocyclyl, 3- to 15-membered heterocyclylaminoacyl-C1-C6 alkyl, Ph-CH₂-CH(COORₘ)-, -CH₂-COORₘ, -CH(COORₘ)-CH₂-COORₘ, -CH(CH₃)-COORₘ, RₘOOC-(CH₂)₂-CH(COORₘ)-, -CH(COORₘ)-CH₂-S-S-CH₂-CH(NH₂)-COORₘ, H₂N-CO-(CH₂)₂-CH(COORₘ)-, *N=CH-NH-CH=*C-CH₂-CH(COORₘ)- (or 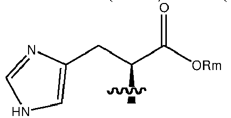 ), HO-p-Ph-CH₂-CH(COORₘ)-, HO-CH₂-CH(COORₘ)-, CH₃-S-(CH₂)₂-CH(COORₘ)-, HN=C(NH₂)-NH-(CH₂)₃-CH(COORₘ)-, (CH₃)₂CH-CH₂-CH(COORₘ)-;
each Rₘ is independently selected from the group consisting of: hydrogen, C1-C6 alkyl, C1-C6 alkoxyacyl-O-L₃-;
L₂ and L₃ are each independently selected from the group consisting of: C1-C6 alkyl, C2-C6 alkenyl, C3-C7 cycloalkyl, 3- to 7-membered heteroalkyl, L₂ and L₃ are each optionally substituted with one or more groups independently selected from the group consisting of halogen, amino, and hydroxyl;
Rn₃ and Rn₄ are each independently selected from the group consisting of: hydrogen, hydroxyl, H(C=O)-, C1-C6 alkyl, C2-C6 alkenyl, C3-C7 cycloalkyl, C6-C10 aryl, 3- to 15-membered heterocyclyl, aminoacyl, C1-C6 alkylacyl, C1-C6 alkoxyacyl, C1-C6 alkylaminoacyl, amino-C1-C6 alkanoyl, 3- to 15-membered heterocyclylacyl, C3-C7 cycloalkylacyl, C6-C10 aryl-C1-C6 alkoxy acyl;
Y is C;
y is 0, 1 or 2.

In some embodiments, in the above Formula 1-4, Rn₁ and Rn₂ are each independently selected from the group consisting of: C1-C6 alkyl, -O-R^{a4}, -O-L₂-O-R^{a4}, -NR^{a5}R^{a6};
each R^{a4} is independently selected from the group consisting of: hydrogen, C1-C6 alkyl, C2-C6 alkenyl, C3-C7 cycloalkyl, C6-C10 aryl, C6-C10 aryl-C1-C6 alkyl, 3- 15-membered heterocyclyl, 3- to 15-membered heterocyclyl-C1-C6 alkyl, -(O=)C-CH₂-CH(NH₂)-COORₘ, Ph-CH₂-CH(NH₂)-C(=O)-, NH₂-CH₂-C(=O)-, CH₃-CH(NH₂)-C(=O)-, RₘOOC-(CH₂)₂-CH(NH₂)-C(=O)-, RₘOOC-CH(NH₂)-CH₂-S-S-CH₂-CH(NH₂)-C(=O)-, H₂N-CO-(CH₂)₂-CH(NH₂)-C(=O)-, *N=CH-NH-CH=*C-CH₂-CH(NH₂)-C(=O)- (or 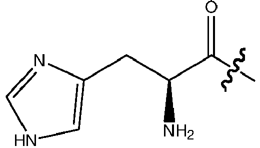 ), HO-p-Ph-CH₂-CH(NH₂)-C(=O)-, HO-CH₂-CH(NH₂)-C(=O)-, CH₃-S-(CH₂)₂-CH(NH₂)-C(=O)-, HN=C(NH₂)-NH-(CH₂)₃-CH(NH₂)-C(=O)-, (CH₃)₂CH-CH₂-CH(NH₂)-C(=O)-;
R^{a5} and R^{a6} are each independently selected from the group consisting of: hydrogen, C1-C6 alkyl, C3-C7 cycloalkyl, C6-C10 aryl, 3- to 15-membered heterocyclyl, 3- to 15-membered heterocyclylaminoacyl-C1-C6 alkyl, Ph-CH₂-CH(COORₘ)-, -CH₂-COORₘ, -CH(COORₘ)-CH₂-COORₘ, -CH(CH₃)-COORₘ, RₘOOC-(CH₂)₂-CH(COORₘ)-, -CH(COORₘ)-CH₂-S-S-CH₂-CH(NH₂)-COORₘ, H₂N-CO-(CH₂)₂-CH(COORₘ)-, *N=CH-NH-CH=*C-CH₂-CH(COORₘ)- (or 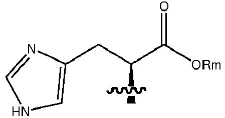 ), HO-p-Ph-CH₂-CH(COORₘ)-, HO-CH₂-CH(COORₘ)-, CH₃-S-(CH₂)₂-CH(COORₘ)-, HN=C(NH₂)-NH-(CH₂)₃-CH(COORₘ)-, (CH₃)₂CH-CH₂-CH(COORₘ)-;
each Rₘ is independently selected from the group consisting of: hydrogen, C1-C6 alkyl, C1-C6 alkoxyacyl-O-L₃-;
L₂ and L₃ are each independently selected from the group consisting of: C1-C6 alkyl, L₂ and L₃ are each optionally substituted with one or more groups independently selected from the group consisting of halogen, amino, and hydroxyl;
Rn₃ and Rn₄ are each independently selected from the group consisting of: hydrogen, hydroxyl, C1-C6 alkyl, C2-C6 alkenyl, C3-C7 cycloalkyl, C6-C10 aryl, 3- to 15-membered heterocyclyl, aminoacyl, C1-C6 alkylacyl, C1-C6 alkoxyacyl, C1-C6 alkylaminoacyl, amino-C1-C6 alkanoyl, 3- to 15-membered heterocyclylacyl, C3-C7 cycloalkylacyl, C6-C10 aryl-C1-C6 alkoxyacyl;
Y is C;
y is 0, 1 or 2.

In some embodiments of the first and second aspects of the present invention, the compound represented by Formula I or 1-1, 1-2, 1-3 or 1-4 is selected from the group consisting of the following:

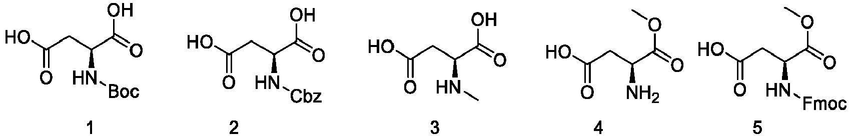

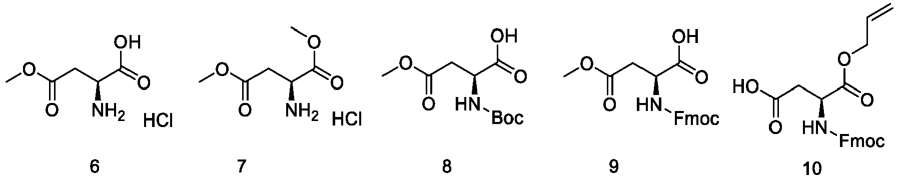

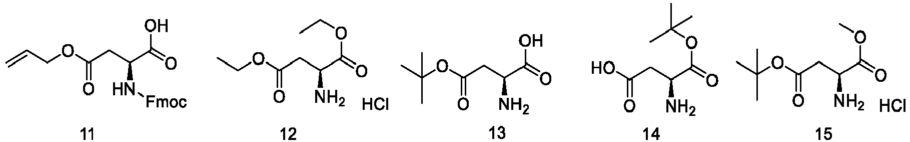

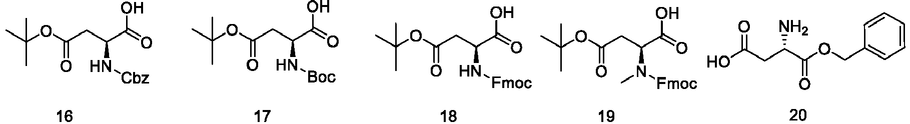

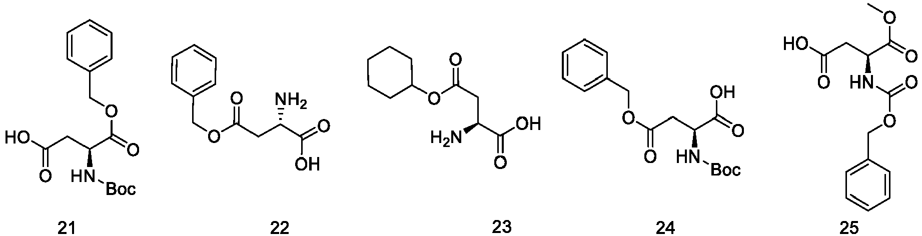

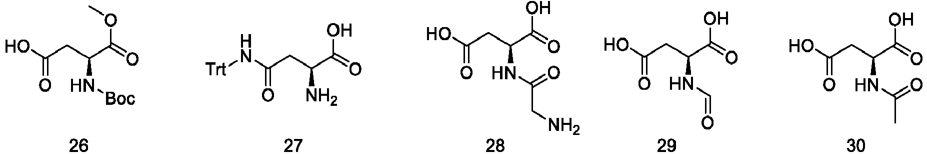

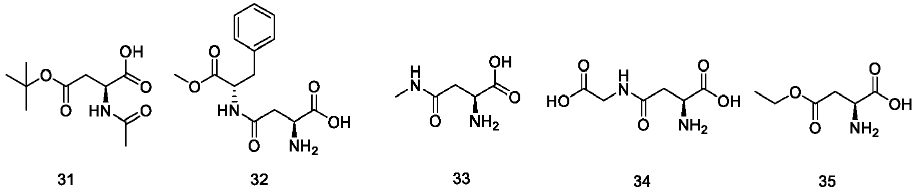

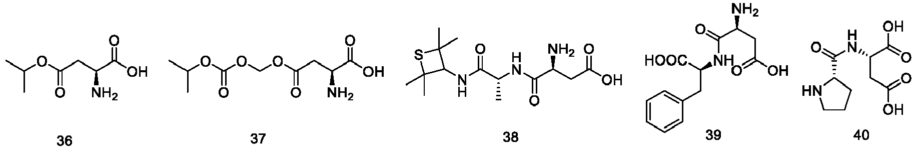

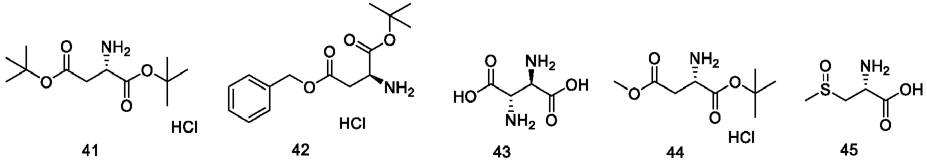

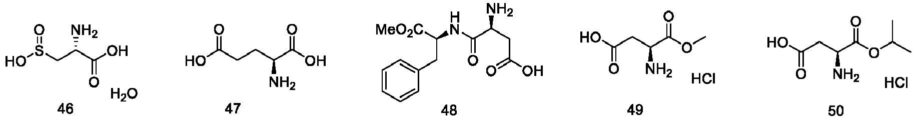

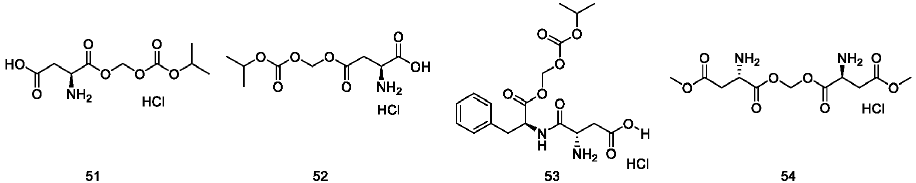

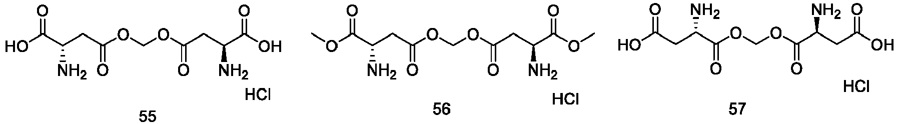

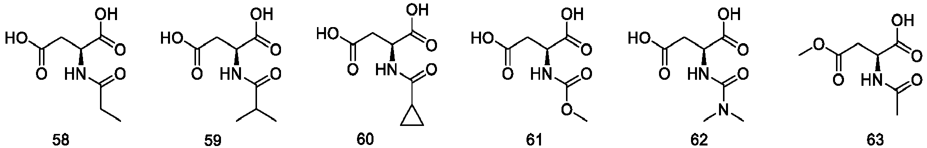

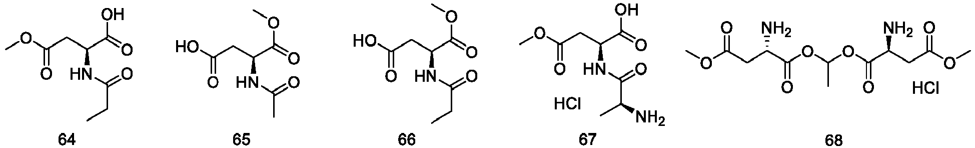

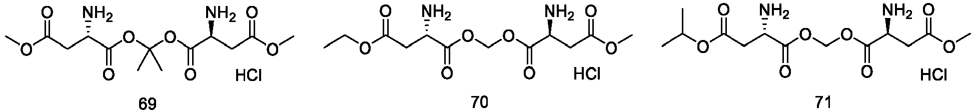

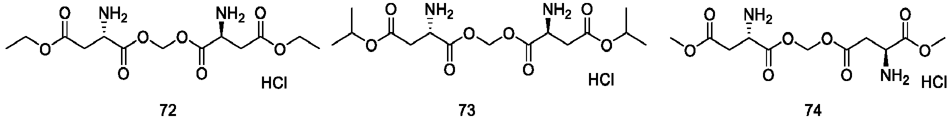

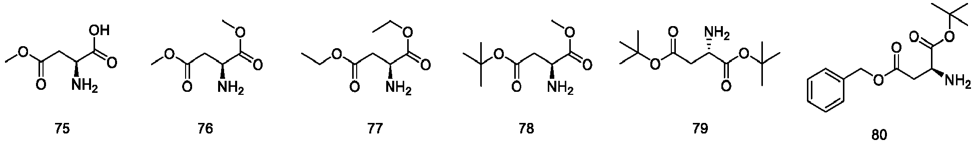

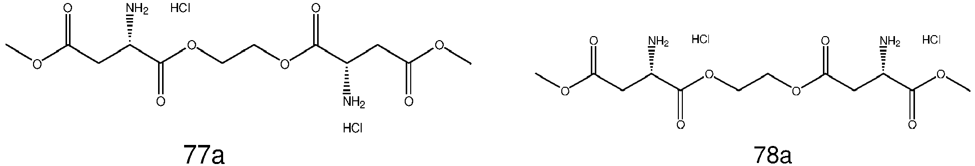

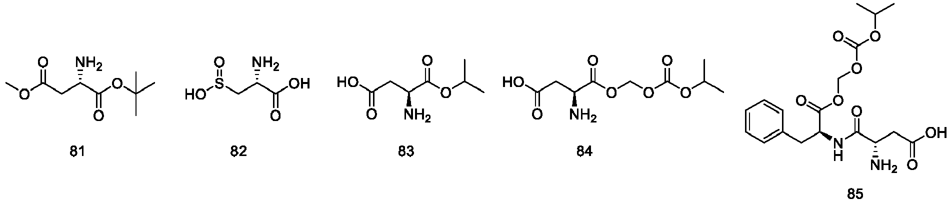

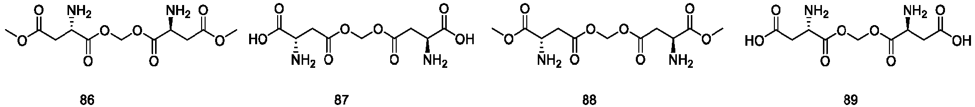

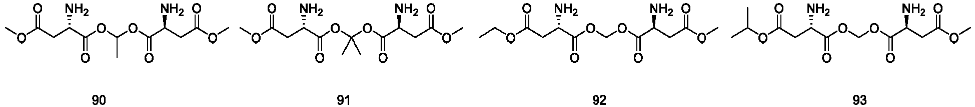

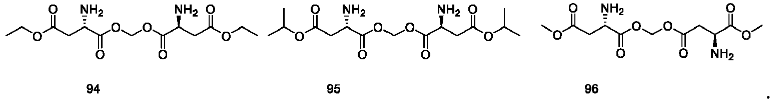

In some embodiments of the first and second aspects of the present invention, the compound represented by Formula I or 1-1, 1-2, 1-3 or 1-4 is selected from the group consisting of the following:

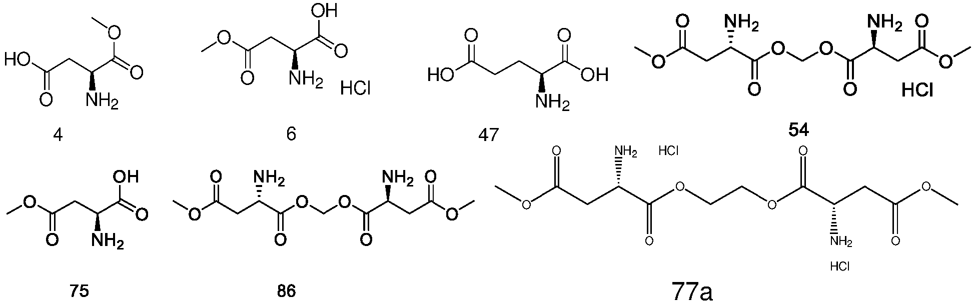

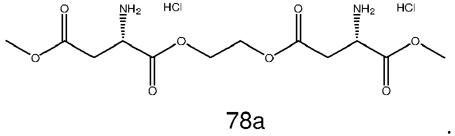

In the third aspect of the present invention, the present invention provides a use of a peptide containing 2 to 10 or 2 to 3 amino acids, or pharmaceutically acceptable salt, ester, prodrug, stereoisomer, hydrate, solvate, isotopic compound, crystalline form, metabolite form thereof, or any combination or mixture thereof, in the manufacture of a medicament or agent,
the drug or agent is used for at least one of the following:
   1) treating, improving, or preventing a liver disease (e.g., NAFLD, NASH, liver fibrosis);
   2) alleviating a mammalian liver fibrosis, liver steatosis, or inflammation;
   3) inhibiting the activation of a stellate cell;
   4) regulating the expression of NS3TP1;
in the peptide, at least one amino acid is aspartic acid, and when aspartic acid has a free carboxyl group, the free carboxyl group can optionally form an ester with C1-C6 alkyl-OH;
optionally, the amino acid is aspartic acid, phenylalanine, glycine, alanine, glutamic acid, cystine, glutamine, histidine, tyrosine, serine, methionine, arginine acid or leucine;
or, the free amino acid located on one side of the peptide is aspartic acid, and optionally, the free carboxyl group of the free aspartic acid is optionally esterified with C1-C6 alkyl-OH;
optionally, the remaining amino acids are aspartic acid, phenylalanine, glycine, alanine, glutamic acid, cystine, glutamine, histidine, tyrosine, serine, methionine, arginine or leucine.

In the fourth aspect of the present invention, the present invention provides the compound represented by the above Formula I, or pharmaceutically acceptable salt, ester, prodrug, stereoisomer, hydrate, solvate, isotope compound, crystalline form, metabolite form thereof, or any combination or mixture thereof, or the compound represented by the above Formula 1-4, or pharmaceutically acceptable salt, ester, prodrug, stereoisomer, hydrate, solvent, isotopic compound, crystalline form, metabolite form thereof, or any combination or mixture thereof, or the peptide containing 2 to 10 or 2 to 3 amino acids, or pharmaceutical above acceptable salt, ester, prodrug, stereoisomer, hydrate, solvate, isotopic compound, crystalline form, metabolite form thereof, or any combination or mixture thereof, as described in the third aspect above,
which is used for at least one of the following:
1) treating, improving, or preventing a liver disease (e.g., NAFLD, NASH, liver fibrosis);
2) alleviating a mammalian liver fibrosis, liver steatosis, and inflammation;
3) inhibiting the activation of a stellate cell;
4) regulating the expression of NS3TP1.

In a fifth aspect of the present invention, the present invention provides a method for obtaining the following effects:
1) treating, improving, or preventing a liver disease (e.g., NAFLD, NASH, liver fibrosis);
2) alleviating a mammalian liver fibrosis, liver steatosis, and inflammation;
3) inhibiting the activation of a stellate cell;
4) regulating the expression of NS3TP1;
which comprises administering to a subject in need thereof a therapeutically effective amount of the compound represented by the above Formula I, or pharmaceutically acceptable salt, ester, prodrug, stereoisomer, hydrate, solvate, isotope compound, crystalline form, metabolite form thereof, or any combination or mixture thereof, or the compound represented by the above Formula 1-4, or pharmaceutically acceptable salt, ester, prodrug, stereoisomer, hydrate, solvent, isotopic compound, crystalline form, metabolite form thereof, or any combination or mixture thereof, or the peptide containing 2 to 10 or 2 to 3 amino acids, or pharmaceutical above acceptable salt, ester, prodrug, stereoisomer, hydrate, solvate, isotopic compound, crystalline form, metabolite form thereof, or any combination or mixture thereof, as described in the third aspect above.

In the above-described first to fifth aspects of the present invention,
in some embodiments, the liver fibrosis includes, but is not limited to: liver fibrosis caused by chronic viral liver diseases, liver fibrosis caused by alcoholism or long-term drinking, liver fibrosis caused by non-alcoholic factor such as obesity, portal liver fibrosis caused by repeated schistosomiasis infection, biliary liver fibrosis caused by chronic cholestasis, metabolic liver fibrosis caused by hepatolenticular degeneration and hemoglobin deposition, toxic liver fibrosis caused by various toxic substances, malnutrition-induced liver fibrosis caused by a preference for low-protein diets and fatty fried foods, and cardiogenic liver fibrosis caused by chronic congestive heart failure.

In the sixth aspect of the present invention, the present invention provides a use of a pharmaceutical composition in the manufacture of a medicament, in which the pharmaceutical composition comprising the compound represented by the above Formula I, or pharmaceutically acceptable salt, ester, prodrug, stereoisomer, hydrate, solvate, isotope compound, crystalline form, metabolite form thereof, or any combination or mixture thereof, or the compound represented by the above Formula 1-4, or pharmaceutically acceptable salt, ester, prodrug, stereoisomer, hydrate, solvent, isotopic compound, crystalline form, metabolite form thereof, or any combination or mixture thereof, or the peptide containing 2 to 10 or 2 to 3 amino acids, or pharmaceutical above acceptable salt, ester, prodrug, stereoisomer, hydrate, solvate, isotopic compound, crystalline form, metabolite form thereof, or any combination or mixture thereof, as described in the third aspect above, and the medicament is used for at least one of the following:
1) treating, improving, or preventing a liver disease (e.g., NAFLD, NASH, liver fibrosis);
2) alleviating a mammalian liver fibrosis, liver steatosis, and inflammation;
3) inhibiting the activation of a stellate cell;
4) regulating the expression of NS3TP1.

In a seventh aspect of the present invention, the present invention provides a pharmaceutical composition, which is used for at least one of the following:
1) treating, improving, or preventing a liver disease (e.g., NAFLD, NASH, liver fibrosis);
2) alleviating a mammalian liver fibrosis, liver steatosis, and inflammation;
3) inhibiting the activation of a stellate cell;
4) regulating the expression of NS3TP1;
the pharmaceutical composition comprises the compound represented by the above Formula I, or pharmaceutically acceptable salt, ester, prodrug, stereoisomer, hydrate, solvate, isotope compound, crystalline form, metabolite form thereof, or any combination or mixture thereof, or the compound represented by the above Formula 1-4, or pharmaceutically acceptable salt, ester, prodrug, stereoisomer, hydrate, solvent, isotopic compound, crystalline form, metabolite form thereof, or any combination or mixture thereof, or the peptide containing 2 to 10 or 2 to 3 amino acids, or pharmaceutical above acceptable salt, ester, prodrug, stereoisomer, hydrate, solvate, isotopic compound, crystalline form, metabolite form thereof, or any combination or mixture thereof, as described in the third aspect above.

In an eighth aspect of the present invention, the present invention provides a method for obtaining the following effects:
1) treating, improving, or preventing a liver disease (e.g., NAFLD, NASH, liver fibrosis);
2) alleviating a mammalian liver fibrosis, liver steatosis, and inflammation;
3) inhibiting the activation of a stellate cell;
4) regulating the expression of NS3TP1;
which comprises administering to a subject in need thereof a therapeutically effective amount of the pharmaceutical composition, and the pharmaceutical composition comprises the compound represented by the above Formula I, or pharmaceutically acceptable salt, ester, prodrug, stereoisomer, hydrate, solvate, isotope compound, crystalline form, metabolite form thereof, or any combination or mixture thereof, or the compound represented by the above Formula 1-4, or pharmaceutically acceptable salt, ester, prodrug, stereoisomer, hydrate, solvent, isotopic compound, crystalline form, metabolite form thereof, or any combination or mixture thereof, or the peptide containing 2 to 10 or 2 to 3 amino acids, or pharmaceutical above acceptable salt, ester, prodrug, stereoisomer, hydrate, solvate, isotopic compound, crystalline form, metabolite form thereof, or any combination or mixture thereof, as described in the third aspect above.

In the above-mentioned sixth to eighth aspects of the present invention,
in some embodiments, the liver fibrosis includes, but is not limited to: liver fibrosis caused by chronic viral liver diseases, liver fibrosis caused by alcoholism or long-term drinking, liver fibrosis caused by non-alcoholic factor such as obesity, portal liver fibrosis caused by repeated schistosomiasis infection, biliary liver fibrosis caused by chronic cholestasis, metabolic liver fibrosis caused by hepatolenticular degeneration and hemoglobin deposition, toxic liver fibrosis caused by various toxic substances, malnutrition-induced liver fibrosis caused by a preference for low-protein diets and fatty fried foods, and cardiogenic liver fibrosis caused by chronic congestive heart failure.

In some embodiments, the pharmaceutical composition further comprises an additional active ingredient: other amino acids for improving liver function (including but not limited to alanine, glutamic acid, cystine, glutamine, glycine, histidine, tyrosine, serine, methionine, arginine, leucine), cholesterol absorption inhibitors (e.g., Ezetimibe), HSC activation and proliferation inhibitors (e.g., Pirfenidone, Fluorofenidone, Pegbelfermin), PCSK9 inhibitors, PPAR agonists (e.g., Gemfibrozil, Fenofibrate, Clofibrate, Bezafibrate, Pemafibrate, Elafibranor), ACE inhibitors, CCR2/5 inhibitors, TLR4 inhibition agents, LOXL2 inhibitors, TIMP-1 inhibitors, FXR agonists, AT1R blockers, NOX inhibitors, calcium channel blockers, ARBs, diuretics, renin, GLP-1 or synthetic variants thereof, insulin or synthetic variants thereof, metformin, sulfonylurea compounds, thiazolidinediones (TZD), SGLT2 inhibitors, DPP-IV inhibitors, inhibitors of HMGCoA reductase, inhibitors of proprotein convertase subtilisin/kexin type 9 (PCSK9), Gemcabene (CI-1027), ACC inhibitors, ApoC-III inhibitors, ACL-inhibitors (e.g., bepedic acid), prescription fish oil, CETP inhibitors, ursodeoxycholic acid, obeticholic acid, polyene phosphatidylcholine, glucocorticoids, silymarin, glycyrrhizic acid preparations (e.g., magnesium isoglycyrrhizinate injection and diammonium glycyrrhizinate enteric-coated capsules), and combinations thereof.

In some embodiments, the pharmaceutical composition further comprises a pharmaceutically acceptable carrier or excipient.

In some embodiments, the pharmaceutical composition is a solid preparation, an injection, a topical preparation, a spray, a liquid preparation or a compound preparation.

In the ninth aspect of the present invention, the present invention provides a compound represented by Formula II, pharmaceutically acceptable salt or ester, prodrug, stereoisomer, hydrate, solvate, crystalline form, and metabolism form thereof,

A₁-(L₁)ₓ-A₁' Formula II

wherein, A₁ is 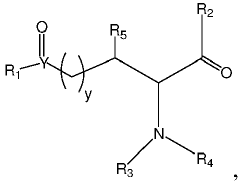 A₁' is 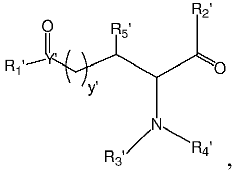 A₁ and A₁' are the same or different;
x is selected from the group consisting of 0, 1 and 2;

1) when x is 0, A' is absent; for A₁, wherein:
   R₁ and R₂ are independently selected from the group consisting of: 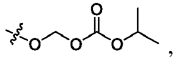 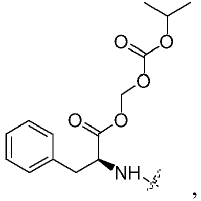 -O-R^{a},
   R^{a} is each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-NR^{b4}-(CO)-R^{b5}, -(C1-C6 alkyl)ₙ-NR^{b6}-(CO)-O-R^{b7}, the alkyl, alkenyl, alkynyl, cycloalkyl, aryl, and heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
   R^{b1}, R^{b2}, R^{b3}, R^{b4}, R^{b5}, R^{b6}, R^{b7} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, 3- to 15-membered heterocyclyl;
   R₃ and R₄ are independently selected from the group consisting of: -COCH(CH₃)(NH₂), hydrogen, -COCH₂CH₃, deuterium, C1-C6 alkyl, the alkyl is optionally substituted with one or more groups independently selected from the group consisting of halogen, C1-C6 alkyl, halogenated C1-C6 alkyl, aryl, heterocyclyl;
   and for "x is 0, A' is absent", A₁ meets one of the following conditions, (1), (2), (3) or (4):
      (1) at least one of R₁ and R₂ is 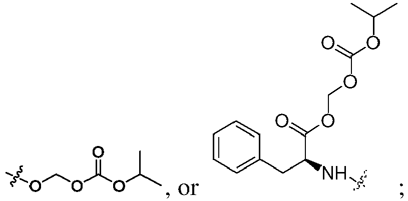
      (2) at least one of R₃ and R₄ is -COCH(CH₃)(NH₂) or -COCH₂CH₃;
      (3) when the situation is: R₁ and R₂ are independently selected from the group consisting of: 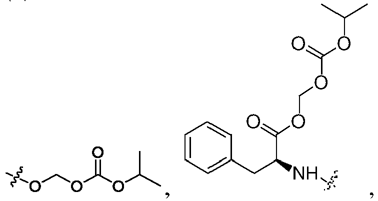 hydroxyl, methoxy, and at the same time, R₃ and R₄ are independently selected from the group consisting of: hydrogen, -COCH₂CH₃ and -COCH(CH₃)(NH₂),
   A₁ meets the following conditions:
      when one of R₁ and R₂ is hydroxyl or methoxy, the other of R₁ and R₂ is 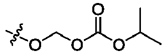 or 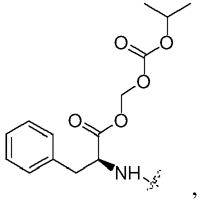 or one of R₃ and R₄ is -COCH(CH₃)(NH₂);
      (4) when the situation is: R₁ is selected from the group consisting of: methoxy, 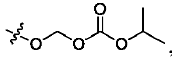 , hydroxyl,
      R₂ is selected from the group consisting of: 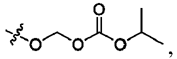 hydroxyl, 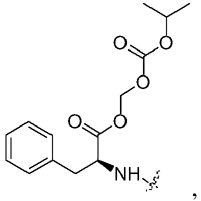 methoxy,
      and at the same time, R₃ and R₄ are independently selected from the group consisting of: hydrogen, -COCH₂CH₃ and -COCH(CH₃)(NH₂),
      A₁ meets the following conditions:
         when R₁ is hydroxyl or methoxy, R₂ is 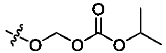 or 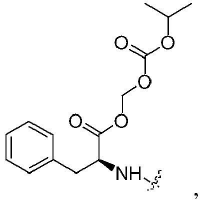 or one of R₃ and R₄ is -COCH(CH₃)(NH₂), or
         when R₂ is hydroxyl or methoxy, R₁ is 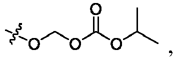 or one of R₃ and R₄ is -COCH(CH₃)(NH₂);
2) when x is 1, A₁ and A₁' are the same or different, wherein
   any one of R₁, R₂, R₃, and R₄ of a unit A₁ is connected to any one of R₁', R₂', R₃' and R₄' of an adjacent unit A₁' through L₁;
   each L₁ is independently a unit-linking group selected from the group consisting of: absence, O, S, carbonyl, alkyl (or C1-C6 alkyl), alkenyl (or C2-C6 alkenyl), alkynyl (or C2-C6 alkynyl), -O-alkyl-O- (or -O-C1-C6 alkyl-O-), -O-alkenyl-O- (or -O-C2-C6 alkenyl-O-), -O-alkynyl-O-(or -O-C2-C6 alkynyl-O-), cycloalkyl (or C3-C7 cycloalkyl), heteroalkyl (or 3- to 7-membered heteroalkyl), wherein the alkyl, cycloalkyl, heteroalkyl are optionally substituted with one or more groups independently selected from the group consisting of Z;
   Z is independently selected from the group consisting of: halogen, amino, alkyl (or C1-C6 alkyl), alkenyl (or C2-C6 alkenyl), alkynyl (or C2-C6 alkynyl), haloalkyl (or halogenated C1-C6 alkyl), cycloalkyl (or C3-C7 cycloalkyl), aryl (or C6-C12 aryl), heterocyclyl;
   or, L₁ is selected from the group consisting of: absence, -CR'R", wherein R' and R" are each independently H, C1-C6 alkyl (or C1-C3 alkyl);
   or, L₁ is selected from the group consisting of: absence, -CH₂-, -CH(CH₃)-, -C-(CH₃)₂;
   for A₁, wherein:
      R₁ and R₂ are each independently selected from the group consisting of: C1-C6 alkyl, -O-, -O-R^{a1}, -NR^{a2}R^{a3};
      R₃ and R₄ are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), heterocyclyl;
      R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
      or, R₁ is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
      or, R₁ is selected from the group consisting of: -O-, hydroxyl, -O-R^{a}: R^{a} is C1-C6 alkyl;
      or, R₁ is selected from the group consisting of: -O-, hydroxyl, methoxy, ethoxy, -OCH(CH₃)₂;
      or, R₂ is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
      or, R₂ is selected from the group consisting of: hydroxyl, -O-, -O-R^{a}: R^{a} is C1-C6 alkyl;
      or, R₂ is selected from the group consisting of: hydroxyl, -O-, methoxy,
      R₃ and R₄ are each independently selected from the group consisting of: hydrogen, hydroxyl, C1-C6 alkyl;
      or, R₃ and R₄ are each independently hydrogen;
      R₅ is selected from the group consisting of: hydrogen, deuterium, C1-C6 alkyl, halogen, and amino;
      or, R₅ is selected from the group consisting of hydrogen and amino;
      or, R₅ is selected from the group consisting of hydrogen;
      Y is C;
      y is selected from the group consisting of 0, 1, 2, 3; or y is 0 or 1; or y is 0;
      for A₁', wherein:
         R₁' and R₂' are each independently selected from the group consisting of: C1-C6 alkyl, -O-, -O-R^{a1}, -NR^{a2}R^{a3};
         R₃' and R₄' are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl) or heterocyclyl;
         R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
         or, R₁' is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
         or, R₁' is selected from the group consisting of: -O-, hydroxyl, -O-R^{a}: R^{a} is C1-C6 alkyl;
         or, R₁' is selected from the group consisting of: -O-, hydroxyl, methoxy, ethoxy, -OCH(CH₃)₂;
         or, R₂' is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
         or, R₂' is selected from the group consisting of: hydroxyl, -O-, -O-R^{a}: R^{a} is C1-C6 alkyl;
         or, R₂' is selected from the group consisting of: hydroxyl, -O-, methoxy;
         R₃' and R₄' are each independently selected from the group consisting of: hydrogen, hydroxyl, C1-C6 alkyl;
         or, R₃' and R₄' are each independently hydrogen;
         R₅' is selected from the group consisting of: hydrogen, deuterium, C1-C6 alkyl, halogen, and amino;
         or, R₅' is selected from the group consisting of hydrogen and amino;
         or, R₅' is selected from the group consisting of hydrogen;
         Y' is C;
         y' is selected from the group consisting of 0, 1, 2, 3; or y is 0 or 1; or y' is 0;
3) when x is 2, A₁ and A₁' are the same or different, wherein
   any one of R₁, R₂, R₃ and R₄ of a unit A₁ is connected to any one of R₁', R₂', R₃' and R₄' of an adjacent unit A₁' through L₁;
   each L₁ is independently a unit-linking group selected from the group consisting of: absence, O, S, carbonyl, alkyl (or C1-C6 alkyl), alkenyl (or C2-C6 alkenyl), alkynyl (or C2-C6 alkynyl), -O-alkyl-O- (or -O-C1-C6 alkyl-O-), -O-alkenyl-O- (or -O-C2-C6 alkenyl-O-), -O-alkynyl-O-(or -O-C2-C6 alkynyl-O-), cycloalkyl (or C3-C7 cycloalkyl), heteroalkyl (or 3- to 7-membered heteroalkyl), wherein the alkyl, cycloalkyl, heteroalkyl are optionally substituted with one or more groups independently selected from the group consisting of Z;
   Z is independently selected from the group consisting of: halogen, amino, alkyl (or C1-C6 alkyl), alkenyl (or C2-C6 alkenyl), alkynyl (or C2-C6 alkynyl), haloalkyl (or halogenated C1-C6 alkyl), cycloalkyl (or C3-C7 cycloalkyl), aryl (or C6-C12 aryl), heterocyclyl;
   or, L₁ is selected from the group consisting of: absence, -CR'R", wherein R' and R" are each independently H, C1-C6 alkyl (or C1-C3 alkyl);
   or, L₁ is selected from the group consisting of: absence, -CH₂-, -CH(CH₃)-, -C-(CH₃)₂;
   or, L₁ is selected from the group consisting of: -CH₂-;
   for A₁, wherein:
      R₁ and R₂ are each independently selected from the group consisting of: C1-C6 alkyl, -O-, -O-R^{a1}, -NR^{a2}R^{a3};
      R₃ and R₄ are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), heterocyclyl;
      R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
      or, R₁ is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
      or, R₁ is selected from the group consisting of: -O-, hydroxyl, -O-R^{a}: R^{a} is C1-C6 alkyl;
      or, R₁ is selected from the group consisting of: -O-, hydroxyl, methoxy, ethoxy, -OCH(CH₃)₂;
      or, R₁ is selected from the group consisting of: methoxy;
      or, R₂ is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
      or, R₂ is selected from the group consisting of: hydroxyl, -O-, -O-R^{a}: R^{a} is C1-C6 alkyl;
      or, R₂ is selected from the group consisting of: hydroxyl, -O-, methoxy,
      or, R₂ is selected from the group consisting of: -O-;
      R₃ and R₄ are each independently selected from the group consisting of: hydrogen, hydroxyl, C1-C6 alkyl;
      or, R₃ and R₄ are each independently hydrogen;
      R₅ is selected from the group consisting of: hydrogen, deuterium, C1-C6 alkyl, halogen, and amino;
      or, R₅ is selected from the group consisting of hydrogen and amino;
      or, R₅ is selected from the group consisting of hydrogen;
      Y is C;
      y is selected from the group consisting of 0, 1, 2, 3; or y is 0 or 1; or y is 0;
      for A₁', wherein:
         R₁' and R₂' are each independently selected from the group consisting of: C1-C6 alkyl, -O-, -O-R^{a1}, -NR^{a2}R^{a3};
         R₃' and R₄' are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl) and heterocyclyl;
         R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
         or, R₁' is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
         or, R₁' is selected from the group consisting of: -O-, hydroxyl, -O-R^{a}: R^{a} is C1-C6 alkyl;
         or, R₁' is selected from the group consisting of: -O-, hydroxyl, methoxy, ethoxy, -OCH(CH₃)₂;
         or, R₁' is selected from the group consisting of: methoxy;
         or, R₂' is selected from the group consisting of: -O-, C1-C6 alkyl, -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), -(C1-C6 alkyl)-(3- to 15-membered heterocyclyl);
         or, R₂' is selected from the group consisting of: hydroxyl, -O-, -O-R^{a}: R^{a} is C1-C6 alkyl;
         or, R₂' is selected from the group consisting of: hydroxyl, -O-, methoxy;
         or, R₂' is selected from the group consisting of: -O-;
         R₃' and R₄' are each independently selected from the group consisting of: hydrogen, hydroxyl, C1-C6 alkyl;
         or, R₃' and R₄' are each independently hydrogen;
         Rs' is selected from the group consisting of: hydrogen, deuterium, C1-C6 alkyl, halogen, and amino;
         or, Rs' is selected from the group consisting of hydrogen and amino;
         or, Rs' is selected from the group consisting of hydrogen;
         Y is C;
         y' is selected from the group consisting of 0, 1, 2, 3; or y is 0 or 1; or y' is 0;
         or, Formula II is:

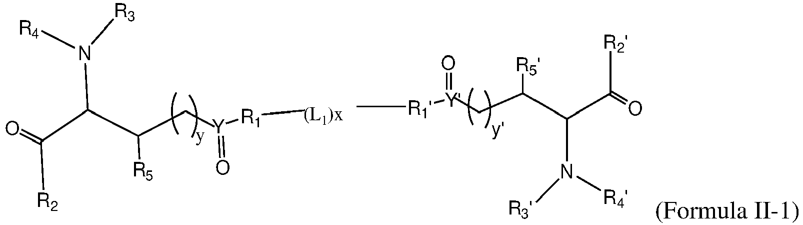

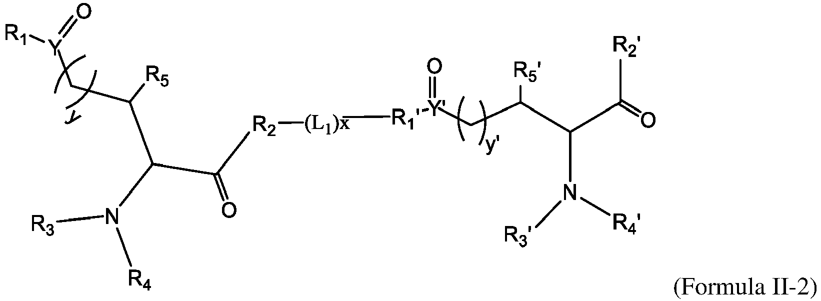

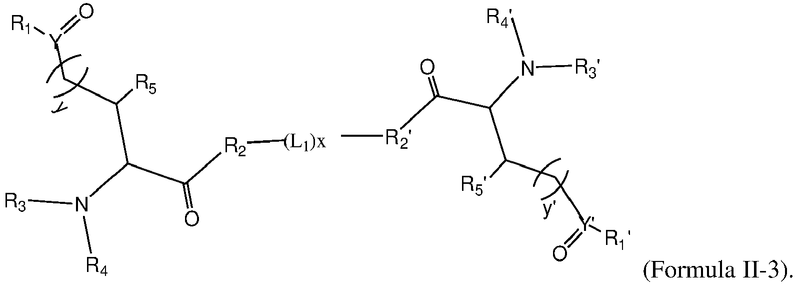

In some embodiments, the compound represented by Formula II or II-1, II-2, II-3 is selected from the group consisting of the following:

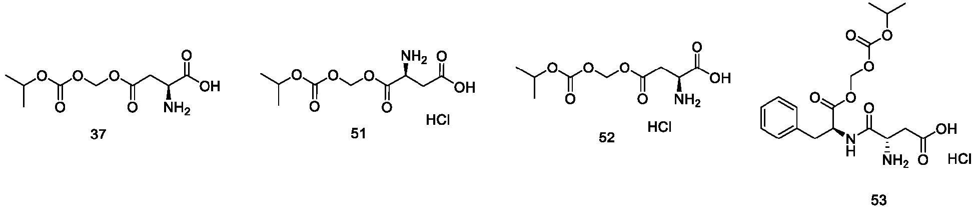

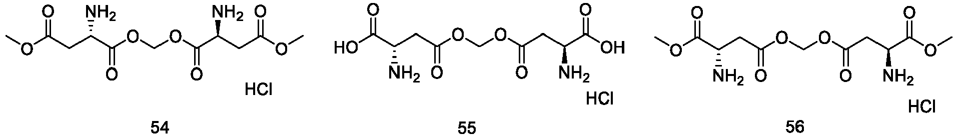

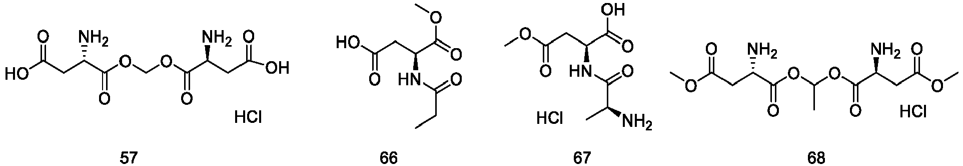

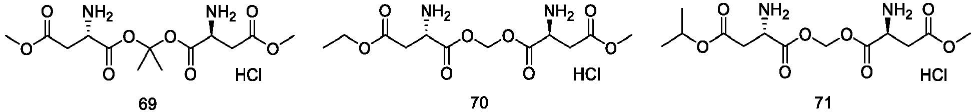

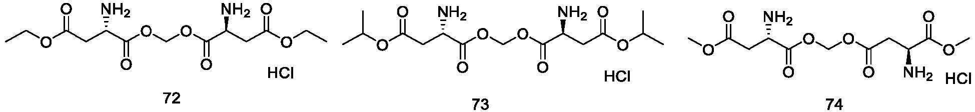

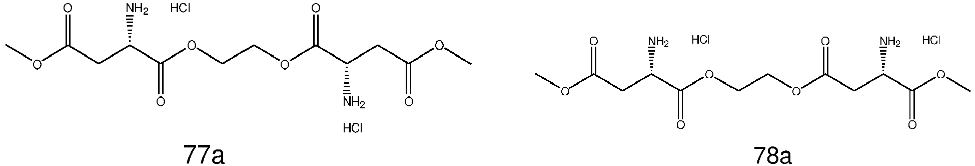

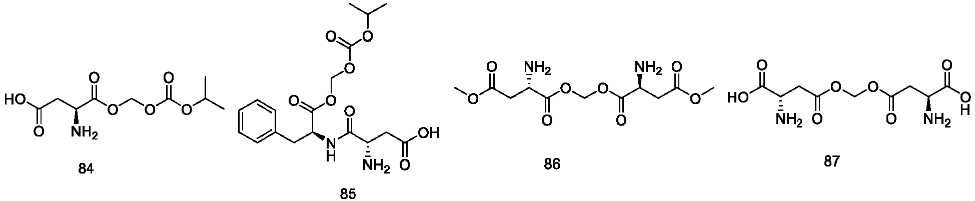

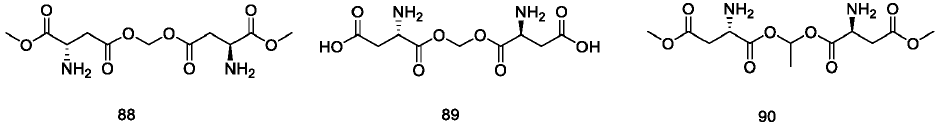

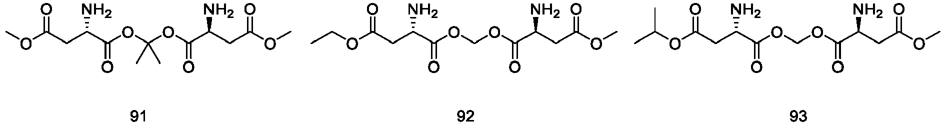

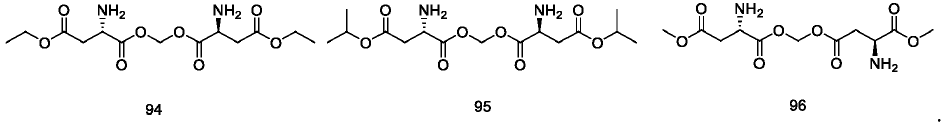

In the tenth aspect of the present invention, the present invention provides a pharmaceutical composition, which comprises the compound represented by the above Formula II, pharmaceutically acceptable salt or ester, prodrug, stereoisomer, hydrate, and solvate, crystalline form, metabolite form thereof, or any combination or mixture thereof.

In the eleventh aspect of the present invention, the present invention provides a use of the compound represented by the above Formula II, pharmaceutically acceptable salt, prodrug, stereoisomer, hydrate, solvate, crystalline form, metabolism thereof, or any combination or mixture thereof, in the manufacture of a medicament or agent, the medicament is used for at least one of the following:
1) treating, improving, or preventing a liver disease (e.g., NAFLD, NASH, liver fibrosis);
2) Reduce mammalian liver fibrosis, liver steatosis, or inflammation;
3) inhibiting the activation of stellate cells;
4) regulating the expression of NS3TP1.

In a twelfth aspect of the present invention, the present invention provides a use of a pharmaceutical composition in the manufacture of a medicament or agent, the medicament is used for at least one of the following:
1) treating, improving, or preventing a liver disease (e.g., NAFLD, NASH, liver fibrosis);
2) Reduce mammalian liver fibrosis, liver steatosis, or inflammation;
3) inhibiting the activation of stellate cells;
4) regulating the expression of NS3TP1;
the pharmaceutical composition comprises the compound represented by Formula II above, pharmaceutically acceptable salt or ester, prodrug, stereoisomer, hydrate, solvate, crystalline form, metabolite form thereof, or any combination or mixture thereof.

In the thirteenth aspect of the present invention, the present invention provides the compound represented by the above Formula II, pharmaceutically acceptable salt, prodrug, stereoisomer, hydrate, solvate, crystalline form, metabolism form thereof, or any combination or mixture thereof, or the pharmaceutical composition comprising the compound represented by Formula II, pharmaceutically acceptable salt or ester, prodrug, stereoisomer, hydrate, solvate, crystalline form, metabolite form thereof, or any combination or mixture thereof, which is used for at least one of the following:
1) treating, improving, or preventing a liver disease (e.g., NAFLD, NASH, liver fibrosis);
2) alleviating a mammalian liver fibrosis, liver steatosis, and inflammation;
3) inhibiting the activation of stellate cells;
4) regulating the expression of NS3TP1.

In a fourteenth aspect of the present invention, the present invention provides a method for obtaining the following effects:
1) treating, improving, or preventing a liver disease (e.g., NAFLD, NASH, liver fibrosis);
2) alleviating a mammalian liver fibrosis, liver steatosis, and inflammation;
3) inhibiting the activation of stellate cells;
4) regulating the expression of NS3TP1;
which comprises administering to a subject in need thereof a therapeutically effective amount of the compound represented by the above Formula II, pharmaceutically acceptable salt, prodrug, stereoisomer, hydrate, solvate, crystalline form, metabolism form thereof, or any combination or mixture thereof, or the pharmaceutical composition comprising the compound represented by Formula II, pharmaceutically acceptable salt or ester, prodrug, stereoisomer, hydrate, solvate, crystalline form, metabolite form thereof, or any combination or mixture thereof.

In the above-described ninth to fourteenth aspects of the present invention,
in some embodiments, the liver fibrosis includes, but is not limited to: liver fibrosis caused by chronic viral liver diseases, liver fibrosis caused by alcoholism or long-term drinking, liver fibrosis caused by non-alcoholic factor such as obesity, portal liver fibrosis caused by repeated schistosomiasis infection, biliary liver fibrosis caused by chronic cholestasis, metabolic liver fibrosis caused by hepatolenticular degeneration and hemoglobin deposition, toxic liver fibrosis caused by various toxic substances, malnutrition-induced liver fibrosis caused by a preference for low-protein diets and fatty fried foods, and cardiogenic liver fibrosis caused by chronic congestive heart failure.

In some embodiments, the pharmaceutical composition further comprises an additional active ingredient: other amino acids for improving liver function (including but not limited to alanine, glutamic acid, cystine, glutamine, glycine, histidine, tyrosine, serine, methionine, arginine, leucine), cholesterol absorption inhibitors (e.g., Ezetimibe), HSC activation and proliferation inhibitors (e.g., Pirfenidone, Fluorofenidone, Pegbelfermin), PCSK9 inhibitors, PPAR agonists (e.g., Gemfibrozil, Fenofibrate, Clofibrate, Bezafibrate, Pemafibrate, Elafibranor), ACE inhibitors, CCR2/5 inhibitors, TLR4 inhibition agents, LOXL2 inhibitors, TIMP-1 inhibitors, FXR agonists, AT1R blockers, NOX inhibitors, calcium channel blockers, ARBs, diuretics, renin, GLP-1 or synthetic variants thereof, insulin or synthetic variants thereof, metformin, sulfonylurea compounds, thiazolidinediones (TZD), SGLT2 inhibitors, DPP-IV inhibitors, inhibitors of HMGCoA reductase, inhibitors of proprotein convertase subtilisin/kexin type 9 (PCSK9), Gemcabene (CI-1027), ACC inhibitors, ApoC-III inhibitors, ACL-inhibitors (e.g., bepedic acid), prescription fish oil, CETP inhibitors, ursodeoxycholic acid, obeticholic acid, polyene phosphatidylcholine, glucocorticoids, silymarin, glycyrrhizic acid preparations (e.g., magnesium isoglycyrrhizinate injection and diammonium glycyrrhizinate enteric-coated capsules), and combinations thereof.

In some embodiments, the pharmaceutical composition further comprises a pharmaceutically acceptable carrier or excipient.

In some embodiments, the pharmaceutical composition is a solid preparation, an injection, a topical preparation, a spray, a liquid preparation or a compound preparation.

In the above aspects of the present invention and embodiments thereof,
The "C1-C6 alkyl" in "C1-C6 alkyl" and various composite groups involving "C1-C6 alkyl" (e.g., "halogenated C1-C6 alkyl") can be replaced by "C1-C20 alkyl", "C1-C12 alkyl", "C1-C10 alkyl", "C1-C8 alkyl", "C1-C4 alkyl", "C1-C3 alkyl" or "C1-C2 alkyl";
The "C2-C6 alkenyl" in "C2-C6 alkenyl" and various composite groups involving "C2-C6 alkenyl" can be replaced by "C2-C12 alkenyl", "C2-C8 alkenyl" or "C2-C4 alkenyl";
The "C2-C6 alkynyl" in "C2-C6 alkynyl" and various composite groups involving "C2-C6 alkynyl" can be replaced by "C2-C12 alkynyl", "C2-C8 alkynyl" or "C2-C4 alkynyl";
The "C3-C7 cycloalkyl" in "C3-C7 cycloalkyl" and various composite groups involving "C3-C7 cycloalkyl" can be replaced by "C3-C20 cycloalkyl", "C3-C12 "Cycloalkyl" or "C3-C10 cycloalkyl";
The "C1-C6 alkoxy" in "C1-C6 alkoxy" and various composite groups involving "C1-C6 alkoxy" can be replaced by "C1-C20 alkoxy", "C1-C12 alkoxy" or "C1 -C10 alkoxy";
The "C6-C10 aryl" in "C6-C10 aryl" and various composite groups involving "C6-C10 aryl" can be replaced by "C6-C12 aryl";
The "3- to 7-membered heteroalkyl" in "3-7-membered heteroalkyl" and various composite groups involving it can be replaced with "2- to 14-membered heteroalkyl";
The "3- to 15-membered heterocyclyl" in "3- to 15-membered heterocyclyl" and various composite groups involving it can be replaced by "3- to 20-membered heterocyclyl" or "3- to 12-membered heterocyclyl" " or "3- to 10-membered heterocyclyl".

### Brief Description of the Drawings

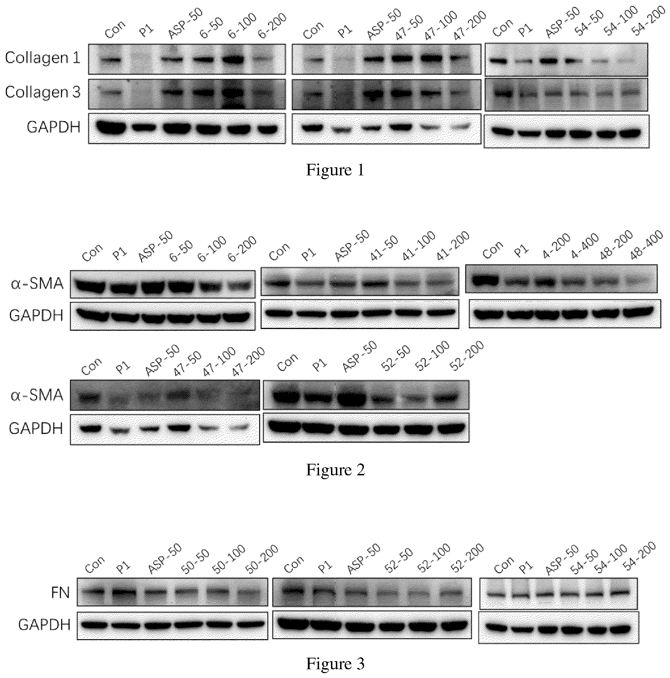

Figure 1 shows the inhibitory effects of Compound **6,** Compound **47,** and Compound **54** on the protein expression of collagen I and collagen III; GAPDH (glyceraldehyde-3-phosphate dehydrogenase) was used as a control.

Therein, "con" represents the control well, "P1" represents the positive control well (hydronidone), and "ASP-50" represents that the aspartic acid concentration was 50 µM; the remaining numbers are all in the form of compound number-concentration (µM): "6-50" represents that the concentration of Compound **6** was 50 µM, "6-100" represents that the concentration of Compound **6** was 100 µM, "6-200" represents that the concentration of Compound **6** was 200 µM; "47-50" represents that the concentration of Compound **47** was 50 µM, and "47-100" represents that the concentration of Compound **47** was 100 µM, "47-200" represents that the concentration of Compound **47** was 200 µM; "54-50" represents that the concentration of Compound **54** was 50 µM, "54-100" represents that the concentration of Compound **54** was 100 µM, "54-200" represents that the concentration of Compound **54** was 200 µM.

Figure 2 shows the inhibitory effects of Compound **4,** Compound **6,** Compound **41,** Compound **47,** Compound **48,** and Compound **52** on α-SMA protein expression; GAPDH was used as a control.

Therein, "con" represents the control well, "P1" represents the positive control well (hydronidone), and "ASP-50" represents that the aspartic acid concentration was 50 µM; the remaining numbers are all in the form of compound number-concentration (µM).

Figure 3 shows the inhibitory effects of Compound **50**, Compound **52**, and Compound **54** on FN protein expression; GAPDH was used as a control.

Therein, "con" represents the control well, "P1" represents the positive control well (hydronidone), and "ASP-50" represents the aspartic acid concentration of 50 µM; the remaining numbers are all in the form of compound number-concentration (µM).

Figure 4 shows the inhibitory effects of Compound **54,** Compound **77a,** and Compound **78a** on the expression of Collagen I protein and α-SMA protein in LX2 cells stimulated by TGFβ; GAPDH was used as a control.

Figure 5 shows the decrease in NS3TP1 expression in the liver tissue of the liver injury mouse model, and the regulatory effects of different doses of Compound **6** on NS3TP1 expression in liver tissue of the liver injury mouse model; in the figure, "mpk" represents "mg per kg".

Figure 6 shows the Masson-stained sections of mouse liver tissue after intraperitoneal injection of carbon tetrachloride and treatment with Compound **4,** Compound **6,** Compound **47,** and Compound **54.**

Therein, "40×" means that the microscope magnification was 40 times, and "100×" means that the microscope magnification was 100 times.

Figure 7 shows the Masson-stained sections of mouse liver tissue after intraperitoneal injection of carbon tetrachloride and treatment with different doses of Compound **6.**

Therein, "40×" means that the microscope magnification was 40 times, and "100×" means that the microscope magnification was 100 times.

Figure 8 shows the concentration of Compound **6** in rats after oral administration and injection of Compound **6** (top left), the concentration of aspartic acid in rats after oral administration and injection of Compound **6** (top right), the concentration of Compound **6** in rats after injection of normal saline (bottom left), and the concentration of aspartic acid in rats after injection of physiological saline (bottom right).

Figure 9 shows the H&E-stained sections of mouse liver tissue after HFD diet feeding and treatment with different doses of Compound **6.**

Therein, "100×" means that the magnification of the microscope was 100 times, and "200×" means that the magnification of the microscope was 200 times.

Figure 10 shows the H&E-stained sections of mouse liver tissue after CHOL diet feeding and treatment with different doses of Compound **6.**

Therein, "100×" means that the magnification of the microscope was 100 times, and "200×" means that the magnification of the microscope was 200 times.

### Specific Models for Carrying Out the present invention

The examples of the present invention are described in detail below. The examples described below are exemplary and are intended to explain the present invention, but should not be understood as limiting the present invention.

As used herein, the term "effective amount" or "therapeutically effective amount" refers to an amount that is sufficient, within the scope of reasonable medical judgment, to treat or prevent a patient's disease but low enough to avoid serious side effects (at a reasonable benefit/risk ratio). The therapeutically effective amount of a compound will vary upon the specific compound selected (e.g., taking into account the potency, effectiveness, and half-life of the compound), the route of administration selected, the disease being treated, the severity of the disease being treated, the factors of patient being treated such as age, size, weight and medical illness, the medical history of the patient being treated, duration of treatment, the nature of concurrent therapies, the desired therapeutic effect, etc., but can still be routinely determined by those skilled in the art.

As used herein, the term "mammal" refers to a warm-blooded animal that suffers from or is at risk of developing the diseases described herein, including but not limited to guinea pig, dog, cat, rat, mouse, hamster, and primate, including human.

In addition, it should be pointed out that the specific dosage and usage of the compound represented by Formula I or Formula 1-4 or Formula II, stereoisomer thereof or pharmaceutically acceptable salt and/or solvate thereof and/or hydrate thereof are determined by many factors, including the patient's age, body weight, gender, natural health status, nutritional status, activity intensity of drug, administration time, metabolic rate, severity of disease, and the subjective judgment of physician. The preferred dosage used herein is 0.001 to 1000 mg/kg body weight/day.

The pharmaceutically acceptable salt of the compound represented by Formula I, Formula 1-4 or Formula II of the present invention includes inorganic or organic acid salt thereof, as well as inorganic or organic alkali salt. The present invention relates to all forms of the above-mentioned salt, which includes, but is not limited to: sodium salt, potassium salt, calcium salt, lithium salt, meglumine salt, hydrochloride, hydrobromide, hydriodate, nitrate, sulfate, bisulfate, phosphate, hydrophosphate, acetate, propionate, butyrate, oxalate, trimethylacetate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and pamoate, etc.

The pharmaceutical composition involved in the present invention may comprise a pharmaceutically acceptable carrier. The carrier includes but is not limited to: ion exchanger, aluminum oxide, aluminum stearate, lecithin, serum protein such as human albumin, and buffer substance such as phosphate, glycerin, sorbic acid, potassium sorbate, partial glyceride mixture of saturated vegetable fatty acid, water, salt or electrolyte such as protamine sulfate, disodium hydrogen phosphate, potassium hydrogen phosphate, sodium chloride, zinc salt, colloidal silicon oxide, magnesium trisilicate, polyvinylpyrrolidone, cellulosic substance, polyethylene glycol, sodium carboxymethylcellulose, polyacrylate, beeswax, lanolin.

The pharmaceutical composition of the present invention can be prepared in various forms according to different administration routes.

According to the present invention, the pharmaceutical composition can be administered in any of the following ways: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration by means of an explanted reservoir. Therein, oral, intraperitoneal or intravenous administration is preferred.

When used for oral administration, the compound represented by Formula I or Formula 1-4 or Formula II, stereoisomer thereof or pharmaceutically acceptable salt and/or solvate thereof and/or hydrate thereof can be made into any orally acceptable preparation forms, including, but not limited to, tablets, capsules, aqueous solutions or aqueous suspensions. Therein, commonly used carriers for tablets include lactose and corn starch, and lubricants such as magnesium stearate can also be added. Commonly used diluents for capsule formulations include lactose and dried corn starch. Aqueous suspension formulations usually consist of the active ingredient mixed with suitable emulsifying and suspending agents. If desired, some sweetening, flavoring or coloring agents may be added to the above oral preparation forms.

When used for rectal administration, the compound represented by Formula I or Formula 1-4 or Formula II, stereoisomer thereof or pharmaceutically acceptable salt and/or solvate thereof and/or hydrate thereof can generally be made into a suppository form, which is prepared by mixing the drug with a suitable non-irritating excipient. The excipient is solid at room temperature and melts at rectal temperature to release the drug. Such excipient includes cocoa butter, beeswax and polyethylene glycol.

When used for topical administration, especially when treating affected surfaces or organs easily accessible by topical application, such as eye, skin or lower intestinal neurological diseases, the compound represented by Formula I, Formula 1-4 or Formula II, stereoisomer thereof or pharmaceutically acceptable salt and/or solvate thereof and/or hydrate thereof can be made into different topical preparation forms according to different affected surfaces or organs. The specific instructions are as follows:
When used for topical administration to eyes, the compound represented by Formula I or Formula 1-4 or Formula II, stereoisomer thereof or pharmaceutically acceptable salt and/or solvate thereof and/or hydrate thereof can be formulated in the form of micronized suspension or solution, the carrier used is isotonic sterile saline with a certain pH, in which a preservative such as chlorobenzyl alkoxide may or may not be added. Additionally, for ophthalmic use, the compound can also be formulated in the form of ointment such as petroleum jelly.
When used for topical administration to skin, the compound represented by Formula I or Formula 1-4 or Formula II, stereoisomer thereof or pharmaceutically acceptable salt and/or solvate thereof and/or hydrate thereof can be formulated in the form of suitable ointment, lotion or cream in which the active ingredient is suspended or dissolved in one or more carriers. The carriers that can be used for ointment include, but are not limited to: mineral oil, liquid vaseline, white vaseline, propylene glycol, polyoxyethylene, polyoxypropylene, emulsified wax and water; the carriers that can be used for lotion or cream include, but are not limited to: mineral oil, sorbitan monostearate, Tween 60, cetyl ester wax, hexadecene aromatic alcohol, 2-octyldodecanol, benzyl alcohol and water.
When used for topical administration to lower intestine, the compound represented by Formula I or Formula 1-4 or Formula II, stereoisomer thereof or pharmaceutically acceptable salt and/or solvate thereof and/or hydrate thereof can be formulated in the form of rectal suppository or suitable enema preparation as described above, and topical transdermal patch may also be used.

The compound represented by Formula I or Formula 1-4 or Formula II, stereoisomer thereof or pharmaceutically acceptable salt and/or solvate thereof and/or hydrate thereof can also be administered in the form of sterile injection preparation, including sterile injectable aqueous or oily suspension, or sterile injectable solution. Therein, the carriers and solvents that can be used include water, Ringer's solution and isotonic sodium chloride solution. Alternatively, sterile nonvolatile oil such as monoglyceride or diglyceride may also be used as solvent or suspending medium.

The drugs in various dosage forms mentioned above can be prepared according to conventional methods in the pharmaceutical field.

In various parts of the present specification, substituents of the compounds disclosed herein are disclosed according to group types or ranges. In particular, the present invention comprises each and every individual subcombination of the individual members of these group types and ranges. For example, the term "C1-C6 alkyl" specifically refers to the independently disclosed methyl, ethyl, C3 alkyl, C4 alkyl, C5 alkyl and C6 alkyl, or "C1-C4 alkyl", or "C1-C3 alkyl".

In addition, it should be noted that, unless otherwise clearly stated, the description "... independently selected from the group consisting of" used throughout herein should be understood in a broad sense, which refers to that the specific options expressed by different symbols do not affect each other and can be the same or different.

"Optionally" means that the situation mentioned may or may not exist. For example, when it is described that a certain structure is "optionally" substituted by certain groups, it means that the "substitution" may or may not exist.

Unless otherwise indicated, the structural formulas described in the present invention include all isomeric forms such as enantiomers, diastereomers, and geometric isomers (or conformational isomers): for example, R, S configurations that contain an asymmetric center, (Z), (E) isomers of double bond, and (Z), (E) conformational isomers. Therefore, the individual stereochemical isomers, diastereomers, or mixtures of geometric isomers (or conformational isomers) of the compound of the present invention all fall within the scope of the present invention.

Any asymmetric atom (e.g., carbon, etc.) of the compound disclosed in the present invention may exist in a racemic or enantioenriched form, such as (R), (*S*) or (R,S) configuration. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in the (R) or (*S*) configuration.

The term "prodrug" used in the present invention represents a compound that is converted into the compound represented by Formula (I) in vivo. Such conversion is affected by the hydrolysis of the prodrug in the blood or by the enzymatic conversion of the prodrug to the parent structure in the blood or tissue. The prodrug compound of the present invention can be an ester. In the existing invention, the ester that can be used as prodrug includes phenyl ester, aliphatic (C1-C24) ester, acyloxymethyl ester, carbonate ester, carbamate ester and amino acid ester.

"Metabolite" refers to a product obtained by metabolism of a specific compound or salt thereof in the body. The metabolites of a compound can be identified by techniques well known in the art, and the activity thereof can be characterized by assays as described herein. Such product can be obtained by oxidation, reduction, hydrolysis, amidation, deamidation, esterification, deesterification, enzymatic cleavage, etc., of the compound administered. Accordingly, the present invention comprises a metabolite of the compound, which comprises a metabolite produced by sufficiently contacting the compound of the present invention with a mammal for a period of time.

"Solvate" as used herein refers to an association complex of one or more solvent molecules with the compound of the present invention. Solvents that form the solvate include, but are not limited to, water, isopropanol, ethanol, methanol, dimethyl sulfoxide, ethyl acetate, acetic acid, and aminoethanol. The term "hydrate" refers to an association complex formed with water as solvent molecules.

The terms "halogen" and "halo" are used interchangeably in the present invention and refer to fluorine (F), chlorine (Cl), bromine (Br) or iodine (I).

The term "alkyl" used in the present invention refers to a saturated straight or branched monovalent hydrocarbonyl containing 1 to 20 carbon atoms (C1-C20 alkyl), wherein the alkyl can be independently optionally substituted with one or more substituents as described in the present invention. In some embodiments, the alkyl contains 1 to 12 carbon atoms (C1-C12 alkyl); in other embodiments, the alkyl contains 1 to 10 carbon atoms (C1-C10 alkyl); in other embodiments, the alkyl contains 1 to 8 carbon atoms (C1-C8 alkyl); in other embodiments, the alkyl contains 1 to 6 carbon atoms (C1-C6 alkyl); in other embodiments, the alkyl contains 1 to 4 carbon atoms (C1-C4 alkyl); in other embodiments, the alkyl contains 1 to 3 carbon atoms (C1-C3 alkyl); in other embodiments, the alkyl contains 1 to 2 carbon atoms (C1-C2 alkyl). Further examples of alkyl include, but are not limited to, methyl (Me, -CH₃), ethyl (Et, -CH₂CH₃), n-propyl (n-Pr, -CH₂CH₂CH₃), isopropyl (i-Pr, -CH(CH₃)₂), n-butyl (n-Bu, -CH₂CH₂CH₂CH₃), 2-methylpropyl or isobutyl (i-Bu, -CH₂CH(CH₃)₂), 1-methylpropyl or sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, etc.

The term "C1-C6 alkyl" refers to any alkyl containing 1 to 6 carbon atoms, such as methyl, ethyl, n-propyl, isopropyl, n-butyl, isobutyl, tert-butyl, sec-butyl, n-pentyl, tert-pentyl, n-hexyl, etc.

The term "alkenyl" refers to a straight or branched monovalent hydrocarbonyl containing 2 to 12 carbon atoms (C2-C12 alkenyl), or 2 to 8 carbon atoms (C2-C8 alkenyl), or 2 to 6 carbon atoms (C2-C6 alkenyl), or 2 to 4 carbon atoms (C2-C4 alkenyl), wherein at least one position of C-C is an sp2 double bond, which comprises "cis", "trans" or "Z", "E" isomers. Specific examples thereof include, but are not limited to, vinyl (-CH= CH₂), propenyl (-CH=CHCH₃), allyl (-CH₂CH=CH₂), etc.

The term "C2-C6 alkenyl" refers to any alkenyl containing 2 to 6 carbon atoms and containing at least one double bond, and examples thereof include vinyl, allyl, 1-propenyl, isopropenyl, 1-butenyl, 2-butenyl, 3-butenyl, 2-pentenyl, 1-hexenyl, etc.

The term "alkynyl" refers to a straight or branched monovalent hydrocarbonyl containing 2 to 12 carbon atoms (C2-C12 alkynyl), or 2 to 8 carbon atoms (C2-C8 alkynyl), or 2 to 6 carbon atoms (C2-C6 alkynyl), or 2 to 4 carbon atoms (C2-C4 alkynyl), in which at least one position of C-C is an sp triple bond. Specific examples thereof include, but are not limited to, ethynyl (-C=CH), propargyl (-CH₂C≡CH), propynyl (-C≡C-CH₃), 1-butynyl (-CH₂CH₂C≡CH), 2-butynyl (-CH₂C≡ CCH₃), 3-butynyl (-C≡CCH₂CH₃), etc.

The term "C2-C6 alkynyl" refers to any alkynyl containing 2 to 6 carbon atoms and containing at least one triple bond, such as ethynyl, 2-propynyl, 4-pentynyl, etc.

The term "alkoxy" refers to that an alkyl attached to the remainder of molecule through an oxygen atom, wherein the alkyl has the meaning described herein. Unless otherwise specified, the alkoxy contains 1 to 12 carbon atoms and may be represented as C1-C12 alkoxy. In some embodiments, the alkoxy contains 1 to 8 carbon atoms, which may be represented by C1-C8 alkoxy; in other embodiments, the alkoxy contains 1 to 6 carbon atoms, which may be represented by C1-C6 alkoxy; in other embodiments, the alkoxy contains 1 to 4 carbon atoms, which may be represented by C1-C4 alkoxy; in yet other embodiments, the alkoxy contains 1 to 3 carbon atoms, which may be represented by C1-C3 alkoxy. Examples of alkoxy include, but are not limited to, methoxy (MeO, -OCH₃), ethoxy (EtO, -OCH₂CH₃), 1-propoxy (n-PrO, n-propoxy, -OCH₂CH₂CH₃), 2-propoxy (i-PrO, i-propoxy, -OCH(CH₃)₂), 1-butoxy (n-BuO, n-butoxy, -OCH₂CH₂CH₂CH₃), 2-methyl-l-propoxy (i-BuO, i-butoxy, -OCH₂CH(CH₃)₂), 2-butoxy (s-BuO, s-butoxy, -OCH(CH₃)CH₂CH₃), 2-methyl-2-propoxy (t-BuO, t-butoxy, -OC(CH₃)₃), 1-pentyloxy (n-pentyloxy, -OCH₂CH₂CH₂CH₂CH₃), 2-pentyloxy (-OCH(CH₃)CH₂CH₂CH₃), 3-pentyloxy (-OCH(CH₂CH₃)₂), 2-methyl-2-butoxy (-OC(CH₃)₂CH₂CH₃), 3-methyl-2-butoxy (-OCH(CH₃)CH(CH₃)₂), 3-methyl-l-butoxy (-OCH₂CH₂CH(CH₃)₂), 2-methyl-l-butoxy (-OCH₂CH(CH₃))CH₂CH₃), etc.

The term "alkylamino" or "alkylamino group" comprises "N-alkylamino" and "*N*,*N*-dialkylamino", wherein the amino group is each independently substituted with one or two alkyl groups, wherein the alkyl has the meaning as described in the present invention. Suitable alkylamino may be monoalkylamino or dialkylamino, examples of which include, but are not limited to, N-methylamino, N-ethylamino, N,N-dimethylamino, N,N-diethylamino etc. The alkylamino is optionally substituted with one or more substituents described herein.

The term "haloalkoxy" means that an alkoxy is substituted with one or more halogen atoms, wherein the alkoxy has the meaning as described in the present invention, and such examples include, but are not limited to, -OCHF₂, -OCF₃, -OCHFCH₂F, -OCF₂CHF₂, -OCH₂CF₃, -OCHFCH₃, -OCH₂CH₂F, -OCF₂CH₃, -OCH₂CF₂CHF₂, etc. In one embodiment, C1-C6 haloalkoxy comprises fluorine-substituted C1-C6 alkoxy; in another embodiment, C1-C4 haloalkoxy comprises fluorine-substituted C1-C4 alkoxy; in yet another embodiments, C1-C2 haloalkoxy comprises fluorine-substituted C1-C2 alkoxy.

The term "cycloalkyl" refers to a saturated or partially unsaturated monocyclic or polycyclic cyclic hydrocarbon substituent, and the cycloalkyl ring contains 3 to 20 carbon atoms (i.e., "C3-C20 cycloalkyl"), preferably 3 to 12 carbon atoms (i.e., "C3-C12 cycloalkyl"), more preferably 3 to 10 carbon atoms (i.e., "C3-C10 cycloalkyl"), most preferably 3 to 7 carbon atoms (i.e., "C3-C7 cycloalkyl"). Non-limiting examples of monocyclic cycloalkyl (e.g., "C3-C7 cycloalkyl") include cyclopropyl, cyclobutyl, cyclopentyl, cyclopentenyl, cyclohexyl, cyclohexenyl, cyclohexadienyl, cycloheptyl, cycloheptatrienyl, etc.; and polycyclic cycloalkyl comprises spiro-, fused- and bridged-cycloalkyl.

The term "halogenated C1-C6 alkyl" refers to a group in which C1-C6 alkyl skeleton is substituted with one or more halogens, such as monofluoromethyl, difluoroethyl, trifluoromethyl, etc.

The term "halogen" refers to fluorine, chlorine, bromine, and iodine.

The term "C6-C12 aryl" refers to a group of a carbocyclic aromatic system having 6 to 12 carbon atoms.

The term "C6-C10 aryl" refers to a group of a carbocyclic aromatic system having 6 to 10 carbon atoms, such as phenyl, naphthyl, etc.

The term "heteroalkyl" refers to a straight or branched alkyl (preferably an alkyl having 2 to 14 or 3 to 7 carbon atoms), in which one or more carbon atoms are independently replaced by heteroatoms selected from the group consisting of S, O, P and N (i.e., "2- to 14-membered heteroalkyl" or "3- to 7-membered heteroalkyl"). Exemplary heteroalkyl groups include alkyl ethers, alkylamines, secondary alkylamines, thioethers, and the like.

The term "heterocyclyl" refers to a saturated or partially unsaturated monocyclic or polycyclic cyclic hydrocarbon substituent containing 3 to 20 ring atoms (i.e., a "3- to 20-membered heterocyclyl"), in which one or more ring atoms are heteroatoms selected from the group consisting of nitrogen, oxygen and S(O)ₘ (wherein m is an integer of 0 to 2), but it does not contain a ring part of -O-O-, -O-S- or -S-S-, and the remaining ring atoms are carbon atoms. Preferably, it contains 3 to 15 ring atoms (i.e., "3- to 15-membered heterocyclyl") or 3 to 12 ring atoms (i.e., "3- to 12-membered heterocyclyl"), of which 1 to 4 are heteroatoms; more preferably, the heterocyclyl contains 3 to 10 ring atoms (i.e., "3- to 10-membered heterocyclyl"). Non-limiting examples of monocyclic heterocyclyl (e.g., 3- to 7-membered heterocyclyl) include pyrrolidinyl, piperidinyl, piperazinyl, morpholinyl, thiomorpholinyl, homopiperazinyl, and the like. Polycyclic heterocyclyl includes spiro-, fused- or bridged-heterocyclyl.

The term "3- to 15-membered heterocyclyl" refers to 3-, 4-, 5-, 6- and 7-membered up to 15-membered saturated or partially unsaturated carbocyclic rings, in which one or more carbon atoms are replaced by heteroatoms such as nitrogen, oxygen and sulfur. "3- to 15-membered heterocyclyl" includes, for example, "3- to 7-membered heterocyclyl", and non-limiting examples thereof include, such as, thiacyclobutyl, pyran, pyrrolidine, pyrroline, imidazoline, imidazolidine, pyrazolidine, pyrazoline, thiazoline, thiazolidine, dihydrofuran, tetrahydrofuran, 1,3-dioxolane, piperidine, piperazine, morpholine, morpholinyl, tetrahydropyrrolyl, thiomorpholinyl, et al.

The term "polycyclic aromatic hydrocarbonyl" refers to a group formed by fusing 1 to 2 C5-C6 aromatic rings and 1 to 2 aromatic rings or non-aromatic rings together, and non-limiting examples thereof include, such as indenyl, naphthalenyl, phenanthrenyl, fluorenyl, etc.

Composite structures such as "-(C1-C6 alkyl)ₙ-O-(CO)-R^{b3}" refer to that any n C1-C6 alkyl groups (e.g., methyl, ethyl) and the like as described above are connected to R^{b3} through oxygen (-O-) and carbonyl (-(C=O)-). The meanings of other similar composite structures can be understood with reference to the foregoing contents.

Peptide: The amino group of one amino acid condenses with the carboxyl group of another amino acid to form a peptide; for example: C^{#}OOH-CH(N^{#}H₂)-CH₂-C^{#}OOH, which can form peptide bonds with other amino acids through one, two or three #-signed atoms.

Any structural formulas given herein are also intended to represent non-isotopically enriched as well as isotopically enriched forms of these compounds. Isotopically enriched compounds have the structure depicted by the general formula of the present invention, except that one or more atoms are replaced by atoms that have selected atomic weights or mass numbers. Exemplary isotopes that may be introduced into the compounds of the present invention include isotopes of hydrogen, carbon, nitrogen, oxygen, phosphorus, sulfur, fluorine and chlorine, such as ²H, ³H, ¹¹C , ¹³C , ¹⁴C, ¹⁵N, ¹⁷O, ¹⁸O, ¹⁸F , ³¹P, ³²P, ³⁵S, ³⁶Cl and ¹²⁵I.

In *N=CH-NH-CH=*C-CH₂-CH(NH₂)-C(=O)-, N and C are marked with *, indicating that the two atoms marked with * are single bonded.

### Abbreviations

Boc: tert-butoxycarbonyl
Cbz: benzyloxycarbonyl
Ph: phenyl
Fmoc: fluorenylmethoxycarbonyl
Trt: trityl
HO-p-Ph-CH₂-CH(NH₂)-C(=O)-: p-hydroxybenzylaminomethylcarbonyl

The present invention will be further explained and described below in conjunction with specific examples.

### Commercial sources of some known compounds:

| Compound | CAS | Supplier | Compound | CAS | Supplier |
|---|---|---|---|---|---|
| 1 | 13726-67-5 | Bidepharm | 24 | 7536-58-5 | Bidepharm |
| 2 | 1152-61-0 | Bidepharm | 25 | 4668-42-2 | Bidepharm |
| 3 | 4226-18-0 | Bidepharm | 26 | 98045-03-5 | Bidepharm |
| 4 | 17812-32-7 | Bidepharm | 27 | 132388-58-0 | Bidepharm |
| 5 | 145038-52-4 | Bidepharm | 28 | 4685-12-5 | Bidepharm |
| 6 | 16856-13-6 | Bidepharm | 29 | 19427-28-2 | Bidepharm |
| 7 | 32213-95-9 | Bidepharm | 30 | 997-55-7 | Bidepharm |
| 8 | 59768-74-0 | Bidepharm | 31 | 117833-18-8 | Bidepharm |
| 9 | 145038-53-5 | Bidepharm | 32 | 22839-61-8 | Bidepharm |
| 10 | 144120-53-6 | Bidepharm | 33 | 7175-34-0 | Bidepharm |
| 11 | 146982-24-3 | Bidepharm | 34 | 3790-52-1 | Bidepharm |
| 12 | 16115-68-7 | Bidepharm | 38 | 80863-62-3 | Jingdong (JD.com) |
| 13 | 3057-74-7 | Bidepharm | 39 | 13433-09-5 | Bidepharm |
| 14 | 4125-93-3 | Bidepharm | 40 | 85227-98-1 | Bidepharm |
| 15 | 2673-19-0 | Bidepharm | 41 | 1791-13-5 | Bidepharm |
| 16 | 5545-52-8 | Bidepharm | 42 | 52615-97-1 | Bidepharm |
| 17 | 1676-90-0 | Bidepharm | 43 | 23220-52-2 | Bidepharm |
| 18 | 71989-14-5 | Bidepharm | 44 | 34582-30-4 | Bidepharm |
| 19 | 152548-66-8 | Bidepharm | 45 | 6853-87-8 | Bidepharm |
| 20 | 7362-93-8 | Bidepharm | 46 | 207121-48-0 | Bidepharm |
| 21 | 30925-18-9 | Bidepharm | 47 | 56-86-0 | Bidepharm |
| 22 | 2177-63-1 | Bidepharm | 48 | 22839-47-0 | Bidepharm |
| 23 | 112259-66-2 | Bidepharm | | | |

### Synthesis examples

### Example 1: Synthesis of Compound 35

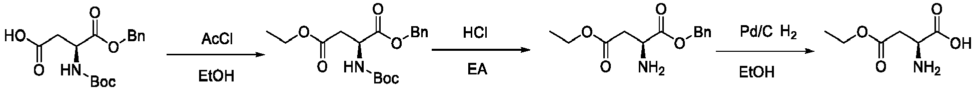

### Step 1: Synthesis of 1-benzyl 4-ethyl (tert-butoxycarbonyl)-L-aspartic acid ester

(S)-4-(Benzyloxy)-3-((tert-butoxycarbonyl)amino)-4-oxobutyric acid (1 g, 3.096 mmol) was dissolved in dichloromethane (10 mL), added dropwise with acetyl chloride (490 mg, 6.19 mmol) to the reaction system, and the reaction solution was stirred at room temperature for 1 hour. The reaction solution was concentrated, then diluted by adding dichloromethane, the above solution was added dropwise into absolute ethanol (10 ml), and the reaction solution was stirred at room temperature for 1 hour. After it was detected that the reaction was completed, the reaction solution was washed with water, and the organic phase was separated, dried, and concentrated to obtain 900 mg of 1-benzyl 4-ethyl (tert-butoxycarbonyl)-L-aspartic acid ester. MS m/z (ESI): 352 [M+H]⁺.

### Step 2: Synthesis of 1-benzyl 4-ethyl L-aspartic acid ester

1-Benzyl 4-ethyl (tert-butoxycarbonyl)-L-aspartic acid ester (900 mg, 2.56 mmol) was dissolved in ethyl acetate (5 mL) at room temperature, and a solution of hydrogen chloride in ethyl acetate (2M) (7.7 ml, 15.38 mmol) was added to the reaction system, the reaction solution was allowed to react at room temperature for 1 hour. After it was monitored by TLC that the reaction was completed, the reaction solution was concentrated to obtain 600 mg of 1-benzyl 4-ethyl L-aspartic acid ester. MS m/z (ESI): 252[M+H]⁺.

### Step 3: Synthesis of 4-ethyl L-aspartic acid ester

1-Benzyl 4-ethyl L-aspartic acid ester (600 mg, 2.39 mmol) was dissolved in ethanol (5 mL), added with palladium-on-carbon (120 mg), stirred and reacted at room temperature under a hydrogen atmosphere for 16 h. After it was monitored that the reaction was completed, the palladium-on-carbon was filtered out using diatomaceous earth, and the filtrate was concentrated to obtain 200 mg of 4-ethyl L-aspartic acid ester (Compound 35). MS m/z (ESI): 162[M+H]⁺. ¹H NMR (400 MHz, DMSO-d6) δ 13.92 (s, 1H), 8.49 (s, 2H), 4.17 (t, *J =* 5.6 Hz, 1H), 4.13 - 4.06 (m, 2H), 2.98 - 2.86 (m, 2H), 1.18 (t, *J=* 7.1 Hz, 3H).

### Example 2: Synthesis of Compound 36

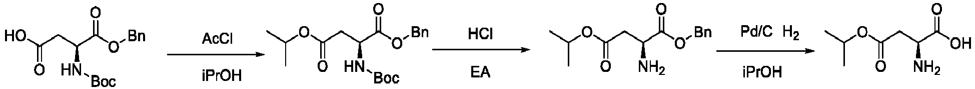

### Step 1: Synthesis of 1-benzyl 4-isopropyl (tert-butoxycarbonyl)-L-aspartic acid ester

(*S*)-4-(Benzyloxy)-3-((tert-butoxycarbonyl)amino)-4-oxobutyric acid (1 g, 3.096 mmol) was dissolved in dichloromethane (10 mL), acetyl chloride (490 mg, 6.19 mmol) was added dropwise to the reaction system, and the reaction solution was stirred at room temperature for 1 hour. The reaction solution was concentrated and diluted with dichloromethane. The above solution was added dropwise to isopropanol (10 ml), and the reaction solution was stirred at room temperature for 1 hour. After it was detected by TLC that the reaction was completed, the reaction solution was washed with water, and the organic phase was separated, dried and concentrated to obtain 850 mg of 1-benzyl 4-isopropyl (tert-butoxycarbonyl)-L-aspartic acid ester. MS m/z (ESI): 366 [M+H]⁺.

### Step 2: Synthesis of 1-benzyl 4-isopropyl L-aspartic acid ester

1-Benzyl 4-isopropyl (tert-butoxycarbonyl)-L-aspartic acid ester (860 mg, 2.35mmol) was dissolved in ethyl acetate (5 mL) at room temperature, then a solution of hydrogen chloride in ethyl acetate(2M) (7.07 ml, 14.14 mmol) was added, and the solution was reacted at room temperature for 1 hour. After it was monitored by TLC that the reaction was completed, the reaction solution was concentrated to obtain 500 mg of 1-benzyl 4-isopropyl L-aspartic acid ester. MS m/z (ESI): 266[M+H]⁺.

### Step 3: Synthesis of 4-isopropyl L-aspartic acid ester

1-Benzyl 4-isopropyl L-aspartic acid ester (500 mg, 1.88 mmol) was dissolved in ethanol (5 mL), added with palladium-on-carbon (100 mg), stirred and reacted at room temperature under a hydrogen atmosphere for 16 h. After it was monitored that the reaction was completed, the palladium-on-carbon was filtered out using diatomaceous earth, and the filtrate was concentrated to obtain 200 mg of 4-isopropyl L-aspartic acid ester (Compound 36). MS m/z (ESI): 176 [M+H]⁺. ¹H NMR (400 MHz, DMSO-d6) δ 8.90 (s, 2H), 4.99 - 4.93 (m, 1H), 4.17 (t, *J=* 5.2 Hz, 1H), 2.92 - 2.89 (m, 2H), 1.18 (dd, *J* = 6.2, 1.5 Hz, 6H).

### Example 3: Synthesis of Compound 37

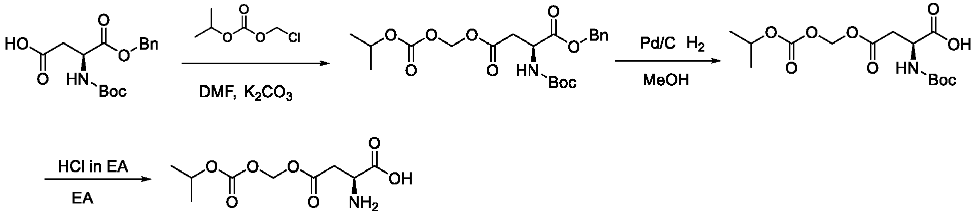

### Step 1: Synthesis of 1-benzyl 4-(((isopropoxycarbonyl)oxy)methyl) (tert-butoxycarbonyl)-L-aspartic acid ester

(S)-4-(Benzyloxy)-3-((tert-butoxycarbonyl)amino)-4-oxobutyric acid (2 g, 6.19 mmol), chloromethyl isopropyl carbonate (1.47 g, 9.29 mmol) and potassium carbonate (1.7 g, 12.38 mmol) were dissolved in DMF (10 mL), and the reaction solution was stirred at room temperature for 16 hours. After the reaction was completed, the reaction solution was diluted by adding water, extracted with ethyl acetate, then the reaction solution was washed with brine, and the organic phase was separated, dried and concentrated to obtain 900 mg of 1-benzyl 4-(((isopropoxycarbonyl)oxy)methyl) (tert-butoxycarbonyl)-L-aspartic acid ester. MS m/z (ESI): 440 [M+H]⁺.

### Step 2: Synthesis of (S)-2-((tert-butoxycarbonyl)amino)-4-(((isopropoxycarbonyl)oxy) methoxy)-4-oxobutyric acid

1-Benzyl 4-(((isopropoxycarbonyl)oxy)methyl) (tert-butoxycarbonyl)-L-aspartic acid ester (900 mg, 2.05mmol) was dissolved in methanol (5 mL), added with palladium-on-carbon (180 mg), stirred and reacted for 16 h at room temperature under a hydrogen atmosphere. After it was monitored by TLC that the reaction was completed, the palladium-on-carbon was filtered out using diatomaceous earth, and the filtrate was concentrated to obtain 500 mg of (S)-2-((tert-butoxycarbonyl)amino)-4-(((isopropoxycarbonyl)oxy)methoxy)-4-oxobutyric acid. MS m/z (ESI): 350 [M+H]⁺.

### Step 3: Synthesis of 4-(((isopropoxycarbonyl)oxy)methyl)-L-aspartic acid

(*S*)-2-((Tert-butoxycarbonyl)amino)-4-(((isopropoxycarbonyl)oxy)methoxy)-4-oxobutyric acid (500 mg, 1.43 mmol) was dissolved in ethyl acetate (5 mL), a solution of hydrogen chloride in ethyl acetate (2M) (4.3 ml, 8.6 mmol) was added, and the solution was reacted at room temperature for 1 hour. After it was monitored by TLC that the reaction was completed, the reaction solution was concentrated, water was added to dilute the reaction solution, and sodium bicarbonate was added to adjust the pH of the solution to neutral. The mixture was extracted with ethyl acetate, and the organic phase was separated. The organic phase was dried over anhydrous sodium sulfate and concentrated under reduced pressure to obtain 100 mg of 4-(((isopropoxycarbonyl)oxy)methyl)-L-aspartic acid (Compound **37**). ¹H NMR (300 MHz, DMSO-*d6*) δ 13.93 (s, 1H), 8.66 (s, 2H), 5.69 (d, *J* = 12.8 Hz, 2H), 4.79 (h, *J* = 6.2 Hz, 1H), 4.18 (s, 1H), 3.16 - 2.94 (m, 2H), 1.23 (d, *J* = 6.2 Hz, 6H). MS m/z (ESI): 250[M+H]⁺.

### Example 4: Synthesis of Compound 49

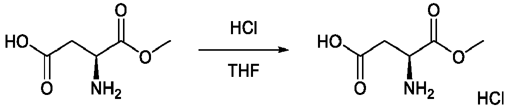

### Synthesis of (S)-3-amino-4-methoxy-4-oxobutyric acid hydrochloride

(*S*)-3-Amino-4-methoxy-4-oxobutyric acid (100 mg, 0.68 mmol) was dissolved in tetrahydrofuran (3 ml), added with a solution of hydrogen chloride in ethyl acetate (0.34 ml, 0.68 mmol), and stirred at room temperature for 30 minutes, and then solid precipitation could be observed. The mixture was filtered to obtain the filter residue, and the filter residue was dried to obtain 80 mg of (*S*)-3-amino-4-methoxy-4-oxobutyric acid hydrochloride (Compound **49**). ¹H NMR (300 MHz, DMSO-*d6*) δ 9.60 (s, 1H), 4.23 (t, *J* = 5.2 Hz, 1H), 3.70 (s, 3H), 2.94 (d, *J* = 5.2 Hz, 2H). MS m/z (ESI): 184[M+H]⁺.

### Example 5: Synthesis of Compound 50

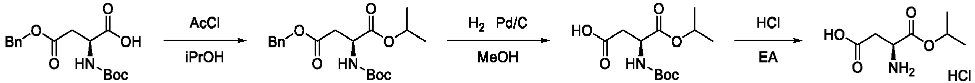

### Step 1: Synthesis of 4-benzyl 1-isopropyl (tert-butoxycarbonyl)-L-aspartic acid ester

(S)-4-(Benzyloxy)-2-((tert-butoxycarbonyl)amino)-4-oxobutyric acid (1 g, 3.096 mmol) was dissolved in dichloromethane (10 mL), acetyl chloride (490 mg, 6.19 mmol) was added dropwise to the reaction system, and the reaction solution was stirred at room temperature for 1 hour. The reaction solution was concentrated, diluted by adding dichloromethane, the above solution was added dropwise to isopropanol (10 ml), and the reaction solution was stirred at room temperature for 1 hour. After it was detected by TLC that the reaction was completed, the reaction solution was washed with water, and the organic phase was separated, dried, and concentrated to obtain 840 mg of 4-benzyl 1-isopropyl (tert-butoxycarbonyl)-L-aspartic acid ester. MS m/z (ESI): 366 [M+H]⁺.

### Step 2: Synthesis of (S)-3-((tert-butoxycarbonyl)amino)-4-isopropoxy-4-oxobutyric acid

4-Benzyl 1-isopropyl (tert-butoxycarbonyl)-L-aspartic acid ester (840 mg, 2.3 mmol) was dissolved in ethanol (5 mL), added with palladium-on-carbon (160 mg), stirred and reacted at room temperature for 16 h under a hydrogen atmosphere. After it was monitored that the reaction was completed, the palladium-on-carbon was filtered out using diatomaceous earth, and the filtrate was concentrated to obtain 300 mg of (S)-3-((tert-butoxycarbonyl)amino)-4-isopropoxy-4-oxobutyric acid. MS m/z (ESI): 276 [M+H]⁺.

### Step 3: Synthesis of 1-isopropyl L-aspartic acid ester hydrochloride

(S)-3-((Tert-butoxycarbonyl)amino)-4-isopropoxy-4-oxobutyric acid (300 mg, 1.1mmol) was dissolved in ethyl acetate (5 mL) at room temperature, a solution of hydrogen chloride in ethyl acetate(2M) (3.3 ml, 6.55 mmol) was added to the reaction system, and the solution was reacted at room temperature for 1 hour. After it was monitored by TLC that the reaction was completed, the reaction solution was concentrated to obtain 130 mg of 1-isopropyl L-aspartic acid ester hydrochloride (Compound **50**). ¹H NMR (300 MHz, DMSO-*d6*) δ 13.00 (s, 1H), 8.58 (s, 2H), 4.97 (p, *J* = 6.3 Hz, 1H), 4.19 (t, *J* = 5.2 Hz, 1H), 2.90 (dd, *J* = 5.2, 1.8 Hz, 2H), 1.20 (dd, *J* = 10.2, 6.2 Hz, 6H). MS m/z (ESI): 212[M+H]⁺.

### Example 6: Synthesis of Compound 51

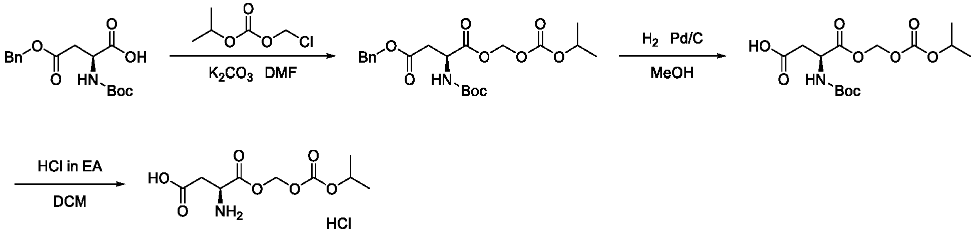

### Step 1: Synthesis of 4-benzyl 1-(((isopropoxycarbonyl)oxy)methyl) (tert-butoxycarbonyl)-L-aspartic acid ester

(*S*)-4-(Benzyloxy)-2-((tert-butoxycarbonyl)amino)-4-oxobutyric acid (10 g, 30.96 mmol), chloromethyl isopropyl carbonate (5.65 g, 37.15 mmol) and potassium carbonate (8.54 g, 61.92 mmol) were dissolved in DMF (100 mL), and the reaction solution was stirred at room temperature for 16 hours. After the reaction was completed, the reaction solution was diluted by adding water, extracted with ethyl acetate, washed with brine, and the organic phase was separated, dried and concentrated to obtain 4.2 g of 4-benzyl 1-(((isopropoxycarbonyl)oxy)methyl) (tert-butoxycarbonyl)-L-aspartic acid ester. MS m/z (ESI): 440 [M+H]⁺.

### Step 2: Synthesis of (S)-3-((tert-butoxycarbonyl)amino)-4-(((isopropoxycarbonyl)oxy) methoxy)-4-oxobutyric acid

4-Benzyl 1-(((isopropoxycarbonyl)oxy)methyl) (tert-butoxycarbonyl)-L-aspartic acid ester (4.2 g, 9.57mmol) was dissolved in methanol (50 mL), added with palladium-on-carbon (810 mg), stirred and reacted for 16 h at room temperature under a hydrogen atmosphere. After it was monitored that the reaction was completed, the palladium-on-carbon was filtered out using diatomaceous earth, and the filtrate was concentrated to obtain 3 g of (S)-3-((tert-butoxycarbonyl)amino)-4-(((isopropoxycarbonyl)oxy)methoxy methyl)-4-oxobutyric acid. MS m/z (ESI): 350 [M+H]⁺.

### Step 3: Synthesis of 1-(((isopropoxycarbonyl)oxy)methyl)L-aspartic acid ester hydrochloride

At room temperature, (S)-3-((tert-butoxycarbonyl)amino)-4-(((isopropoxycarbonyl)oxy) methoxy)-4-oxobutyric acid (3 g, 8.60 mmol) was dissolved in ethyl acetate (30 mL), and added with a solution of hydrogen chloride in ethyl acetate (2M) (25.8 ml, 51.58 mmol), and the solution was reacted at room temperature for 1 hour. After it was monitored by TLC that the reaction was completed, the reaction solution was concentrated to obtain 1.2 g of 1-(((isopropoxycarbonyl)oxy)methyl)L-aspartic acid ester hydrochloride (Compound **51**). ¹H NMR (400 MHz, DMSO-d6) δ 8.80 (s, 2H), 5.79 - 5.68 (m, 2H), 4.80 (p, *J* = 6.2 Hz, 1H), 4.34 (s, 1H), 2.97 (d, *J* = 5.1 Hz, 2H), 1.23 (d, *J* = 6.2 Hz, 6H). MS m/z (ESI): 286[M+H]⁺.

### Example 7: Synthesis of Compound 52

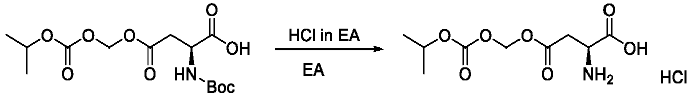

### Synthesis of 4-(((isopropoxycarbonyl)oxy)methyl) L-aspartic acid ester hydrochloride

(*S*)-2-((Tert-butoxycarbonyl)amino)-4-(((isopropoxycarbonyl)oxy)methoxy)-4-oxobutyric acid (500 mg, 1.43 mmol) was dissolved in ethyl acetate (5 mL), added with a solution of hydrogen chloride in ethyl acetate (2M) (4.3 ml, 8.6 mmol), and the solution was reacted at room temperature for 1 hour. After it was monitored by TLC that the reaction was completed, the reaction solution was concentrated to obtain 130 mg of 4-(((isopropoxycarbonyl)oxy)methyl) L-aspartic acid ester hydrochloride (Compound **52**). ¹H NMR (300 MHz, DMSO-d6) δ 13.93 (s, 1H), 8.66 (s, 2H), 5.69 (d, *J* = 12.8 Hz, 2H), 4.79 (h, *J* = 6.2 Hz, 1H), 4.18 (s, 1H), 3.16 - 2.94 (m, 2H), 1.23 (d, *J* = 6.2 Hz, 6H). MS m/z (ESI): 286[M+H]⁺.

### Example 8: Synthesis of Compound 53

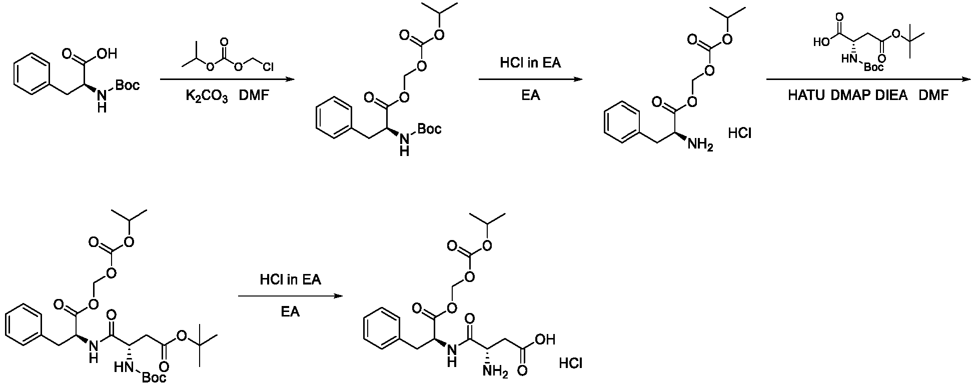

### Step 1: Synthesis of ((isopropoxycarbonyl)oxy)methyl ester (tert-butoxycarbonyl)-L-phenylalanine

(Tert-butoxycarbonyl)-L-phenylalanine (2 g, 7.55 mmol), chloromethyl isopropyl carbonate (1.38 g, 9.06 mmol) and potassium carbonate (2.08 g, 15.09 mmol) were dissolved in DMF (10 mL), the reaction solution was stirred at room temperature for 16 hours. After the reaction was completed, the reaction solution was diluted by adding water, extracted with ethyl acetate, then the reaction solution was washed with brine, and the organic phase was separated, dried, and concentrated to obtain 550 mg of ((isopropoxycarbonyl)oxy)methyl ester (tert-butoxycarbonyl)-L-phenylalanine. MS m/z (ESI): 382[M+H]⁺.

### Step 2: Synthesis of ((isopropoxycarbonyl)oxy)methyl ester L-phenylalanine hydrochloride

((Isopropoxycarbonyl)oxy)methyl ester (tert-butoxycarbonyl)-L-phenylalanine (550 mg, 1.44 mmol) was dissolved in ethyl acetate (5 mL) at room temperature, then added with a solution of hydrogen chloride in ethyl acetate (2M) (4.33 ml, 8.66 mmol), and the above reaction solution was allowed to react at room temperature for 1 hour. After it was monitored by TLC that the reaction was completed, the reaction solution was concentrated to obtain 416 mg of ((isopropoxycarbonyl)oxy)methyl ester L-phenylalanine hydrochloride. MS m/z (ESI): 318[M+H]⁺.

### Step 3: Synthesis of tert-butyl (S)-3-((tert-butoxycarbonyl)amino)-4-(((S)-1-(((isopropoxycarbonyl)oxy)methoxy)-1-oxo-3-phenylprop-2-yl)amino)-4-oxobutyrate

((Isopropoxycarbonyl)oxy)methyl ester L-phenylalanine hydrochloride (416 mg, 1.3 mmol), (S)-4-(tert-butoxy)-2-((tert-butyloxycarbonyl)amino)-4-oxobutyric acid (416 mg, 1.44 mmol), HATU (994 mg, 2.62 mmol) and DMAP (16 mg, 0.13 mmol) were dissolved in DMF (5 mL), added dropwise with DIPEA (506 mg, 3.9mmol), stirred and reacted at room temperature for 2 h. After it was monitored by TLC that the reaction was completed, the reaction solution was diluted by adding water, extracted with ethyl acetate (5 mL*2), and separated to obtain an organic phase. The organic phase was dried, concentrated, and purified by column chromatography to obtain 500 mg of tert-butyl (*S*)-3-((tert-butoxycarbonyl)amino)-4-(((*S*)-1-(((isopropoxycarbonyl)oxy) methoxy)-1-oxo-3-phenylpropan-2-yl)amino)-4-oxobutyrate. MS m/z (ESI): 553[M+H]⁺.

### Step 4: Synthesis of (S)-3-amino-4-(((S)-1-(((isopropoxycarbonyl)oxy)methoxy)-1-oxo-3-phenylpropan- 2-yl)amino)-4-oxobutyric acid hydrochloride

Tert-butyl (*S*)-3-((tert-butoxycarbonyl)amino)-4-(((*S*)-1-(((isopropoxycarbonyl)oxy) methoxy)-1-oxo-3-phenylpropan-2-yl)amino)-4-oxobutyrate (500 mg, 0.91 mmol) was dissolved in ethyl acetate (5 mL), and added with a solution of hydrogen chloride in ethyl acetate (2M) (2.72 ml, 5.43 mmol), and the above reaction solution was reacted at room temperature for 1 hour. After it was monitored by TLC that the reaction was completed, the reaction solution was concentrated to obtain 320 mg of (*S*)-3-amino-4-(((*S*)-1-(((isopropoxycarbonyl)oxy)methoxy)-1-oxo-3-phenylpropan-2-yl)amino)-4-oxobutyric acid hydrochloride (Compound 53). ¹H NMR (400 MHz, DMSO-d6) δ 12.86 (s, 1H), 9.17 (d, *J* = 7.3 Hz, 1H), 8.31 (s, 2H), 7.31 - 7.22 (m, 5H), 5.74 - 5.63 (m, 2H), 4.81 (hept, *J* = 6.2 Hz, 1H), 4.56 (ddd, *J* = 8.6, 7.3, 5.6 Hz, 1H), 4.10 - 4.03 (m, 1H), 3.09 - 2.95 (m, 2H), 2.89 - 2.69 (m, 2H), 1.24 (d, *J* = 6.2 Hz, 6H). MS m/z (ESI): 433[M+H]⁺.

### Example 9: Synthesis of Compound 54

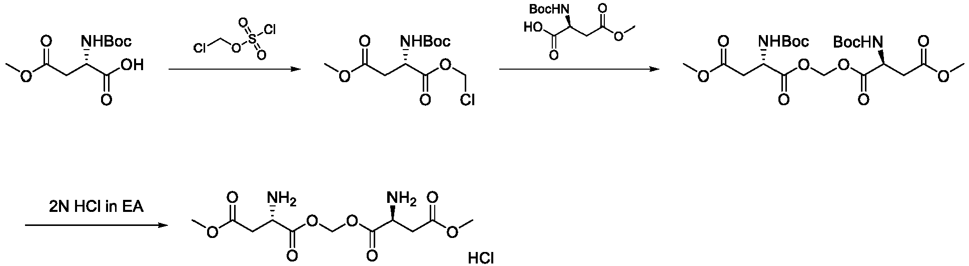

### Step 1: Synthesis of 1-(chloromethyl) 4-methyl (tert-butoxycarbonyl)-L-aspartic acid ester

Boc-L-aspartic acid 4-methyl ester (1.2 g, 5.0 mmol) and tetrabutylammonium bisulfate (84.0 mg, 0.3 mmol) were dissolved in dichloromethane (5 mL), placed in an ice bath, stirred and cooled to 0°C. A solution of potassium carbonate (2.7 g, 20.0 mmol) in water (5 mL) and a solution of chloromethyl chlorosulfonate (1.0 g, 6.0 mmol) in dichloromethane (5 mL) were added dropwise to the above reaction system in sequence, stirred and slowly warmed to room temperature, and stirred overnight. After it was monitored by TLC that the reaction was completed, 5 mL of dichloromethane was added, washing was performed with water three times, and the organic phase was separated. The organic phase was dried over anhydrous sodium sulfate, filtered, concentrated, and purified by column chromatography (EA/PE gradient elution, 20:1 to 5:1) to obtain 1.1 g of 1-(chloromethyl) 4-methyl (tert-butoxycarbonyl)-L-aspartic acid ester (colorless oil, yield 83%).

### Step 2: Synthesis of 4-dimethyl O'1,O1-methylene (2S,2'S)-bis(2-((tert-butoxycarbonyl)amino)succinate)

Sodium iodide (174 mg, 1.1 mmol) and potassium carbonate (276 mg, 2.0 mmol) were dissolved in DMF (1.5 mL), placed in an ice bath, stirred and cooled to 0 °C. Boc-L-aspartic acid 4-methyl ester (296.7 mg, 1.2 mmol) was dissolved in DMF (1.0 mL) and added dropwise into the above reaction system, placed in an ice bath and stirred for 30 minutes. 1-(Chloromethyl) 4-methyl (tert-butoxycarbonyl)-L-aspartic acid ester (295.7 mg, 1.0 mmol) was dissolved in DMF (1.0 mL) and added dropwise into the reaction system, stirred and slowly warmed to room temperature, and stirred about overnight, and the reaction was monitored by TLC. After the reaction of the raw materials was completed, 10 mL of water was added, extracted with ethyl acetate three times, and washing was performed with water three times. The organic phases were separated and combined, dried over anhydrous sodium sulfate, filtered, concentrated, and purified by column chromatography (EA/PE gradient elution, 10:1 to 3:1) to obtain 60 mg of 4-dimethyl O'1,O1-methylene (2S,2'S)-bis(2-((tert-butoxycarbonyl)amino)succinate) (white solid, yield 12%).

### Step 3: Synthesis of 4-dimethyl O'1,O1-methylene (2S,2'S)-bis(2-aminosuccinate) hydrochloride

4-Dimethyl O'1,O1-methylene (2S,2'S)-bis(2-((tert-butoxycarbonyl)amino)succinate) (1.0 g, 2.0 mmol) was dissolved in dichloromethane (6 mL), placed in an ice bath, stirred and cooled to 0°C, added dropwise with 2N solution of hydrochloric acid in ethyl acetate (12 mL), stirred and slowly warmed to room temperature, then stirred for about 2 hours. After it was monitored by HPLC that the reaction was completed, the reaction solution was filtered by suction, the filter cake was washed with a small amount of ethyl acetate, and the filter cake was dried to obtain 530 mg of 4-dimethyl O'1,O1-methylene (2S,2'S)-bis(2-aminosuccinate) hydrochloride (Compound **54**) (white solid, yield 60%). ¹H NMR (400 MHz, CD3OD), δ 5.99 (s, 2H), 4.52 (t, *J=* 6 Hz, 2H), 3.77 (s, 6H), 3.10 (m, 4H). MS m/z (ESI) :343[M+H]⁺.

### Example 10: Synthesis of Compound 56

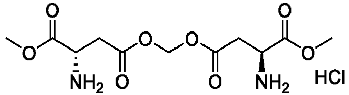

For the synthesis method of the titled compound, the synthesis of Compound **54** in Example 9 of the present application was referred to. Boc-L-aspartic acid 4-methyl ester was replaced by *N*-tert-butoxycarbonyl-L-aspartic acid 1-methyl ester in step 1 and step 2 to obtain 1-dimethyl O'4,O4-methylene (2S,2'S)-bis(2-aminosuccinate) hydrochloride (Compound **56**), yield 46%, as a white solid. ¹H NMR (400 MHz, CD3OD), δ 5.88 (s, 2H), 4.45 (t, *J* = 8 Hz, 2H), 3.85 (s, 6H), 3.02 (m, 4H). MS m/z (ESI) :343[M+H]⁺.

### Example 11: Synthesis of Compound 57

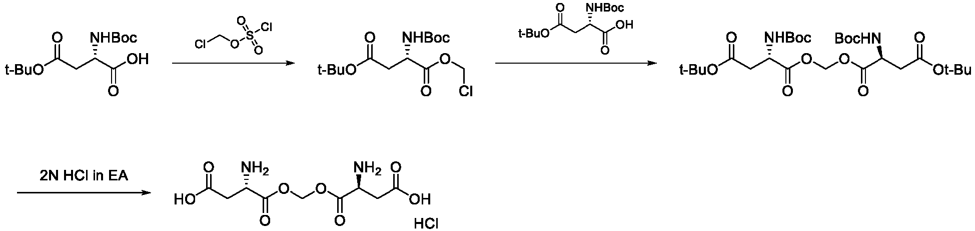

### Step 1: Synthesis of 1-(chloromethyl) 4-n-butyl (tert-butoxycarbonyl)-L-aspartic acid ester

Tert-butoxycarbonyl-L-aspartic acid 4-tert-butyl ester (2.3 g, 8.0 mmol) and tetrabutylammonium bisulfate (136 mg, 0.4 mmol) were dissolved in dichloromethane (8 mL), placed in an ice bath, stirred and cooled to 0°C. A solution of potassium carbonate (4.4 g, 32 mmol) in water (8 mL) and a solution of chloromethyl chlorosulfonate (1.7 g, 10.0. mmol) in dichloromethane (8 mL) were added dropwise to the reaction system in sequence, stirred and slowly warmed to room temperature overnight. After it was monitored by TLC that the reaction was completed, 8 mL of dichloromethane was added, washing was performed with water three times, and the organic phase was separated. The organic phase was dried over anhydrous sodium sulfate, filtered, concentrated, and purified by column chromatography (EA/PE gradient elution, 20:1 to 5:1) to obtain 1.4 g of 1-(chloromethyl) 4-n-butyl (tert-butoxycarbonyl)-L-aspartic acid ester (colorless oil, yield 53%).

### Step 2: Synthesis of 4-di(n-butyl) O'1,O1-methylene (2S,2'S)-bis(2-((tert-butoxycarbonyl) amino) succinate)

Sodium iodide (349 mg, 2.1 mmol) and potassium carbonate (552 mg, 4.0 mmol) were dissolved in DMF (2 mL), placed in an ice bath, stirred and cooled to 0°C. 1-(Chloromethyl) 4-n-butyl (tert-butoxycarbonyl)-L-aspartic acid ester (694.0 mg, 2.4 mmol) was dissolved in DMF (2 mL) and added dropwise into the above reaction system, placed in an ice bath and stirred for 30 minutes. 1-(Chloromethyl) 4-n-butyl (tert-butoxycarbonyl)-L-aspartic acid ester (676.0 mg, 2.0 mmo) was dissolved in DMF (2 mL) and added dropwise into the reaction system, stirred and slowly warmed to room temperature, and stirred for about overnight. After it was monitored by TLC that the reaction was completed, 10 mL of water was added, extracted with ethyl acetate three times, then the organic phase was separated and washed with water, the organic phases were combined, dried over anhydrous sodium sulfate, filtered, concentrated, and purified by a chromatographic column (EA/PE gradient elution, 10:1 to 3:1) to obtain 619 mg of 4-di(n-butyl) O'1,O1-methylene (2S,2'S)-bis(2-((tert-butoxycarbonyl)amino)succinate) (white solid, yield 52%).

### Step 3: Synthesis of (3S,3'S)-4,4'-(methylenebis(oxy))bis(3-amino-4-oxobutyric acid) hydrochloride

4-Di(n-butyl) O'1,O1-methylene (2S,2'S)-bis(2-((tert-butoxycarbonyl)amino)succinate) (295.0 mg, 0.5 mmol) was dissolved in dichloromethane (2 mL), placed in an ice bath, stirred and cooled to 0°C, added dropwise with 2N solution of hydrochloric acid in ethyl acetate (6 mL), stirred and slowly warmed to room temperature and stirred for about 2 hours. After it was monitored by HPLC that the reaction was completed, the reaction solution was filtered by suction, the filter cake was washed with a small amount of ethyl acetate, and the filter cake was dried to obtain 95 mg of (3S,3'S)-4,4'-(methylenebis(oxy))bis(3-amino-4-oxobutyric acid) hydrochloride (Compound **57**) (white solid, yield 61%). ¹H NMR (400 MHz, CD3OD), δ 5.99 (s, 2H), 4.16 (t, *J=* 4 Hz, 2H), 3.05 (m, 4H). MS m/z (ESI): 315[M+H]⁺.

### Example 12: Synthesis of Compound 55

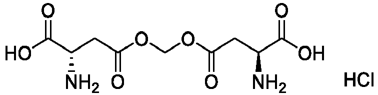

For the synthesis method of the titled compound, the synthesis of Compound **57** in Example 11 of the present application was referred to. Tert-butoxycarbonyl-L-aspartic acid 4-tert-butyl ester was replaced by N-tert-butoxycarbonyl-L-aspartic acid 1-tert-butyl ester in step 1 and step 2 to obtain (2S,2'S)-4,4'-(methylenebis(oxy)) bis(2-amino-4-oxobutyric acid) hydrochloride (Compound **55**) (yield 54%, white solid). ¹H NMR (400 MHz, CD3OD), δ 5.89 (s, 2H), 4.36 (dd, *J*₁ = 8 Hz, *J*₂ = 4 Hz, 2H), 3.12 (m, 4H). MS m/z (ESI): 315 [M+H]⁺.

### Example 13: Synthesis of Compound 58

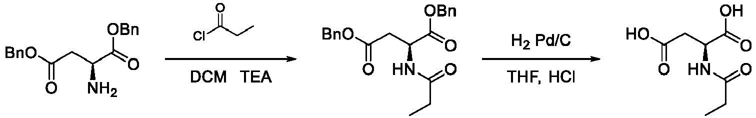

### Step 1: Synthesis of dibenzyl propionyl-L-aspartic acid ester

L-Aspartic acid dibenzyl ester (1 g, 3.19 mmol) and propionyl chloride (353 mg, 3.83 mmol) were dissolved in dichloromethane (10 mL), triethylamine (0.88 mL, 6.39 ml) was added dropwise into the reaction system, and the reaction solution was stirred at room temperature for 2 h. The reaction solution was added with an appropriate amount of water, and extracted with dichloromethane. The organic phase was separated. The organic phase was washed with water, dried, concentrated, and purified by column chromatography to obtain 980 mg of dibenzyl propionyl-L-aspartic acid ester. MS m/z (ESI): 370[M+H]⁺.

### Step 2: Synthesis of propionyl-L-aspartic acid

Dibenzyl propionyl-L-aspartic acid ester (200 mg, 0.54 mmol) and palladium-on-carbon (40 mg) were dissolved in tetrahydrofuran (3 mL), added with two drops of concentrated hydrochloric acid, and the reaction solution was stirred at room temperature for 16 h under a hydrogen atmosphere. After it was monitored that the reaction was completed, the palladium-on-carbon was filtered out using diatomaceous earth, and the organic phase was concentrated to obtain 80 mg of propionyl-L-aspartic acid (Compound **58**). ¹H NMR (500 MHz, DMSO-*d6*) δ 12.48 (s, 2H), 8.07 (d, *J* = 8.0 Hz, 1H), 4.51 (td, *J* = 7.5, 5.7 Hz, 1H), 2.69 - 2.51 (m, 2H), 2.09 (q, *J* = 7.6 Hz, 2H), 0.96 (t, *J* = 7.6 Hz, 3H). MS m/z (ESI): 190 [M+H]⁺.

### Example 14: Synthesis of Compound 59

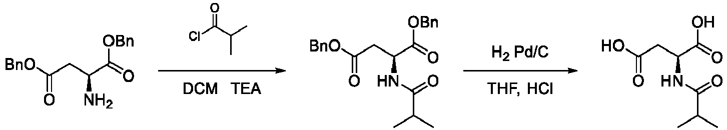

### Step 1: Synthesis of dibenzyl isobutyryl-L-aspartic acid ester

L-aspartic acid dibenzyl ester (1 g, 3.19 mmol) and isobutyryl chloride (406 mg, 3.83mmol) were dissolved in dichloromethane (10 mL), then added dropwise with triethylamine (0.88 mL, 6.39 ml), and the reaction solution was stirred at room temperature for 2 h. The reaction solution was added with an appropriate amount of water, and extracted with dichloromethane. The organic phase was separated. The organic phase was washed with water, dried, concentrated, and purified by column chromatography to obtain 960 mg of dibenzyl isobutyryl-L-aspartic acid ester. MS m/z (ESI): 384[M+H]⁺.

### Step 2: Synthesis of isobutyryl-L-aspartic acid

Dibenzyl isobutyryl-L-aspartic acid ester (200 mg, 0.52mmol) and palladium-on-carbon (40 mg) were dissolved in tetrahydrofuran (3 mL), and added with two drops of concentrated hydrochloric acid, and the reaction solution was stirred at room temperature for 16 h under a hydrogen atmosphere. After it was monitored that the reaction was completed, the palladium-on-carbon was filtered out using diatomaceous earth, and the organic phase was concentrated to obtain 85 mg of isobutyryl-L-aspartic acid (Compound **59**). ¹H NMR (500 MHz, DMSO-*d6*) δ 12.47 (s, 2H), 8.02 (d, *J* = 8.0Hz, 1H), 4.50 (td, *J* = 7.6, 5.7 Hz, 1H), 2.70 - 2.51 (m, 2H), 2.40 (p, *J* = 6.8 Hz, 1H), 0.97 (t, *J* = 6.5 Hz, 6H). MS m/z (ESI): 204 [M+H]⁺.

### Example 15: Synthesis of Compound 60

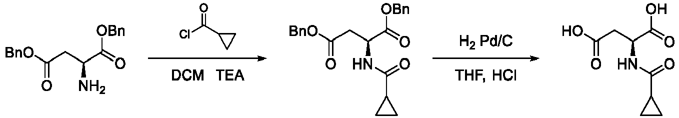

### Step 1: Synthesis of dibenzyl (cyclopropylcarbonyl)-L-aspartic acid ester

L-Aspartic acid dibenzyl ester (1 g, 3.19 mmol) and cyclopropylformyl chloride (398 mg, 3.83mmol) were dissolved in dichloromethane (10 mL), then added dropwise with triethylamine (0.88 mL, 6.39 ml), and the reaction solution was stirred at room temperature for 2 h. The reaction solution was added with an appropriate amount of water, and extracted with dichloromethane. The organic phase was separated. The organic phase was washed with water, dried, concentrated, and purified by column chromatography to obtain 480 mg of dibenzyl (cyclopropylcarbonyl)-L-aspartic acid ester. MS m/z (ESI): 382[M+H]⁺.

### Step 2: Synthesis of (cyclopropylcarbonyl)-L-aspartic acid

Dibenzyl (cyclopropylcarbonyl)-L-aspartic acid ester (200 mg, 0.52mmol) and palladium-on-carbon (40 mg) were dissolved in tetrahydrofuran (3 mL), then added with two drops of concentrated hydrochloric acid, and the reaction solution was stirred at room temperature for 16 h under a hydrogen atmosphere. After it was monitored that the reaction was completed, the palladium-on-carbon was filtered out using diatomaceous earth, and the organic phase was concentrated to obtain 90 mg of (cyclopropylcarbonyl)-L-aspartic acid (Compound **6**0). ¹H NMR (500 MHz, DMSO-*d6*) δ 12.51 (s, 2H), 8.39 (d, *J =* 8.0 Hz, 1H), 4.53 (td, *J* = 7.4, 5.6 Hz, 1H), 2.70 - 2.52 (m, 2H), 1.66 - 1.59 (m, 1H), 0.68 - 0.61 (m, 4H). MS m/z (ESI): 202 [M+H]⁺.

### Example 16: Synthesis of Compound 61

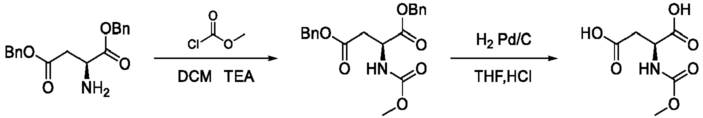

### Step 1: Synthesis of dibenzyl (methoxycarbonyl)-L-aspartic acid ester

L-Aspartic acid dibenzyl ester (1 g, 3.19 mmol) and methyl chloroformate (360 mg, 3.83 mmol) were dissolved in dichloromethane (10 mL), then added dropwise with triethylamine (0.88 mL, 6.39 ml), and the reaction solution was stirred at room temperature for 2 h. The reaction solution was added with an appropriate amount of water, and extracted with dichloromethane. The organic phase was separated. The organic phase was washed with water, dried, concentrated, and purified by column chromatography to obtain 600 mg of dibenzyl (methoxycarbonyl)-L-aspartic acid ester. MS m/z (ESI): 372[M+H]⁺.

### Step 2: Synthesis of (methoxycarbonyl)-L-aspartic acid

Dibenzyl (methoxycarbonyl)-L-aspartic acid ester (500 mg, 1.35 mmol) and palladium-on-carbon (100 mg) were dissolved in tetrahydrofuran (5 mL), then added with two drops of concentrated hydrochloric acid, and the reaction solution was stirred at room temperature for 16 h under a hydrogen atmosphere. After it was monitored that the reaction was completed, the palladium-on-carbon was filtered out using diatomaceous earth, and the organic phase was concentrated to obtain 130 mg of (methoxycarbonyl)-L-aspartic acid (Compound **61**). ¹H NMR (500 MHz, DMSO-*d6*) δ 12.55 (s, 2H), 7.43 (d, *J* = 8.4 Hz, 1H), 4.30 (td, *J* = 8.2, 5.4 Hz, 1H), 3.53 (s, 3H), 2.73 - 2.51 (m, 2H). MS m/z (ESI): 192 [M+H]⁺.

### Example 17: Synthesis of Compound 62

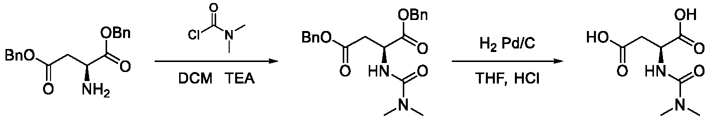

### Step 1: Synthesis of dibenzyl (dimethylcarbamoyl)-L-aspartic acid ester

L-Aspartic acid dibenzyl ester (1 g, 3.19 mmol) and dimethylcarbamoyl chloride (411 mg, 3.83mmol) were dissolved in dichloromethane (10 mL), then added dropwise with triethylamine (0.88 mL, 6.39 ml), and the reaction solution was stirred at room temperature for 2 h. The reaction solution was added with an appropriate amount of water, and extracted with dichloromethane. The organic phase was separated. The organic phase was washed with water, dried, concentrated, and purified by column chromatography to obtain 940 mg of dibenzyl (methylcarbamoyl)-L-aspartic acid ester. MS m/z (ESI): 385[M+H]⁺.

### Step 2: Synthesis of (dimethylcarbamoyl)-L-aspartic acid

Dibenzyl (dimethylcarbamoyl)-L-aspartic acid ester (500 mg, 1.30 mmol) and palladium-on-carbon (100 mg) were dissolved in tetrahydrofuran (5 mL), then added with two drops of concentrated hydrochloric acid, and the reaction solution was stirred at room temperature for 16 h under a hydrogen atmosphere. After it was monitored that the reaction was completed, the palladium-on-carbon was filtered out using diatomaceous earth, and the organic phase was concentrated to obtain 120 mg of (dimethylcarbamoyl)-L-aspartic acid (Compound **62**). ¹H NMR (500 MHz, DMSO-*d6*) δ 12.38 (s, 2H), 6.47 (d, *J* = 8.1 Hz, 1H), 4.39 (td, *J* = 7.6, 5.7 Hz, 1H), 2.77 (s, 6H), 2.73 - 2.53 (m, 2H). MS m/z (ESI): 205 [M+H]⁺.

### Example 18: Synthesis of Compound 63

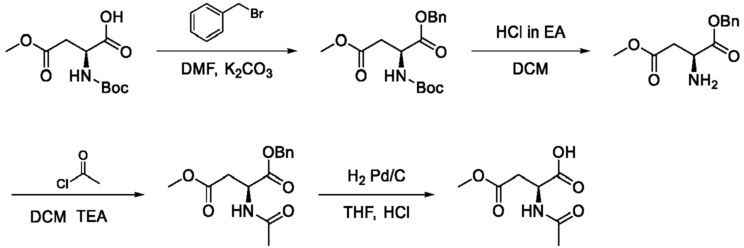

### Step 1: Synthesis of 1-benzyl 4-methyl (tert-butoxycarbonyl)-L-aspartic acid ester

(*S*)-2-((Tert-butoxycarbonyl)amino)-4-methoxy-4-oxobutyric acid (9 g, 36.44 mmol), benzyl bromide (7.47 g, 43.72 mmol) and potassium carbonate (10.06 g, 72.87 mmol) were dissolved in DMF (10 mL), and the reaction solution was stirred at room temperature for 16 hours. After the reaction was completed, the reaction solution was diluted by adding water, and extracted with ethyl acetate, the reaction solution was washed with brine, and the organic phase was dried, and concentrated to obtain 9 g of 1-benzyl 4-methyl (tert-butoxycarbonyl)-L-aspartic acid ester. MS m/z (ESI): 338[M+H]⁺.

### Step 2: Synthesis of 1-benzyl 4-methyl L-aspartic acid ester

1-Benzyl 4-methyl (tert-butoxycarbonyl)-L-aspartic acid ester (9 g, 26.71 mmol) was dissolved in dichloromethane (20 mL) at room temperature, then added with a solution of hydrogen chloride in ethyl acetate (2M) (80 ml, 160.24 mmol), and the solution was reacted at room temperature for 1 hour. After it was monitored that the reaction was completed, the precipitated solid was collected by filtration to obtain 6 g of 1-benzyl 4-methyl L-aspartic acid ester. MS m/z (ESI): 238[M+H]⁺.

### Step 3: Synthesis of 1-benzyl 4-methyl acetyl-L-aspartic acid ester

1-Benzyl 4-methyl L-aspartic acid ester (1 g, 4.22 mmol) and acetyl chloride (397 mg, 5.06 mmol) were dissolved in dichloromethane (10 mL), and then added dropwise with triethylamine (1.17 mL, 8.44 ml), and the reaction solution was stirred at room temperature for 2 h. The reaction solution was added with an appropriate amount of water, and extracted with dichloromethane. The organic phase was separated. The organic phase was washed with water, dried, concentrated, and purified by column chromatography to obtain 900 mg of 1-benzyl 4-methyl acetyl-L-aspartic acid ester. MS m/z (ESI): 280[M+H]⁺.

### Step 4: Synthesis of 4-methyl acetyl-L-aspartic acid ester

1-Benzyl 4-methyl acetyl-L-aspartic acid ester (900 mg, 3.23 mmol) and palladium-on-carbon (180 mg) were dissolved in tetrahydrofuran (5 mL), and added with two drops of concentrated hydrochloric acid, and the reaction solution was stirred at room temperature for 16 h under a hydrogen atmosphere. After it was monitored that the reaction was completed, the palladium-on-carbon was filtered out using diatomaceous earth, and the organic phase was concentrated, and purified by column chromatography to obtain 50 mg of 4-methyl acetyl-L-aspartic acid ester (Compound **63**). ¹H NMR (400 MHz, DMSO-*d6*) δ 12.68 (s, 1H), 8.22 (d, *J =* 8.0 Hz, 1H), 4.54 (ddd, *J* = 8.0, 7.3, 5.9 Hz, 1H), 3.59 (s, 3H), 2.78 - 2.59 (m, 2H), 1.82 (s, 3H). MS m/z (ESI): 190 [M+H]⁺.

### Example 19: Synthesis of Compound 64

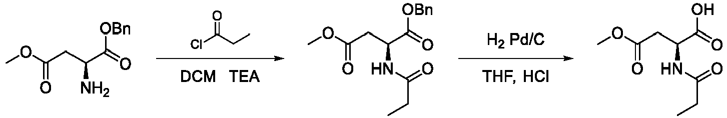

### Step 1: Synthesis of 1-benzyl 4-methyl propionyl-L-aspartic acid ester

1-Benzyl 4-methyl L-aspartic acid ester (1 g, 4.22 mmol) and propionyl chloride (468 mg, 5.06 mmol) were dissolved in dichloromethane (10 mL), and then added dropwise with triethylamine (1.17 mL, 8.44 ml), and the reaction solution was stirred at room temperature for 2 h. The reaction solution was added with an appropriate amount of water, and extracted with dichloromethane. The organic phase was separated. The organic phase was washed with water, dried, concentrated, and purified by column chromatography to obtain 860 mg of 1-benzyl 4-methyl propionyl-L-aspartic acid ester. MS m/z (ESI): 294[M+H]⁺.

### Step 2: Synthesis of 4-methyl propionyl-L-aspartic acid ester

1-Benzyl 4-methyl propionyl-L-aspartic acid ester (860 mg, 2.93 mmol) and palladium-on-carbon (180 mg) were dissolved in tetrahydrofuran (5 mL), and added with two drops of concentrated hydrochloric acid, and the reaction solution was stirred at room temperature for 16 h under a hydrogen atmosphere. After it was monitored that the reaction was completed, the palladium-on-carbon was filtered out using diatomaceous earth, and the organic phase was concentrated, and purified by column chromatography to obtain 65 mg of 4-methyl propionyl-L-aspartic acid ester (Compound **64**). ¹H NMR (400 MHz, DMSO-*d6*) δ 12.70 (s, 1H), 8.12 (d, *J* = 8.0 Hz, 1H), 4.55 (td, *J* = 7.6, 6.0 Hz, 1H), 3.59 (s, 3H), 2.79 - 2.58 (m, 2H), 2.09 (q, *J* = 7.6 Hz, 2H), 0.96 (t, *J* = 7.6 Hz, 3H). MS m/z (ESI): 204 [M+H]⁺.

### Example 20: Synthesis of Compound 65

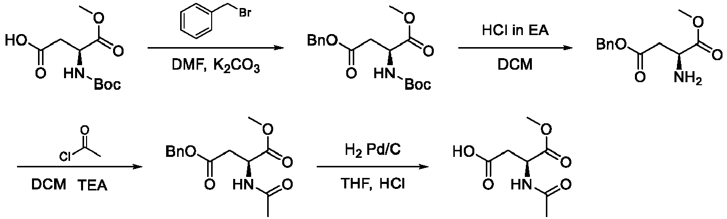

### Step 1: Synthesis of 4-benzyl 1-methyl (tert-butoxycarbonyl)-L-aspartic acid ester

(S)-2-((Tert-butoxycarbonyl)amino)-1-methoxy-1-oxobutyric acid (9 g, 36.44 mmol), benzyl bromide (7.47 g, 43.72 mmol) and potassium carbonate (10.06 g, 72.87 mmol) were dissolved in DMF (10 mL), and the reaction solution was stirred at room temperature for 16 hours. After the reaction was completed, the reaction solution was diluted by adding water, and extracted with ethyl acetate, the reaction solution was washed with brine, and the organic phase was separated, dried and concentrated to obtain 12 g of 4-benzyl 1-methyl (tert-butoxycarbonyl)-L-aspartic acid ester. MS m/z (ESI): 338[M+H]⁺.

### Step 2: Synthesis of 4-benzyl 1-methyl L-aspartic acid ester

4-Benzyl 1-methyl(tert-butoxycarbonyl)-L-aspartic acid ester (12 g, 35.6 mmol) was dissolved in dichloromethane (50 mL) at room temperature, then added with a solution of hydrogen chloride in ethyl acetate (2M) (106 ml, 213.65 mmol), and the solution was reacted at room temperature for 1 hour. After it was monitored that the reaction was completed, the precipitated solid was collected by filtration to obtain 9 g of 4-benzyl 1-methyl L-aspartic acid ester. MS m/z (ESI): 238[M+H]⁺.

### Step 3: Synthesis of 4-benzyl 1-methyl acetyl-L-aspartic acid ester

4-Benzyl 1-methyl L-aspartic acid ester (1 g, 4.22 mmol) and acetyl chloride (397 mg, 5.06 mmol) were dissolved in dichloromethane (10 mL), and then added dropwise with triethylamine (1.17 mL, 8.44 ml), and the reaction solution was stirred at room temperature for 2 h. The reaction solution was added with an appropriate amount of water, and extracted with dichloromethane. The organic phase was separated. The organic phase was washed with water, dried, concentrated, and purified by column chromatography to obtain 600 mg of 4-benzyl 1-methyl acetyl-L-aspartic acid ester. MS m/z (ESI): 280[M+H]⁺.

### Step 4: Synthesis of 1-methyl acetyl-L-aspartic acid ester

4-Benzyl 1-methyl acetyl-L-aspartic acid ester (600 mg, 2.15 mmol) and palladium-on-carbon (120 mg) were dissolved in tetrahydrofuran (5 mL), and added with two drops of concentrated hydrochloric acid, and the reaction solution was stirred at room temperature for 16 h under a hydrogen atmosphere. After it was monitored that the reaction was completed, the palladium-on-carbon was filtered out using diatomaceous earth, the organic phase was concentrated, and purified through column chromatography to obtain 40 mg of 1-methyl acetyl-L-aspartic acid ester (Compound **65**). ¹H NMR (400 MHz, DMSO-*d6*) δ 12.43 (s, 1H), 8.31 (d, *J* = 7.8 Hz, 1H), 4.56 (td, *J* = 7.4, 5.6 Hz, 1H), 3.60 (s, 3H), 2.72 - 2.53 (m, 2H), 1.82 (s, 3H). MS m/z (ESI): 190 [M+H]⁺.

### Example 21: Synthesis of Compound 66

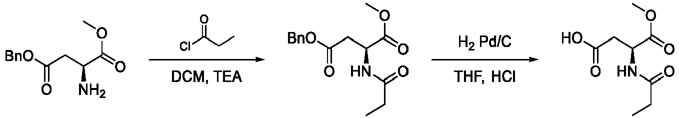

### Step 1: Synthesis of 4-benzyl 1-methyl propionyl-L-aspartic acid ester

4-Benzyl 1-methyl L-aspartic acid ester (1 g, 4.22 mmol) and propionyl chloride (468 mg, 5.06 mmol) were dissolved in dichloromethane (10 mL), and then added dropwise with triethylamine (1.17 mL, 8.44 ml), and the reaction solution was stirred at room temperature for 2 h. The reaction solution was added with an appropriate amount of water, and extracted with dichloromethane. The organic phase was separated. The organic phase was washed with water, dried, concentrated, and purified by column chromatography to obtain 650 mg of 4-benzyl 1-methyl propionyl-L-aspartic acid ester. MS m/z (ESI): 294[M+H]⁺.

### Step 2: Synthesis of 1-methyl propionyl-L-aspartic acid ester

4-Benzyl 1-methyl propionyl-L-aspartic acid ester (650 mg, 2.22 mmol) and palladium-on-carbon (130 mg) were dissolved in tetrahydrofuran (5 mL), and added with two drops of concentrated hydrochloric acid, and the reaction solution was stirred at room temperature for 16 h under a hydrogen atmosphere. After it was monitored that the reaction was completed, the palladium-on-carbon was filtered out using diatomaceous earth, the organic phase was concentrated, and purified by column chromatography to obtain 37 mg of 1-methyl propionyl-L-aspartic acid ester (Compound **66**). ¹H NMR (400 MHz, DMSO-*d6*) δ 12.40 (s, 1H), 8.21 (d, *J* = 7.8 Hz, 1H), 4.57 (td, *J* = 7.5, 5.8 Hz, 1H), 3.60 (s, 3H), 2.72 - 2.54 (m, 2H), 2.10 (q, *J* = 7.6 Hz, 2H), 0.97 (t, *J* = 7.6 Hz, 3H). MS m/z (ESI): 204 [M+H]⁺.

### Example 22: Synthesis of Compound 67

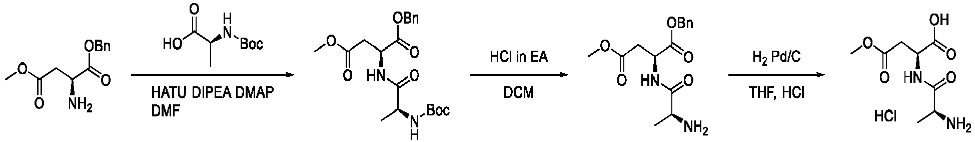

### Step 1: Synthesis of 1-benzyl 4-methyl (tert-butoxycarbonyl)-L-alanyl-L-aspartic acid ester

1-Benzyl 4-methyl L-aspartic acid ester (500 mg, 2.11 mmol), (tert-butoxycarbonyl)-L-alanine (479 mg, 2.5 mmol), HATU (962 mg, 2.53 mmol)) and DMAP (26 mg, 0.21 mmol) were dissolved in DMF (5 mL), added dropwise with DIPEA (506 mg, 3.9 mmol), and then stirred and reacted at room temperature for 2 h. After it was monitored that the reaction was completed, the reaction solution was diluted by adding water, and extracted with ethyl acetate (5 mL*2), the organic phase was washed with water, dried, concentrated, and purified by column chromatography to obtain 400 mg of 1-benzyl 4-methyl (tert-butoxycarbonyl)-L-alanyl-L-aspartic acid ester. MS m/z (ESI): 409[M+H]⁺.

### Step 2: Synthesis of 1-benzyl 4-methyl L-alanyl-L-aspartic acid ester

1-Benzyl 4-methyl (tert-butoxycarbonyl)-L-alanyl-L-aspartic acid ester (400 mg, 0.98 mmol) was dissolved in dichloromethane (5 mL), a solution of hydrogen chloride in ethyl acetate (2M) (2.9 ml, 5.88 mmol) was added to the reaction system, and the solution was reacted at room temperature for 1 hour. After it was monitored that the reaction was completed, the reaction solution was concentrated to obtain 100 mg of 1-benzyl 4-methyl L-alanyl-L-aspartic acid ester. MS m/z (ESI): 309[M+H]⁺

### Step 3: Synthesis of (S)-2-((S)-2-aminopropionamido)-4-methoxy-4-oxobutyric acid hydrochloride

1-Benzyl 4-methyl L-alanyl-L-aspartic acid ester (100 mg, 0.32 mmol) and palladium-on-carbon (50 mg) were dissolved in tetrahydrofuran (3 mL), and added with one drop of concentrated hydrochloric acid, and the reaction solution was stirred at room temperature for 16 h under a hydrogen atmosphere. After it was monitored that the reaction was completed, the palladium-on-carbon was filtered out using diatomaceous earth, and the organic phase was concentrated, and purified by column chromatography to obtain 20 mg of (*S*)-2-((*S*)-2-aminopropionamido)-4-methoxy-4-oxobutyric acid hydrochloride (Compound **67**). ¹H NMR (400 MHz, DMSO-*d6*) δ 12.60 (s, 1H), 8.94 (d, *J* = 8.0 Hz, 1H), 8.35 (s, 2H), 4.62 - 4.55 (m, 1H), 3.84 (dd, MS m/z (ESI) :255 [M+H]⁺.

### Example 23: Synthesis of Compound 68

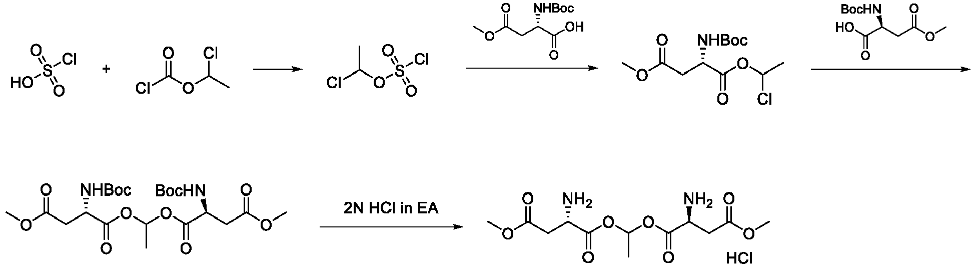

### Step 1: Synthesis of 1-chloroethylsulfonyl chloride

To a solution of 1-chloroethyl chloroformate (3.02 mL, 28.0 mmol) in anhydrous dichloromethane (40 mL), chlorosulfonic acid (3.72 mL, 56.0 mmol) was slowly added at 0°C within 10 minutes. The mixture was stirred at 0°C for 4 h under nitrogen atmosphere. Ice water was added to the reaction system to quench the reaction, and dichloromethane (200 mL) was used for extraction. The organic layer was separated, washed with brine solution, dried over anhydrous sodium sulfate, and concentrated to obtain 4.0 g of 1-chloroethylsulfonyl chloride as a light yellow liquid.

### Step 2: Synthesis of 1-(1-chloroethyl) 4-methyl (tert-butoxycarbonyl)-L-aspartic acid ester

(*S*)-2-((Tert-butoxycarbonyl)amino)-4-methoxy-4-oxobutyric acid and tetrabutylammonium bisulfate were dissolved in dichloromethane, stirred and cooled in an ice bath to 0°C. An aqueous solution of potassium carbonate and a dichloromethane solution of 1-chloroethylsulfonyl chloride were added dropwise to the reaction system in sequence, stirred and slowly heated to room temperature, and stirred overnight. After it was monitored by TLC that the reaction was completed, dichloromethane was added, and washed three times with water, and the organic phase was separated. The organic phase was dried over anhydrous sodium sulfate, filtered, concentrated, and purified by column chromatography (EA/PE gradient elution, 20:1 to 5:1) to obtain 1.0 g of 1-(1-chloroethyl) 4-methyl (tert-butoxycarbonyl)-L-aspartic acid ester as a colorless oil.

### Step 3: Synthesis of O'1,O1-(ethan-1,1-diyl)4-dimethyl (2S,2'S)-bis(2-((tert-butoxycarbonyl)amino)succinate)

Sodium iodide and potassium carbonate were dissolved in DMF, placed in an ice bath, stirred and cooled to 0°C. Boc-L-aspartic acid 4-methyl ester was dissolved in DMF and added dropwise into the above reaction system, placed in an ice bath, and stirred for 30 minutes. 1-(1-Chloroethyl) 4-methyl (tert-butoxycarbonyl)-L-aspartic acid ester was dissolved in DMF and added dropwise into the reaction system, stirred and slowly warmed to room temperature, and stirred about overnight, and the reaction was monitored TLC. After the reaction of the raw materials was completed, 10 mL of water was added, and ethyl acetate was used to perform extraction three times, and washing was performed with water three times. The organic phases were separated and combined, then dried over anhydrous sodium sulfate, filtered, concentrated, and purified by column chromatography (EA/PE gradient elution, 10:1 to 3:1) to obtain 200 mg of O'1,O1-(ethane-1,1-diyl) 4-dimethyl (2S,2'S)-bis(2-((tert-butoxycarbonyl)amino)succinate) as a white solid.

### Step 4: Synthesis of O'1,O1-(ethan-1,1-diyl) 4-dimethyl (2S,2'S)-bis(2-aminosuccinate) hydrochloride

O'1,O1-(Ethan-1,1-diyl) 4-dimethyl (2S,2'S)-bis(2-((tert-butoxycarbonyl)amino)succinate) was dissolved in dichloromethane, placed in an ice bath, stirred and cooled to 0°C, then added dropwise with 2N solution of hydrochloric acid in ethyl acetate, stirred and slowly warmed to room temperature, and stirred for about 2 hours. After it was monitored by HPLC that the reaction was completed, the reaction solution was filtered by suction, the filter cake was washed with a small amount of ethyl acetate, and the filter cake was dried to obtain 105 mg of O'1,O1-(ethan-1,1-diyl) 4-dimethyl (2S,2'S)-bis(2-aminosuccinate) hydrochloride (Compound **68**), as a white solid. MS m/z (ESI): 357[M+H]⁺.

### Example 24: Synthesis of Compound 69

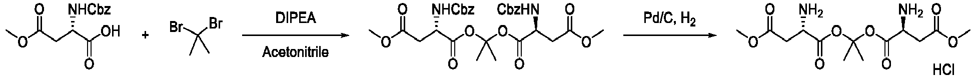

### Step 1: Synthesis of 4-dimethyl O'1,O1-(propan-2,2-diyl) (2S,2'S)-bis(2-(((benzyloxy)carbonyl)amino)succinate)

(*S*)-2-(((Benzyloxy)carbonyl)amino)-4-methoxy-4-oxobutyric acid and 2,2-dibromopropane were dissolved in acetonitrile, added dropwise with DIPEA, stirred and reacted at room temperature for 2 h. After it was monitored that the reaction was completed, the reaction solution was diluted by adding water, the reaction solution was extracted with ethyl acetate, the organic phase was separated, and washed with water, then dried, concentrated, and purified by column chromatography to obtain 4-dimethyl O'1,O1-(propan-2,2-diyl) (2S,2'S)-bis(2-((((benzyloxy)carbonyl)amino)succinate).

### Step 2: Synthesis of 4-dimethyl O'1,O1-(propan-2,2-diyl) (2S,2'S)-bis(2-aminosuccinate) hydrochloride

4-Dimethyl O'1,O1-(propan-2,2-diyl)(2S,2'S)-bis(2-(((benzyloxy)carbonyl)amino)succinate) and palladium on carbon were dissolved in tetrahydrofuran, added with one drop of concentrated hydrochloric acid, and the reaction solution was stirred at room temperature for 16 h under a hydrogen atmosphere. After it was monitored that the reaction was completed, the palladium-on-carbon was filtered out using diatomaceous earth, the organic phase was concentrated, and purified by column chromatography to obtain 4-dimethyl O'1,O1-(propan-2,2-diyl) (2S,2'S)-bis(2-aminosuccinate) hydrochloride (Compound **69**). MS m/z (ESI): 371 [M+H]⁺.

### Example 25: Synthesis of Compound 70

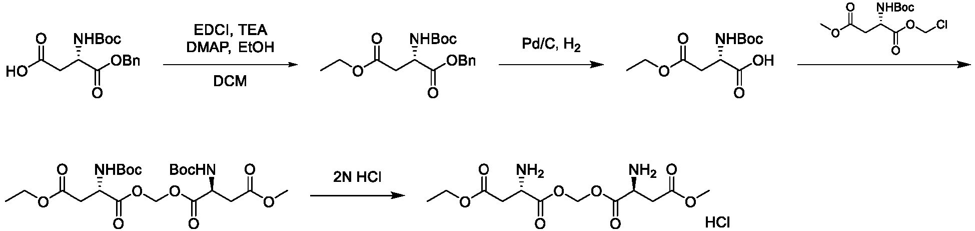

### Step 1: Synthesis of 1-benzyl 4-ethyl (tert-butoxycarbonyl)-L-aspartic acid ester

(S)-4-(Benzyloxy)-3-((tert-butoxycarbonyl)amino)-4-oxobutyric acid (5 g), EDCI, triethylamine, DMAP and ethanol were added into dichloromethane, and stirred at room temperature, and the reaction was monitored by TLC. After it was monitored that the reaction was completed, saturated sodium bicarbonate solution was added to the reaction solution, and the organic layer was separated. The organic layer was dried over anhydrous sodium sulfate, filtered, concentrated, and purified by column chromatography to obtain 4.8 g of 1-benzyl 4-ethyl (tert-butoxycarbonyl)-L-aspartic acid ester.

### Step 2: Synthesis of (S)-2-((tert-butoxycarbonyl)amino)-4-ethoxy-4-oxobutyric acid

1-Benzyl 4-ethyl (tert-butoxycarbonyl)-L-aspartic acid ester (4.8 g) and palladium-on-carbon were dissolved in tetrahydrofuran, and added with one drop of concentrated hydrochloric acid, and the reaction solution was stirred at room temperature for 16 h under a hydrogen atmosphere. After it was monitored that the reaction was completed, the palladium-on-carbon was filtered out using diatomaceous earth, the organic phase was concentrated, and purified by column chromatography to obtain 3.2 g of (*S*)-2-((tert-butoxycarbonyl)amino)-4-ethoxy-4-oxobutyric acid.

### Step 3: Synthesis of 1-((((S)-2-((tert-butylcarbonyl)amino)-4-ethoxy-4-oxobutyryl)oxy) methyl) 4-methyl (tert-butoxycarbonyl)-L-aspartic acid ester

Sodium iodide and potassium carbonate were dissolved in DMF, placed in an ice bath, stirred and cooled to 0°C. (*S*)-2-((Tert-butoxycarbonyl)amino)-4-ethoxy-4-oxobutyric acid (1.2 g) was dissolved in DMF and added dropwise into the above reaction system, placed in an ice bath and stirred for 30 minutes. 1-(Chloromethyl) 4-methyl (tert-butoxycarbonyl)-L-aspartic acid ester was dissolved in DMF and added dropwise into the reaction system, stirred and slowly warmed to room temperature, and stirred about overnight. The reaction was monitored by TLC. After the reaction of the raw materials was completed, water was added, extracted with ethyl acetate three times, and washing was performed with water three times. The organic phases were separated and combined, dried over anhydrous sodium sulfate, filtered, concentrated, and purified by column chromatography to obtain 720 mg of 1-((((*S*)-2-((tert-butylcarbonyl)amino)-4-ethoxy-4-oxobutyryl)oxy)methyl) 4-methyl (tert-butoxycarbonyl)-L-aspartic acid ester.

### Step 4: Synthesis of 1-((((S)-2-amino-4-ethoxy-4-oxobutyryl)oxy)methyl) 4-methyl L-aspartic acid ester hydrochloride

1-((((*S*)-2-((Tert-butylcarbonyl)amino)-4-ethoxy-4-oxobutyryl)oxy)methyl) 4-methyl (tert-butoxycarbonyl)-L-aspartic acid ester (720 mg) was dissolved in dichloromethane, stirred in an ice bath and cooled to 0°C, then added dropwise with 2N solution of hydrochloric acid in ethyl acetate, stirred and slowly warmed to room temperature, and stirred for about 2 hours. After it was monitored by HPLC that the reaction was completed, the reaction solution was filtered by suction, and the filter cake was washed with a small amount of ethyl acetate, and the filter cake was dried to obtain 225 mg of 1-((((S)-2-amino-4-ethoxy-4-oxobutyryl)oxy)methyl) 4-methyl L-aspartic acid ester hydrochloride (Compound **70**). MS m/z (ESI): 357[M+H]⁺.

### Example 26: Synthesis of Compound 71

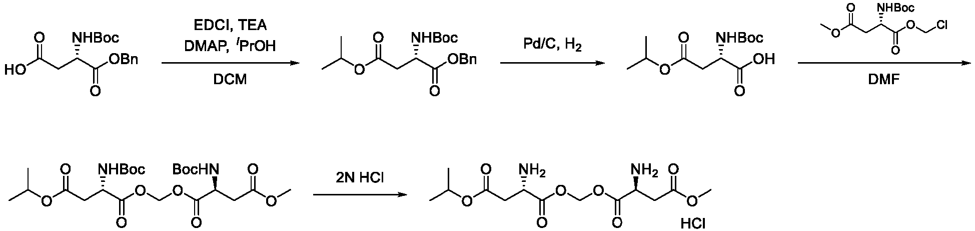

### Step 1: Synthesis of 1-benzyl 4-isopropyl (tert-butoxycarbonyl)-L-aspartic acid ester

(*S*)-4-(Benzyloxy)-3-((tert-butoxycarbonyl)amino)-4-oxobutyric acid (10 g), EDCI, triethylamine, DMAP and isopropanol alcohol were added to dichloromethane, stirred at room temperature, and the reaction was monitored by TLC. After it was monitored that the reaction was completed, a saturated sodium bicarbonate solution was added to the reaction solution, the organic layer was separated, and the organic layer was dried over anhydrous sodium sulfate, filtered, concentrated, and purified to obtain 1-benzyl 4-isopropyl (tert-butoxycarbonyl)-L-aspartic acid ester (8.8 g).

### Step 2: Synthesis of (S)-2-((tert-butoxycarbonyl)amino)-4-isopropoxy-4-oxobutyric acid

1-Benzyl 4-isopropyl (tert-butoxycarbonyl)-L-aspartic acid ester (8.8 g) and palladium-on-carbon were dissolved in tetrahydrofuran, added with one drop of concentrated hydrochloric acid, and the reaction solution was stirred at room temperature for 16 h under a hydrogen atmosphere. After it was monitored that the reaction was completed, the palladium-on-carbon was filtered out using diatomaceous earth, the organic phase was concentrated, and purified by column chromatography to obtain 6.0 g of (*S*)-2-((tert-butoxycarbonyl)amino)-4-isopropoxy-4-oxybutyric acid.

### Step 3: Synthesis of 1-((((S)-2-((tert-butoxycarbonyl)amino)-4-isopropoxy-4-oxobutyryl) oxy)methyl) 4-methyl (tert-butoxycarbonyl)-L-aspartic acid ester

Sodium iodide and potassium carbonate were dissolved in DMF, placed in an ice bath, stirred and cooled to 0°C. (*S*)-2-((Tert-butoxycarbonyl)amino)-4-isopropoxy-4-oxobutyric acid (1.6 g) was dissolved in DMF and added dropwise into the above reaction system, placed in an ice bath, and stirred for 30 minutes. 1-(Chloromethyl) 4-methyl (tert-butoxycarbonyl)-L-aspartic acid ester was dissolved in DMF and added dropwise into the reaction system, stirred and slowly warmed to room temperature, and stirred about overnight. The reaction was monitored by TLC. After the reaction of the raw materials was completed, water is added, extracted three times with ethyl acetate, and washing was performed three times with water, the organic phases were separated and combined, dried over anhydrous sodium sulfate, filtered, concentrated, and purified by chromatography to obtain 870 mg of 1-((((*S*)-2-((tert-butoxycarbonyl)amino)-4-isopropoxy-4-oxobutyryl)oxy)methyl) 4-methyl (tert-butoxycarbonyl)-L-aspartic acid ester.

### Step 4: Synthesis of 1-((((S)-2-amino-4-isopropoxy-4-oxobutyryl)oxy)methyl) 4-methyl L-aspartic acid ester hydrochloride

1-((((*S*)-2-((Tert-butoxycarbonyl)amino)-4-isopropoxy-4-oxobutyryl)oxy)methyl) 4-methyl (tert-butoxycarbonyl)-L-aspartic acid ester (570 mg) was dissolved in dichloromethane, placed in an ice bath, stirred and cooled to 0°C, added dropwise with 2N solution of hydrochloric acid in ethyl acetate, stirred and slowly heated to room temperature, stirred for about 2 h. After it was monitored by HPLC that the reaction was completed, the reaction solution was filtered by suction, and the filter cake was washed with a small amount of ethyl acetate, and the filter cake was dried to obtain 140 mg of 1-((((S)-2-amino-4-isopropoxy-4-oxobutyryl)oxy)methyl) 4-methyl L-aspartic acid ester hydrochloride (Compound **71**). MS m/z (ESI): 371[M+H]⁺.

### Example 27: Synthesis of Compound 72

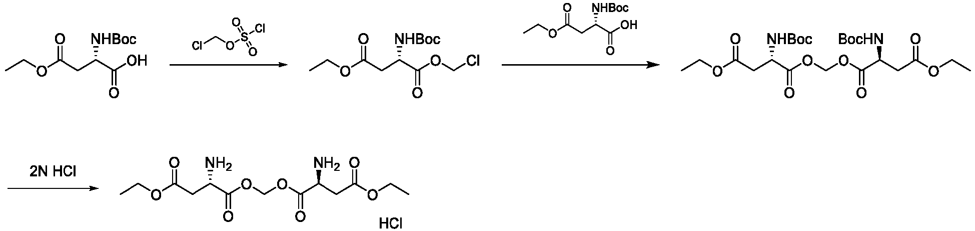

### Step 1: Synthesis of 1-(chloromethyl) 4-ethyl (tert-butoxycarbonyl)-L-aspartic acid ester

(*S*)-2-((Tert-butoxycarbonyl)amino)-4-ethoxy-4-oxobutyric acid (3.2 g) and tetrabutylammonium bisulfate were dissolved in dichloromethane, placed in an ice bath, stirred and cooled to 0°C. An aqueous solution of potassium carbonate and a dichloromethane solution of 1-chloromethylsulfonyl chloride were added dropwise to the reaction system in sequence, stirred and slowly warmed to room temperature, and stirred overnight. After it was monitored by TLC that the reaction was completed, dichloromethane was added, washed three times with water, and the organic phase was separated. The organic phase was dried over anhydrous sodium sulfate, filtered, concentrated, and purified by column chromatography to obtain 1.6 g of 1-(chloromethyl) 4-ethyl (tert-butyloxycarbonyl)-L-aspartic acid ester.

### Step 2: Synthesis of 4-diethyl O'1,O1-methylene (2S,2'S)-bis(2-((tert-butoxycarbonyl)amino)succinate)

Sodium iodide and potassium carbonate were dissolved in DMF, placed in an ice bath, stirred and cooled to 0°C. (*S*)-2-((Tert-butoxycarbonyl)amino)-4-ethoxy-4-oxobutyric acid was dissolved in DMF and added dropwise into the above reaction system, placed in an ice bath and stirred for 30 minutes. 1-(Chloromethyl) 4-ethyl (tert-butoxycarbonyl)-L-aspartic acid ester (1.6 g) was dissolved in DMF and added dropwise into the reaction system, stirred and slowly warmed to room temperature, and stirred about overnight. The reaction was monitored by TLC. After the reaction of the raw materials was completed, water was added, extracted with ethyl acetate three times, and washing was performed with water three times. The organic phases were separated and combined, dried over anhydrous sodium sulfate, filtered, concentrated, and purified by column chromatography to obtain 850 mg of 4-diethyl O'1,O1-methylene (2S,2'S)-bis(2-((tert-butoxycarbonyl)amino)succinate).

### Step 3: Synthesis of (S)-2-((tert-butoxycarbonyl)amino)-4-methoxy-4-oxobutyric hydrochloride

4-Diethyl O'1,O1-methylene (2S,2'S)-bis(2-((tert-butoxycarbonyl)amino)succinate) (850 mg) was dissolved in dichloromethane, placed in an ice bath, stirred and cooled to 0°C, then added dropwise with 2N solution of hydrochloric acid in ethyl acetate, stirred and slowly warmed to room temperature, and stirred for about 2 hours. After it was monitored by HPLC that the reaction was completed, the reaction solution was filtered by suction, the filter cake was washed with a small amount of ethyl acetate, and the filter cake was dried to obtain 174 mg of (*S*)-2-((tert-butoxycarbonyl)amino)-4-methoxy-4-oxobutyric acid hydrochloride (Compound **72**). MS m/z (ESI): 371[M+H]⁺.

### Example 28: Synthesis of Compound 73

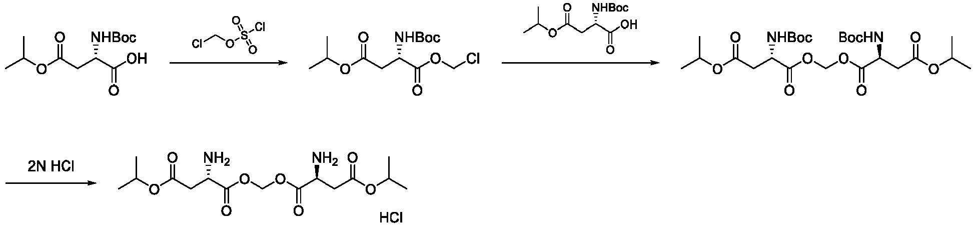

### Step 1: Synthesis of 1-(chloromethyl) 4-isopropyl (tert-butoxycarbonyl)-L-aspartic acid ester

(*S*)-2-((Tert-butoxycarbonyl)amino)-4-isopropoxy-4-oxobutyric acid (4.0 g) and tetrabutylammonium bisulfate were dissolved in dichloromethane, placed in an ice bath, stirred and cooled to 0°C. An aqueous solution of potassium carbonate and a dichloromethane solution of 1-chloromethylsulfonyl chloride were added dropwise to the reaction system in sequence, stirred and slowly warmed to room temperature, and stirred overnight. After it was monitored by TLC that the reaction was completed, dichloromethane was added, washed with water three times, the organic phase was separated, and the organic phase was dried over anhydrous sodium sulfate, filtered, concentrated, and purified by column chromatography to obtain 4.0 g of 1-(chloromethyl) 4-isopropyl (tert-butoxycarbonyl)-L-aspartic acid ester.

### Step 2: Synthesis of 4-diisopropyl O'1,O1-methylene (2S,2'S)-bis(2-((tert-butoxycarbonyl)amino)succinate)

Sodium iodide and potassium carbonate were dissolved in DMF, placed in an ice bath, stirred and cooled to 0°C. (S)-2-((Tert-butoxycarbonyl)amino)-4-isopropoxy-4-oxobutyric acid was dissolved in DMF and added dropwise into the above reaction system, placed in an ice bath and stirred for 30 minutes. 1-(Chloromethyl) 4-isopropyl (tert-butoxycarbonyl)-L-aspartic acid ester (2.5 g) was added in DMF and added dropwise into the reaction system, stirred and slowly warmed to room temperature, and stirred about overnight. The reaction was monitored by TLC. After the reaction of the raw materials was completed, water was added, extracted with ethyl acetate three times, and washing was performed with water three times. The organic phases were separated and combined, dried over anhydrous sodium sulfate, filtered, concentrated, and purified by chromatography to obtain 1.3 g of 4-diisopropyl O'1,O1-methylene (2S,2'S)-bis(2-((tert-butoxycarbonyl)amino)succinate).

### Step 3: Synthesis of 4-diisopropyl O'1,O1-methylene (2S,2'S)-bis(2-aminosuccinate) hydrochloride

4-Diisopropyl O'1,O1-methylene (2S,2'S)-bis(2-((tert-butoxycarbonyl)amino)succinate) (0.8 g) was dissolved in dichloromethane, placed an ice bath, stirred and cooled to 0°C, added dropwise with 2N solution of hydrochloric acid in ethyl acetate, stirred and slowly warmed to room temperature, and stirred for about 2 hours. After it was monitored by HPLC that the reaction was completed, the reaction solution was filtrated by suction, the filter cake was washed with a small amount of ethyl acetate, and the filter cake was dried to obtain 270 mg of 4-diisopropyl O'1,O1-methylene (2S,2'S)-bis(2-aminosuccinate) hydrochloride (Compound **73**). MS m/z (ESI): 399[M+H]⁺.

### Example 29: Synthesis of Compound 74

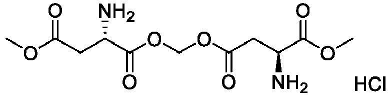

For the synthesis method of the titled compound, the synthesis of Compound **54** in Example 9 of the present application was referred to. Boc-L-aspartic acid 4-methyl ester was replaced by *N*-tert-butoxycarbonyl-L-aspartic acid 1-methyl ester in step 2 to obtain 4-((((*S*)-2-amino-4-methoxy-4-oxobutyryl)oxy)methyl) 1-methyl L-aspartic acid ester hydrochloride (Compound **74**) as a white solid. MS m/z (ESI): 343[M+H]⁺.

### Example 30: Synthesis of Compound 77a

### Step 1: Synthesis of O'1,O1-(ethan-1,2-diyl) 4-dimethyl (2S,2'S)-bis(2-(tert-butoxycarbonyl)amino)succinic acid)

Ethylene glycol (0.3 g, 4.84 mmol), *S*-2-(tert-butoxycarbonyl)amino-4-methoxy- 4-oxobutyric acid (2.5 g, 10.16 mmol), EDCI (2.32 g, 12.1 mmol) and DIPEA (3.12 g, 24.2 mmol) were dissolved in DMF (20 mL), the reaction solution was stirred at room temperature for 2 hours under a nitrogen atmosphere. After the reaction was completed, an appropriate amount of ice water and ethyl acetate were added, the organic phase was separated. The organic phase was washed with water, dried, and purified by column chromatography (PE:EA=3:1) to obtain 1.5 g of O'1,O1-(ethan-1,2-diyl) 4-dimethyl (2S,2'S)-bis(2-(tert-butoxycarbonyl)amino)succinic acid). MS (ESI): mass calcd. For C₂₂H₃₆N₂O₁₂ 520.2 m/z found 521.2 [M+H]⁺.

### Step 2: Synthesis of O'1,O1-(ethan-1,2-diyl) 4-dimethyl (2S,2'S)-bis(2-aminosuccinate) hydrochloride

At room temperature, O'1,O1-(ethan-1,2-diyl) 4-dimethyl (2S,2'S)-bis(2-(tert-butoxycarbonyl)amino)succinic acid) (1.5 g, 2.88 mmol) was dissolved in dichloromethane (20 mL), and then added with a solution of hydrochloric acid in ethyl acetate (17.29 mL, 2M, 34.58 mmol) under ice bath, and the solution was reacted at room temperature for 1 hour. After it was monitored that the reaction was completed, the supernatant was discarded, and the solid was dried to obtain 800 mg of O'1,O1-(ethan-1,2-diyl) 4-dimethyl (2S,2'S)-bis(2-aminosuccinate) hydrochloride. MS (ESI): mass calcd. For C₁₂H₂₀N₂O₈+2HCl, 320.1 m/z found 321.1 [M+H]⁺. ¹H NMR (DMSO-J6) δ: 8.88 (br s, 6H), 4.32-4.42 (m, 6H), 3.66 (s, 6H), 3.00-3.12 (m, 4H).

### Example 31: Synthesis of Compound 78a

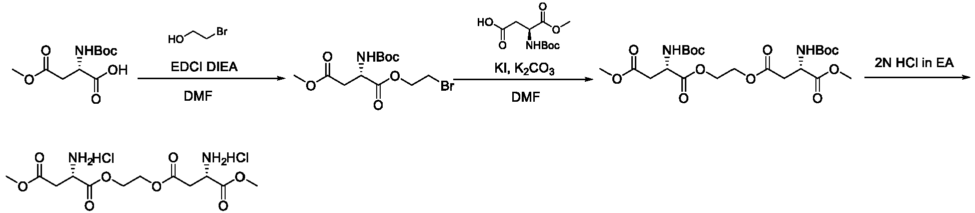

### Step 1: Synthesis of 1-(2-bromoethyl) 4-methyl (tert-butoxycarbonyl)-L-aspartic acid ester

*S*-2-(Tert-butoxycarbonyl)amino-4-methoxy-4-oxobutyric acid (1.9 g, 7.68 mmol), 2-bromoethanol (1.06 g, 8.45 mmol), EDCI (2.21 g, 11.53 mmol) and DIPEA (3.82 ml, 23.05 mmol) were dissolved in DMF (20 mL), the reaction solution was stirred at room temperature for 2 hours under a nitrogen atmosphere. After the reaction was completed, an appropriate amount of ice water and ethyl acetate were added, and the organic phase was separated. The organic phase was washed with water, dried, and purified by column chromatography (PE:EA=4:1) to obtain 1.1 g of 1-(2-bromoethyl) 4-methyl (tert-butoxycarbonyl)-L-aspartic acid ester, MS (ESI): mass calcd. For C₁₂H₂₀BrNO₆ 353.0 m/z found 354.0 [M+H]⁺.

### Step 2: Synthesis of 4-(2-((S)-2-(tert-butoxycarbonyl)amino)-4-methoxy-4-oxobutyryl)oxy) ethyl) 1-methyl (tert-butoxycarbonyl)-L-aspartic acid ester

1-(2-Bromoethyl) 4-methyl (tert-butoxycarbonyl)-L-aspartic acid ester (1 g, 2.82 mmol), (S)-3-(tert-butoxycarbonyl)amino-4-methoxy-4-oxobutyric acid (837 mg, 3.39 mmol), potassium iodide (938 mg, 5.65 mmol), and potassium carbonate (780 mg, 5.65 mmol) were dissolved in DMF (20 mL), and the reaction solution was stirred at room temperature for 2 hours under a nitrogen atmosphere. After the reaction was completed, an appropriate amount of ice water and ethyl acetate were added, and the organic phase was separated. The organic phase was washed with water, dried, and purified by column chromatography (PE:EA=3:1) to obtain 460 mg of 4-(2-((S)-2-(tert-butoxycarbonyl)amino)-4-methoxy-4-oxobutyryl)oxy)ethyl) 1-methyl (tert-butoxycarbonyl)-L-aspartic acid ester, MS (ESI): mass calcd. For C₂₂H₃₆N₂O₁₂ 520.2 m/z found 521.2 [M+ H]⁺.

### Step 3: Synthesis of 4-(2-(S)-2-amino-4-methoxy-4-oxobutyryl)ethyl)1-methyl L-aspartic acid ester hydrochloride

At room temperature, 4-(2-((*S*)-2-(tert-butoxycarbonyl)amino)-4-methoxy-4-oxobutyryl) oxy)ethyl) 1-methyl (tert-butoxycarbonyl)-L-aspartic acid ester (460 mg, 0.88 mmol) was dissolved in dichloromethane (10 mL), a solution of hydrochloric acid in ethyl acetate (5.3 mL, 2M, 10.6 mmol) was added to the reaction system under an ice bath, the solution was reacted at room temperature for 1 hour. After it was monitored that the reaction was completed, the supernatant was poured off, and the solid was dried to obtain 180 mg of 4-(2-(*S*)-2-amino-4-methoxy-4-oxobutyryl)ethyl) 1-methyl L-aspartic acid ester hydrochloride, MS (ESI): mass calcd. For C₁₂H₂₀N₂O₈+2HCl, 320.1 m/z found 321.1 [M+H]⁺. ¹H NMR (DMSO-d6) δ: 8.87 (br s, 6H), 4.33-4.42 (m, 4H), 4.26-4.32 (m, 2H), 3.74 (s, 3H), 3.66 (s, 3H), 2.98 -3.14 (m, 4H).

### Experimental examples for activity

### Test Methods

### (a) In vitro pharmacodynamic evaluation of compounds

### 1. Recovery of LX2 cells:

Preparation: The ultra-clean workbench was irradiated with ultraviolet lamps for 30 minutes in advance; the water bath was preheated at 37°C; the complete culture medium was preheated at 37°C;
After the ultraviolet disinfection of the ultra-clean workbench was completed, the ventilation switch was turned on, the surface was wiped with 75% alcohol cotton balls. A 10cm cell culture dish was prepared, added with 7 mL of complete culture medium containing 10% fetal bovine serum, covered with a lid for later use;
After the working temperature of 37°C of the water bath was achieved, a tube of frozen LX2 cells was taken from the liquid nitrogen tank and quickly transferred to the water bath. The cryopreservation tube was shaken at high frequency and low amplitude to ensure rapid melting of the cryopreservation solution and reduce damage to the cells. When it was observed that the cryopreservation solution was almost completely melted, the cryopreservation tube was quickly taken out, the surface moisture was wiped away, the cryopreservation tube was disinfected with 75% alcohol, the remaining alcohol on the sealing film of the cryopreservation tube was wiped away, and then the sealing film was teared off. The freezing solution was quickly and gently pipetted and transferred into the prepared cell culture dish. The cell culture dish was gently shaken by the "cross" method to ensure that the cells were evenly spread on the cell culture dish. The culture dish was placed in a 37°C, 5% CO₂ incubator. The culture dish was placed as close to the interior as possible to reduce the fluctuation of CO₂ and temperature fluctuations.

### 2. Replacement of cell medium:

After the cells were recovered, the complete medium was replaced according to the cell growth status, degree of adhesion, and color of the medium. LX2 cells were larger in size. After adhesion, the cells were pleomorphic and mainly spindle-shaped. The cells had good refractive index under the microscope and the cells proliferated significantly on the 2^{nd} to 3^{rd} days. Replacement with fresh cell culture medium was carried out according to the density and status of LX2 cells in the culture dish. Note: Before replacement of medium, fresh complete culture medium should be placed in a 37°C water bath and preheated for 10 to 15 minutes. A negative pressure suction device was used to suck out the old culture medium, the cells were washed twice with 4 mL of 1 × PBS buffer, and then the preheated complete culture medium was used for replacement. The cells were placed in a 37°C incubator and continuously cultured.

### 3. Cell passage: When the cell confluence reached about 90%, cell passage culture could be carried out.

Digestion: The old culture medium was sucked out with a negative pressure suction device, then the cells were washed twice with 4 mL of 1×PBS buffer. Then, 1 mL of trypsin was added, the cell culture dish was gently shaken so that the cells were completely infiltrated with the digestion solution;
Termination of digestion: The cell morphology was observed under an inverted microscope. When the cells slowly turned from spindle-shape to round-shape, and the intercellular gaps gradually became obvious, 1 mL of DMEM complete medium containing 10% fetal calf serum was quickly added to stop the digestion. The cells were gently pipetted, so that all the cells fell off the cell culture plate. After being pipetted into a single cell suspension, it was transferred to a 5 mL glass flow cytometry tube, which was tightly closed with lid, and centrifuged at 800 rpm for 5 minutes;
Resuspension: The culture medium in the glass flow cytometry tube was discarded, then the flow cytometry tube was flicked to move the cells, then 2 mL of DMEM complete medium containing 10% fetal calf serum was added to resuspend the cells, and the cells were gently pipetted with a pipette tip to form a suspension of single cells. Passage was performed according to 1:2, or division of plates was performed at a cell density of 5×10⁵ cells/mL, and then the cells were placed in an incubator to continue the culturing. Note: Each cell culture plate required a total of 12 mL of culture medium, so there were 125 µL of complete culture medium per well for the 96-well plate, 250 µL of complete culture medium per well for the 48-well plate, 500 µL of complete culture medium per well for the 24-well plate, 1 mL of complete culture medium per well for the 12-well plate, and 2 mL of complete culture medium per well for the 6-well plate. The cell culture plates were gently shaken by the conventional "cross" method to ensure that the cells were evenly spread on the cell culture plate.

### 4. Western blotting to detect protein expression level

4.1 Extraction of total cell protein: The cells were taken out, the original culture medium was discarded, 1 to 2 mL of 1×PBS was added, washing was performed twice, digestion was performed with trypsin, the supernatant was discarded, the cells at the bottom of the tube were gently moved by flicking, and then 1 mL of PBS was added again to wash the cells twice; after being centrifuged at 1200 rpm for 5 minutes, the supernatant was completely discarded, the cells at the bottom of the tube was mixed well by flicking. Depending on the amount of cells, 50 to 100 µL of protein lysis buffer containing protease inhibitor and phosphatase inhibitor was added, the cells were mixed thoroughly by pipetting, placed on ice and lysed for 30 minutes, then centrifuged at 12,000 rcf for 15 minutes at 4°C, and the supernatant was pipetted and transferred into a new EP tube to obtain the total cell protein, which could be directly used for subsequent determination of protein concentration or stored at -80°C.

4.2 Determination of protein concentration by BCA method: BCA reagent solution A and solution B were mixed well at a ratio of 50:1 to prepare a working solution; 5 µL of the sample to be tested was taken, added into 95 µL of deionized water, mixed, and diluted 20 times, and 20 µL of the diluted sample to be tested was taken and added to a 96-well plate; after 200 µL of the working solution was added, the 96-well plate was placed in a 37°C incubator and incubated for 30 min, and then tested on the machine.

4.3 Protein denaturation: 100 µg of each of samples was taken respectively, supplemented with protein lysis buffer to reach the volume of each sample, then added with 4× loading buffer at 1/3 of the total volume and mixed thoroughly, underwent denaturation at 99°C for 10 min, centrifuged and stored at -80°C.

4.4 Protein electrophoresis: The precast gel was installed into the electrophoresis tank, added with 1×MOPS electrophoresis solution, the comb on the top of the precast gel was removed, the loading well was gently pipetted to remove the residual gel in the well; pre-stained protein Maker and equal quality of the protein sample were carefully added into the gel well; the voltage was adjusted to 60 V to 80 V. After 30 minutes, the voltage was adjusted to 110 V to 120 V. When bromophenol blue reached the groove at the bottom of the gel, the power supply was disconnected to terminate electrophoresis, and the entire electrophoresis process took about 2 h to 2.5 h.

4.5 Electro-transfer: PVDF membrane was immersed in anhydrous methanol to activate for 1 minute; the transfer clamp was placed black-side down, sponge - filter paper - PAGE gel - PVDF membrane - filter paper - sponge were placed in order from bottom to top, and air bubbles between glue/membrane and filter paper were carefully driven out, the transfer clamp was then fasten and put into the transfer tank, with the PAGE gel facing the negative electrode and the PVDF membrane facing the positive electrode; it was placed in an ice box, and pre-cooled 1× electro-transfer solution was poured therein; the power supply was adjusted to 100 V, and membrane transferring was carried out for 1 h to 1.5 h.

4.6 Blocking: After the membrane transferring was completed, the PVDF membrane was taken out, and washed several times with 1×TBST. After the residual electro-transfer solution on the membrane was removed, the membrane was immersed in 5% skim milk for blocking for 2 hours at room temperature, or overnight at 4°C.

4.7 Incubation of primary antibody: The primary antibodies GAPDH and HCBP6 were diluted according to 1/1000; according to the molecular weight of the protein, the PVDF membrane at the corresponding position and the above antibodies were sealed in a hybridization bag, and incubated at 4°C overnight.

4.8 Washing membrane: The hybridization bag was cut to recover the primary antibodies, the PVDF membrane was taken out, placed in 1×TBST, and washed three times on a shaker, 10 minutes each time.

4.9 Incubation of secondary antibody: The secondary antibodies were diluted at a ratio of 1/5,000 to 1/10,000, the above PVDF membrane was immersed in the corresponding secondary antibody, placed on a shaker, and incubated at room temperature for 45-60 minutes.

4.10 Washing membrane: The secondary antibodies were recovered, the PVDF membrane was taken out, placed into 1×TBST, and washed three times on a shaker, 10 minutes each time; when phosphorylated protein was detected, it was necessary to appropriately reduce the washing times and washing time.

4.11 Exposure and color development: The membrane was placed in the dark box of Fusion Solo imager, added dropwise with ECL exposure solution to perform color development.

### 5. Q-PCR detection of target gene expression

### 5.1 Extraction of total cellular RNA

Test compounds were added to the LX-2 cells respectively, and total RNA in the cells was extracted 48 hours later. This experiment was performed strictly in accordance with the instructions of the Total RNA Kit. The steps were as follows (note: this experiment was performed on ice throughout the entire process, and special RNase-free tips were used throughout the entire process):
1) The 12-well cell culture plate was taken out from the incubator, the cell culture medium was discarded, the cells were washed twice with 1 mL of 1×PBS, and a negative pressure suction device was used to fully remove PBS;
2) 350 µL of TRK Lysis Buffer mixture/well was prepared as needed (mixture preparation method: 1 mL of TRKLysis Buffer + 20 µL of β-mercaptoethanol), vortexed and mixed well. 350 µL was added to each well of the 12-well cell culture plate, and allowed to stand at room temperature for 5 minutes to fully lyse the cells;
3) 70% ethanol/well was prepared as needed, vortexed and mixed well. 350 µL was added to each well of the 12-well cell culture plate, mixed well by shaking, and transferred to the spin column (spin column + collection tube were assembled);
4) Centrifugation was performed at 4°C, 12000 g for 60 sec. The waste liquid was discarded after centrifugation, while the spin column was kept;
5) 500 µL of RNA Wash Buffer I was added, centrifuged at 4°C, 12000 g for 60 sec, the waste liquid was discarded after centrifugation, and the collection column was kept;
6) 500 µL of RNA Wash Buffer II (confirmed that ethanol had been added) was added, centrifuged at 4°C, 12000 g for 60 sec, the waste liquid was discarded after centrifugation, and the collection column was kept;
7) step 6) was repeated once again, centrifugation was performed at 4°C, 12000 g for 90 sec, the waste liquid was discarded after centrifugation, and the collection column was kept;
8) Centrifugation was performed at 4°C, 12000 g for 120 sec to ensure that the spin column was fully dried;
9) The dried spin column was inserted into a new 1.5 mL EP tube, and 30 µL of DEPC water was added to the membrane in the center of the spin column. It should be careful not to touch the EP tube cover in this step. It was allowed to stand at room temperature for 5 minutes to ensure that the RNA was fully dissolved in DEPC water. Centrifugation was performed at 4°C, 12000 g for 60 sec, and the EP tube was kept;
10) NanoDrop ultra-micro-volume spectrophotometer was used to detect RNA concentration;

5.2 Reverse transcription of RNA into cDNA: According to the instructions of PrimeScript^{™} RT reagent Kit (Perfect Real Time), the specific experimental steps were as follows:

### 5.2.1 Preparation of reverse transcription reaction system (operated on ice):

**Table 1-10: Reverse transcription reaction system**

| Component | Volume |
|---|---|
| 5×PrimeScript Buffer | 4 µL |
| PrimeScript RT Enzyme Mix I | 1 µL |
| Oligo dT Primer (50 µM) | 1 µL |
| Random 6 mers (100 µM) | 1 µL |
| Total RNA | 1 µg |
| RNase Free H₂O | Supplemented to total volume of 20 µL /Sample |

5.2.2 Reverse transcription reaction conditions on machine were as follows:

**Table 1-11: Reverse transcription reaction conditions**

| Reaction temperature | Reaction time | Number of cycles |
|---|---|---|
| 37°C | 15 min | 1 |
| 85°C | 5 sec | 1 |
| 4°C | +∞ | -- |

5.2.3 The obtained product was cDNA, which could be directly used in subsequent Q-PCR experiment or stored at -20°C.

5.3 Q-PCR detection of target gene expression: According to the instructions of Power SYBR^{®} Green PCR Master Mix, the specific operations were as follows:

5.3.1 The Q-PCR reaction system was prepared according to the instructions as follows:

**Table 1-12: Q-PCR reaction system**

| Component | Volume (µL) |
|---|---|
| SYBR Green qPCR Master Mix | 5 |
| cDNA (5 times dilution) | 2 |
| Forward Primer (10 µM) | 0.25 |
| Reverse Primer (10 µM) | 0.25 |
| DNase Free dH₂O | 2.5 |
| Total | 10 µL/Sample |

5.3.2 The ABI PRISM^{®} 7500 system was used and the following two-step standard amplification procedure was adopted:

**Table 1-13: Q-PCR reaction conditions**

| Step | | Temperature (°C) | Time | Number of cycles |
|---|---|---|---|---|
| 1 | Pre-denaturation | 95 | 2 min | 1 |
| 2 | Thermal cycle | 95 | 15 sec | 40 |
| | | 60 | 1 min | |
| 3 | Melting curve procedure | | | |

After the reaction was completed, the amplification curve and dissolution curve of Q-PCR were confirmed, and the relative quantification method (2^{-ΔΔC}_{T} method) was used to perform data analysis on the experimental results.

5.3.3 The primer sequences used were as follows:

**Table 1-14: Primer sequences used in Q-PCR experiment**

| Detection gene | Upstream primer (5'-3') | Downstream primer (5'-3') |
|---|---|---|
| Actin | GTACTCTGTGTGGATCGGTGG | GCAGCTCAGTAACAGTCCG |
| COL1A1 | TTTGGATGGTGCCAAGGGAG | CACCATCATTTCCACGAGCA |
| COL3A1 | TCGCCCTCCTAATGGTCAAG | TTCTGAGGACCAGTAGGGCA |
| COL1A2 | AAAGAACCCAGCTCGCACAT | ACCAGTTCTTGGCTGGGATG |
| α-SMA | GGGAATGGGACAAAAAGACA | CTTCAGGGGCAACACGAA |
| Smad3 | TGGACGCAGGTTCTCCAAAC | CCGGCTCGCAGTAGGTAAC |
| IL-1β | TGGCAATGAGGATGACTTGT | GTGGTGGTCGGAGATTCGTA |
| IL-18 | ATCGCTTCCTCTCGCAACAA | TCCAGGTTTTCATCATCTTCAGC |
| TGFβ | CAGTGGTTGAGCCGTGGAG | CCGGTAGTGAACCCGTTGATGT |
| NS3TP1 | GATGTCCTGAGTGTAGCAGTCA | TGCTTTCCTATCACACGTTTTCA |

### (b) In vivo pharmacodynamic evaluation of compounds

### 1. Establishment and treatment of carbon tetrachloride-induced liver fibrosis model in mice

Long-term intraperitoneal injection of carbon tetrachloride (CCl₄) induced reversible liver fibrosis, which was often used to screen and evaluate anti-hepatic fibrosis drugs. C57BL/6 wild-type mice were randomly divided into control group, carbon tetrachloride group and treatment group. The treatments were as follows:
Mice in the carbon tetrachloride group: The animals were intraperitoneally injected with carbon tetrachloride in a total amount of 0.5 µL/g, diluted with corn oil to 10 µL/g, 3 times/week. After 4 weeks of modeling, the carbon tetrachloride group was randomly divided into groups, plus the negative control group:
(1) Mice in the negative control group: The animals were intraperitoneally injected with corn oil solution, 10 µL/g, 3 times/week; at the same time, 200 µL of drinking water was administered by gavage.
(2) Mice in the carbon tetrachloride group: The animals were intraperitoneally injected with carbon tetrachloride in a total amount of 0.5 µL/g, diluted with corn oil to 10 µL/g, 3 times/week; 200 µL of normal saline was administered by gavage.
(3) Mice in the aspartic acid group: The animals were intraperitoneally injected with carbon tetrachloride in a total amount of 0.5 µL/g, diluted with corn oil to 10 µL/g, 3 times/week; 30 mg/kg aspartic acid was administered by gavage.
(4) Mice in the treatment group: The animals were intraperitoneally injected with carbon tetrachloride in a total amount of 0.5 µL/g, diluted with corn oil to 10 µL/g, 3 times/week; different doses of the compounds of the present invention were administered by gavage.

The animals were treated for 4 weeks, and blood was collected from the eyeballs 48 hours after the last injection of carbon tetrachloride. About 1 mL of whole blood per animal was collected in an anticoagulant tube, and immediately placed on ice and allowed to stand for 1 hour. Centrifugation was performed at 4°C, 3000 rpm for 15 min. About 0.4 mL of the supernatant was taken and added into an EP tube, in which it should be carefully avoided to pipet the lower layer liquid, and then it was stored in a -80°C refrigerator. The mice were euthanized, and fresh liver tissue was taken, part of which was placed in a cryopreservation tube and stored in liquid nitrogen for later use; the other part was fixed in 4% paraformaldehyde solution for 16 h to 24 h for subsequent experiments.

### 2. Establishment and treatment of mouse NAFLD model induced by HFD or CHOL

(1) C57BL/6J male mice aged 6 to 8 weeks were selected and randomly divided into normal diet group (n=10) and HFD or CHOL group; the weight changes of the mice were recorded during the modeling period, and the modeling was conducted for 8 weeks (CHOL) or 10 weeks (HFD), then the mice in the HFD or CHOL group were randomly divided into groups according to their body weights, plus the negative control group, a total of 5 groups:
(2) Mice in the negative control group (n=10): The animals were fed with normal diet, and 200 µL of drinking water was administered by gavage, bid.
   Mice in HFD or CHOL group (n=10): The animals were fed with HFD or CHOL diet, and 200 µL of normal saline was administered by gavage at the same time, bid. (Note: HFD (60% high fat) feed (Whitby Technology Development (Beijing) Co., Ltd.) (D12492), CHOL (40% high fat + 1.25% cholesterol) feed (Whitby Technology Development (Beijing) Co., Ltd.) (D12108C))
(3) Mice in Compound 6-low dose group (n=10): The animals were fed with HFD or CHOL diet, and administrated simultaneously with Compound **6** at a dose of 10 mg/kg by gavage, bid.
(4) Mice in Compound **6**-middle dose group (n=10): The animals were fed with HFD or CHOL diet, and administrated simultaneously with Compound **6** at a dose of 50 mg/kg by gavage, bid.
(5) Mice in Compound **6**-high dose group (n=10): The animals were fed with HFD or CHOL diet, and administrated simultaneously with Compound **6** at a dose of 150 mg/kg by gavage, bid.

After 8 weeks of administration, blood was collected by enucleating the eyeballs. About 1 mL of whole blood per animal was collected in an anticoagulant tube, and immediately allowed to stand on ice. Centrifugation was performed at 4°C, 3000 rpm for 15 min. 0.4ml of the supernatant was taken and added into an EP tube, and it should be carefully avoided to pipette the lower layer liquid, and then it was stored in a -80°C refrigerator to detect biochemical indicators such as blood lipid and transaminase. The mice were euthanized, and fresh liver tissue was taken and weighed, part of which was placed in a cryopreservation tube and stored in liquid nitrogen for later use, and the other part was fixed in 4% paraformaldehyde solution for 16 h to 24 h, and underwent histological HE staining and oil red O staining to determine the severity of fatty liver.

### 3. Histological analysis of liver

### (1) Paraffin embedding

(1 Fixation: The mice were treated by following standard procedures, and the tissue was taken out, and placed and fixed in 4% paraformaldehyde for 24 hours;
(2 Dehydration: The fixed tissue was placed in 70% ethanol overnight, and then placed in 85%, 95%, 95%, 95%, 100%, and 100% ethanol for 1 hour each time in sequence for step-by-step dehydration;
(3 Transparency: The dehydrated tissue was placed in 50% xylene (equal volume of xylene and ethanol), 100% xylene, and 100% xylene for 20 minutes each time in sequence for transparent processing;
(4 Dipping wax: The transparent tissue was placed in paraffin at 60°C, and the paraffin was renewed every 1 hour for a total of four times. It should be noted to use new paraffin when renewed;
(5 Embedding: Melted paraffin (60°C) was added into a small iron trough, the paraffin-soaked tissue was taken and placed therein, the embedding box at the side with label was buckled, and the melted paraffin was added again. It should be noted that air bubbles should be removed during the operation process;
(6 The paraffin-embedded tissue was placed on an ice table of an automatic embedding machine. After the paraffin solidified, it was placed in a -20°C refrigerator for further cooling.

### (2) Continuous slicing

(1 The wax block with embedded tissue was placed at the corresponding position of a microtome;
(2 The slice thickness was adjusted to the thickness required for the experiment. Generally, 3 to 4 µm should be used for slicing;
(3 6 to 8 Consecutive tissue sections were gently placed and fully stretched in a 55°C water bath expander;
(4 The continuous tissue sections were gently separated by tweezers;
(5 A wax section that was completely stretched and had no wrinkles in the tissue was selected, and taken out with an adhesive slide so that the section was attached to the slide;
(6 The slide was placed horizontally on a 70°C slide dryer for preliminary drying, and then placed in a 70°C oven to be baked for about 1 hour.

### (3) H&E staining (hematoxylin eosin staining, H&E)

(1 Baking section: The tissue section was placed in a 70°C oven for 1 hour;
(2 Dewaxing: The tissue section was placed in xylene (I) → xylene (II) → xylene (III) for 10 minutes each time in sequence for dewaxing;
(3 Rehydration: The dewaxed tissue section was placed in sequence in 100% ethanol for 2 min → 100% ethanol for 2 min → 95% ethanol for 2 min → 85% ethanol for 2 min → 70% ethanol for 2 min → washing with distilled water for 3 min;
(4 Staining cell nuclei: The rehydrated tissue section was placed in hematoxylin solution for staining for about 3 to 5 minutes. The specific time depended on the observation under the microscope; the section was washed with tap water twice, for dozens of seconds each time;
(5 Differentiation: The section was placed in 1% solution of hydrochloric acid in ethanol (prepared with 70% ethanol) for dozens of seconds, and the time was controlled by observing under the microscope until the nuclei were clearly stained;
(6 Turning blue: The section was rinsed with running water for about 15 to 30 minutes until it turned into blue; then the tissue section was placed in distilled water for short rinsing;
(7 The section was placed in an eosin solution and stained for about 30 seconds. The time was adjusted by observing under the microscope. If staining was difficult, 1 to 2 drops of glacial acetic acid could be added to the staining solution to make the tissue section easier to stain and less likely to decolor;
(8 Dehydration: The tissue section was placed in sequence in 70% ethanol for 2 min → 85% ethanol for 2 min → 95% ethanol for 2 min → 100% ethanol for 2 min → 100% ethanol for 2 min;
(9 Transparency: The section was placed in sequence in xylene (I) → xylene (II) → xylene (III) for 10 minutes each time;
(10 Sealing section: An appropriate amount of neutral gum was dropped to the tissue, and covered with a coverslip to seal the section, in which bubbles should be avoided.

### (4) Masson staining

Masson staining is one of the classic and authoritative staining methods for collagen fiber staining. Collagen fibers appear red. The staining steps were as follows:
Conventional baking and dewaxing (steps were the same as before); oxidization was performed with potassium permanganate solution for 5 minutes, washing was performed with water; staining was performed with Masson staining solution for 5 minutes; 0.2% acetic acid solution for 2 to 3 seconds; 5% phosphotungstic acid solution for 5 minutes; aniline blue solution for 7 minutes; washing was performed 3 times with 0.2% acetic acid solution; dehydration, transparency of section, sealing of section, natural drying, and storage at room temperature were performed by routine methods.

An imaging system was used to collect images of stained tissue on the section, and an analysis software was used to automatically read the tissue measurement area, calculate the positive area and tissue area in the measurement area, and calculate the proportion of positive area.

### 4. Testing of liver function indicators

Mouse serum was taken to detect the related levels of ALT and AST in the serum.

### 5. Q-PCR detection of target gene expression in mouse liver tissue

### (1) Extraction of total tissue RNA

(1 10 mg of tissue was taken, weighed and placed in a grinder, added with liquid nitrogen, and ground thoroughly with a grinding rod. During the grinding process, it should be carefully noted to add liquid nitrogen at any time to prevent tissue melting that could result in RNA degradation;
(2 The ground tissue was placed in a 1.5 mL EP tube, added with 1 mL of Trizol lysis solution, and mixed well;
(3 The sample was placed on a thermostatic mixer and shaken at room temperature for about 3 to 5 hours until the tissue was completely dissolved;
(4 200 mL of chloroform was added, shaken vigorously for dozens of seconds, and allowed to stand at room temperature for 5 to 10 minutes;
(5 Centrifugation was performed at 4°C, 12,000 rcf for 15 minutes; 75% alcohol (prepared with DEPC water) was prepared during the centrifugation, and stored at -20°C for later use;
(6 The supernatant after centrifugation was taken, placed in a new EP tube, added with the same volume of isopropanol, inverted up and down 10 times, and allowed to stand at room temperature for 10 minutes;
(7 Centrifugation was performed at 4°C, 12,000 rcf for 15 to 20 minutes;
(8 The supernatant was discarded, the precipitate was kept, added with 1 mL of pre-cooled 75% ethanol to wash away isopropanol, then centrifuged at 4°C, 12,000 rcf for 10 minutes; this step was repeated once, and then the precipitate was air dried;
(9 According to the amount of the precipitation, 30 to 100 µL of DEPC water was added to dissolve the precipitation, which was the extracted tissue RNA.
(10 The extracted RNA was measured using a UV spectrophotometer to determine its concentration and purity, and stored at -80°C.

### (2) Reverse transcription of RNA into cDNA:

According to the instructions of PrimeScript^{™} RT reagent Kit (Perfect Real Time), the specific experimental steps were as follows:

### (1 Preparation of reverse transcription reaction system (operation on ice):

**Table 3-1: Reverse transcription reaction system**

| Component | Volume |
|---|---|
| 5×PrimeScript Buffer | 4 µL |
| PrimeScript RT Enzyme Mix I | 1 µL |
| Oligo dT Primer (50 µM) | 1 µL |
| Random 6 mers (100 µM) | 1 µL |
| Total RNA | 1 µg |
| RNase Free H₂O | Make up to a total volume of 20 µL/Sample |

### (2 Reverse transcription reaction conditions were as follows:

**Table 3-2: Reverse transcription reaction conditions**

| Reaction temperature | Reaction time | Number of cycles |
|---|---|---|
| 37°C | 15 min | 1 |
| 85°C | 5 sec | 1 |
| 4°C | +∞ | -- |

The obtained product was cDNA, which could be directly used in subsequent Q-PCR experiment or stored at -20°C.

### (3) Q-PCR detection of target gene expression:

According to the instructions of Power SYBR^{®} Green PCR Master Mix, the specific operations were as follows:
(1 Q-PCR reaction system was prepared according to the instructions as follows:

**Table 3-3: Q-PCR reaction system**

| Component | Volume (µl) |
|---|---|
| SYBR Green qPCR Master Mix | 5 |
| cDNA (5 times dilution) | 2 |
| Forward Primer (10 µM) | 0.25 |
| Reverse Primer (10 µM) | 0.25 |
| DNase Free dH₂O | 2.5 |
| Total | 10 µl/Sample |

(2 ABI PRISM^{®} 7500 system was used and the following two-step standard amplification procedure was adopted:

**Table 3-4: Q-PCR reaction conditions**

| Step | | Temperature (°C) | Time | Number of cycles |
|---|---|---|---|---|
| 1 | Pre-denaturation | 95 | 2 min | 1 |
| 2 | Thermal cycle | 95 | 15 sec | 40 |
| | | 60 | 1 min | |
| 3 | Melting curve procedure | | | |

After the reaction was completed, the amplification curve and dissolution curve of Q-PCR were confirmed, and the relative quantification method (2^{-ΔΔC}_{T} method) was used to perform data analysis on the experimental results.
(3 The primer sequences used were as follows:

**Table 3-5: Primer sequences used in Q-PCR experiment**

| Detection gene | Upstream primer (5'-3') | Downstream primer (5'-3') |
|---|---|---|
| Mus-β-actin | GGCTGTATTCCCCTCCATCG | CCAGTTGGTAACAATGCCATGT |
| Mus-COL1A1 | CTGGCGGTTCAGGTCCAAT | TTCCAGGCAATCCACGAGC |
| Mus-COL3A1 | CCTGGCTCAAATGGCTCAC | GACCTCGTGTTCCGGGTAT |
| Mus-α-SMA | GGCACCACTGAACCCTAAGG | ACAATACCAGTTGTACGTCCAGA |
| Mus-NS3TP1 | TTGCTGCATAATCTTAGACGGC | ACAGGTTGGATAGTCAAAACACC |

### (c) Thermodynamic solubility test of compounds

Several portions of the drug was taken, and used to prepare a series of solutions from an unsaturated solution to a saturated solution, which were oscillated under constant temperature conditions until equilibrium was reached, and filtered through a membrane, the filtrate was taken for analysis to determine the actual concentration S of the drug in the solution, and a graph for the concentration c of the prepared solution was drawn, and the turning point A of the curve in the graph was the equilibrium solubility of the drug.

Thermodynamic solubility was measured using the shake flask method (pH=7.4) and analyzed using high-performance liquid chromatography-diode array detector (HPLC-DAD) or high-performance liquid chromatography-tandem mass spectrometry (LC/MS/MS).

### (d) Pharmacokinetic testing

1. Research purpose:
   Rats were used as test animals to study the pharmacokinetic behavior of the following compounds in rat plasma.
2. Experimental protocol:
   2.1 Experimental drugs:
      The drugs were self-made according to the examples of the present invention.
   2.2 Experimental animals:
      SD rats, 6-8 weeks old, 3 for each administration method/example, male.
   2.3 Eating status:
      The animals were fasted overnight, ate food 8 hours after taking the drug, and drank water freely.
   2.4 Sample collection:
      Blood was collected before administration and at 5min, 15min, 30min, 1hr, 2hr, 4hr, 8hr and 24hr after administration.
   2.5 Sample processing:
      The blood was placed on wet ice and centrifuged to obtain plasma sample (2000 g, 5 min at 4°C).
   2.6 Analysis by liquid chromatography
      The LC-MS/MS method was used to determine the blood drug concentration. The HPLC conditions were: mobile phase A: H₂O-0.5%FA; mobile phase B: ACN-0.5%FA;
3. Test results and analysis

WinNonlin 8.2 non-compartmental model was used to estimate pharmacokinetic parameters (PK parameters, including but not limited to peak concentration (Cmax), time to peak (Tmax), terminal elimination rate (Ke), terminal elimination half-life (T1/2), area under drug-time curve (AUC), clearance rate (CL), apparent volume of distribution (Vd), mean residence time (MRT), bioavailability (F), etc.).

### Test Results:

### (a) Regulation of NS3TP1 in semi-activated LX2 cells - qPCR

LX2 cells were passaged and plated normally. After 12 hours of adherent growth, 200 µM of the compound to be tested was added. After 48 hours, the cells were collected, total RNA was extracted, and the expression of NS3TP1 gene was detected using Q-PCR method.

The results (Table 1) show that the compounds of the present invention could up-regulate the mRNA expression level.

**Table 1: Up-regulation of NS3TP1 expression in LX-2 cells**

| Example | Up-regulation of NS3TP1 |
|---|---|
| 4** | B |
| 6 | A |
| 54 | A |
| 77a | C |
| 78a* | C |

| | |
|---|---|
| Note: Compared with the control group, NA indicated that no obvious up-regulation was observed; C represented that the up-regulation was 1-20%; B represented that the up-regulation was 20-40%; A represented that the up-regulation was 40% or above; "-" indicated that no result was provided; the compound marked with "*" had a test concentration of 12.5 µM; the compound marked with "**" had a test concentration of 10 mM. | |

### (b) In vitro drug efficacy evaluation in semi-activated LX2 cells - qPCR

LX2 cells were passaged and plated normally. After adherent growth for 12 hours, 50 µM of the test compound was added. After 48 hours, the cells were collected, total RNA was extracted, and Q-PCR was used to detect the changes in the mRNA expression levels of liver fibrosis-related genes (ACTA2 encoding α-SMA, COL1A1 and COL1A2 encoding collagen I, COL3A1 encoding collagen III, SMAD3 encoding Smad3) and inflammation-related genes (IL1B encoding IL-1β, TNFa encoding TNFα) (for specific methods, see 5. Q-PCR detection of target gene expression as described above). α-SMA was a marker of hepatic stellate cell activation, and collagen I and collagen III were the main components of extracellular matrix deposition.

The results (Table 2) showed that the compounds of the present invention could inhibit the mRNA expression levels of liver fibrosis-related genes. Among them, Compound **2,** Compound **4,** Compound **6,** Compound **8,** Compound **10,** Compound **12,** Compound **14,** Compound **15,** Compound **18,** Compound **19,** Compound **21,** Compound **22,** Compound **26,** Compound **28,** Compound **30,** Compound **33,** Compound **35,** Compound **38,** Compound **40,** Compound **42,** Compound **44,** Compound **47,** Compound **48,** Compound **50,** Compound **52,** Compound **53,** Compound **54,** Compound **59,** Compound **61,** Compound **63,** Compound **66,** Compound **70,** Compound **71,** Compound **72,** Compound **74,** etc., could inhibit the expression of collagen I and collagen III at the mRNA level; Compound **4,** Compound **6,** Compound **26,** Compound **28,** Compound **33,** Compound **53,** Compound **54,** Compound **68,** Compound **70,** Compound **73,** etc., could inhibit the expression of α-SMA at the mRNA level; Compound **1,** Compound **4,** Compound **6,** Compound **9,** Compound **12,** Compound **15,** Compound **22,** Compound **26,** and Compound **28** could inhibit the expression of Smad3 at the mRNA level. Overall, Compound **4,** Compound **6,** Compound **12,** Compound **26,** Compound **47,** Compound **48,** Compound **52,** and Compound **54** had stronger effects to inhibit the mRNA expression levels of liver fibrosis-related genes.

**Table 2**

| Example | COL1A1 | COL1A2 | COL3A1 | α-SMA | Smad3 | IL-1β | TNF-α |
|---|---|---|---|---|---|---|---|
| 1 | E | NA | NA | E | D | NA | E |
| 2 | C | E | D | E | E | E | D |
| 3* | C | E | E | NA | E | E | E |
| 4 | C | E | C | C | C | E | C |
| 5 | NA | NA | NA | NA | NA | E | NA |
| 6 | C | E | C | D | C | E | D |
| 7** | NA | NA | NA | NA | NA | E | NA |
| 8 | D | NA | D | NA | E | E | NA |
| 9 | NA | NA | NA | E | D | C | NA |
| 10 | E | E | D | E | E | E | D |
| 11 | NA | NA | NA | NA | E | C | E |
| 12 | C | E | C | E | D | E | D |
| 13 | NA | E | NA | E | NA | E | E |
| 14 | D | NA | E | NA | NA | NA | E |
| 15 | D | NA | D | E | D | NA | E |
| 16 | E | NA | NA | NA | NA | NA | E |
| 17 | E | NA | NA | E | E | NA | D |
| 18 | D | E | E | NA | NA | NA | NA |
| 19 | D | D | D | NA | NA | NA | NA |
| 20* | E | NA | E | E | E | NA | NA |
| 21 | E | NA | D | E | E | NA | NA |
| 22 | D | NA | E | NA | D | E | E |
| 23 | E | E | E | NA | E | E | E |
| 24 | E | NA | E | NA | NA | NA | NA |
| 25 | E | NA | NA | E | NA | NA | NA |
| 26 | C | E | C | D | D | NA | D |
| 27 | NA | NA | NA | NA | E | NA | NA |
| 28 | D | E | D | D | D | D | E |
| 29 | E | E | NA | E | NA | NA | NA |
| 30 | C | E | NA | E | NA | D | NA |
| 31 | E | NA | NA | E | NA | E | NA |
| 32 | D | NA | E | E | NA | NA | NA |
| 33 | C | E | E | C | E | E | NA |
| 34 | NA | NA | E | E | E | E | E |
| 35 | D | NA | E | NA | NA | NA | NA |
| 36 | NA | NA | E | E | E | NA | E |
| 38 | C | NA | E | NA | NA | NA | NA |
| 39 | NA | E | E | NA | NA | NA | E |
| 40 | C | NA | C | NA | E | E | E |
| 41 | NA | NA | E | NA | NA | E | NA |
| 42 | C | NA | E | NA | E | E | D |
| 43 | NA | NA | E | E | E | NA | NA |
| 44 | D | NA | E | NA | NA | E | NA |
| 45** | E | E | E | E | E | E | E |
| 46** | NA | E | NA | NA | NA | NA | NA |
| 47 | C | NA | D | NA | E | E | D |
| 48 | C | NA | D | NA | NA | NA | E |
| 50 | C | NA | E | NA | NA | NA | NA |
| 51 | NA | NA | NA | E | NA | NA | NA |
| 52 | C | E | C | NA | NA | NA | NA |
| 53 | D | E | D | D | NA | NA | NA |
| 54 | C | NA | D | NA | NA | NA | E |
| 55** | E | E | E | E | NA | E | NA |
| 57** | NA | NA | NA | NA | E | NA | NA |
| 58 | NA | E | E | E | NA | E | NA |
| 59 | D | NA | NA | E | NA | NA | E |
| 60 | NA | NA | NA | E | E | NA | NA |
| 61 | C | E | E | NA | E | E | E |
| 62 | E | NA | NA | E | E | E | E |
| 63 | NA | - | D | E | - | - | - |
| 65* | D | - | E | E | - | - | - |
| 66 | C | - | NA | NA | - | - | - |
| 67 | NA | - | E | NA | - | - | - |
| 68*** | E | - | E | C | - | - | - |
| 70*** | E | - | D | B | - | - | - |
| 71* | D | - | E | NA | - | - | - |
| 72** | C | - | C | NA | - | - | - |
| 73* | NA | - | NA | D | - | - | - |
| 74*** | C | - | C | C | - | - | - |

| | | | | | | | |
|---|---|---|---|---|---|---|---|
| Note: Compared with the control group, NA indicated that no obvious inhibitory effect was observed; E represented that the inhibition rate was 1% to 10%; D represented that the inhibition rate was 10% to 25%; C represented that the inhibition rate was 25% to 50%; B represented that the inhibition rate was 50% to 75%; A represented that the inhibition rate was 75% to 100%; "-" indicated that no result was provided; the compound marked with "*" had a test concentration of 12.5 µM; the compound marked with "**" had a test concentration of 25 µM; and the compound marked with "***" had a test concentration of 100 µM. | | | | | | | |

### (c) Evaluation of drug efficacy in in-vitro liver fibrosis model in activated LX2 cells - qPCR

LX2 cells were passaged and plated normally. After 12 hours of adherent growth, TGFβ1 (5 ng/mL) was administered to activate the cells, and a compound to be tested with concentration of 25 µM was added. After 24 hours, the cells were collected, the total RNA was extracted, and the real-time PCR was used to detect the expression levels of liver fibrosis-related genes and inflammation-related genes. α-SMA was a marker of hepatic stellate cell activation, and collagen 1 and collagen 3 were the main components of extracellular matrix deposition.

**Table 3: Regulation of liver fibrosis-related gene expression in activated LX-2 cells**

| Example | collagen 1 | collagen 3 | α-SMA |
|---|---|---|---|
| 4 | A | A | B |
| 6 | B | A | A |

| | | | |
|---|---|---|---|
| Note: Compared with the control group, NA indicated that no obvious inhibitory effect was observed; C represented that the inhibition rate was 1% to 10%; B represented that the inhibition rate was 10% to 25%; A represented that the inhibition rate was 25% or above; "-" indicated that no result was provided. | | | |

The results showed that in this test, the compounds of the present invention could inhibit the mRNA expression levels of liver fibrosis-related genes.

### (d) In vitro drug efficacy evaluation in semi-activated LX2 cells - Western blot

LX2 cells were passaged and plated normally. After 12 hours of adherent growth, a compound to be tested was added at different concentrations (50 µM, 100 µM, 200 µM, 400 µM). After 48 hours, the cells were collected, proteins were extracted, and Western blot was used to detect the changes in expression levels of proteins (α-SMA, collagen I, collagen III, FN) (for specific methods, see 4. Detection of protein expression level by protein immunoblotting) of liver fibrosis-related genes. α-SMA was a marker of hepatic stellate cell activation, collagen I and collagen III were the main components of extracellular matrix deposition, and fibronectin (FN) was an adhesion glycoprotein mainly involved in cell adhesion interaction.

The results showed that the compounds of the present invention could inhibit the mRNA expression levels of liver fibrosis-related genes. The results were not fully displayed (con was the control well, P1 was the positive control well, "ASP-50" was 50 µM aspartic acid, and the other numbers were all in the form of compound number-concentration (µM). Among them, Compound **6,** Compound **47,** and Compound **54** had stronger inhibitory effects on collagen I and collagen III protein expression (Figure 1) as compared to ASP; Compound **4,** Compound **6,** Compound **41,** Compound **47,** Compound **48,** and Compound **52** had stronger inhibitory effects on α-SMA protein (Figure 2) as compared to ASP; and Compound **50,** Compound **52,** and Compound **54** had stronger inhibitory effects on FN protein expression (Figure 3) as compared to ASP.

### (e) In vitro drug efficacy evaluation in activated LX2 cells - Western blot

LX2 cells were passaged and plated normally. After 12 hours of adherent growth, TGFβ1 (5 ng/mL) was administered to activate the cells, and a compound to be tested was added at different concentrations. After 24 hours, the cells were collected, proteins were extracted, and Western blot was used to detect the changes in expression levels of proteins (α-SMA, collagen I) of liver fibrosis-related genes (for specific methods, see 4. Detection of protein expression level by protein immunoblotting). α-SMA was a marker of hepatic stellate cell activation, and collagen I was the main component of extracellular matrix deposition.

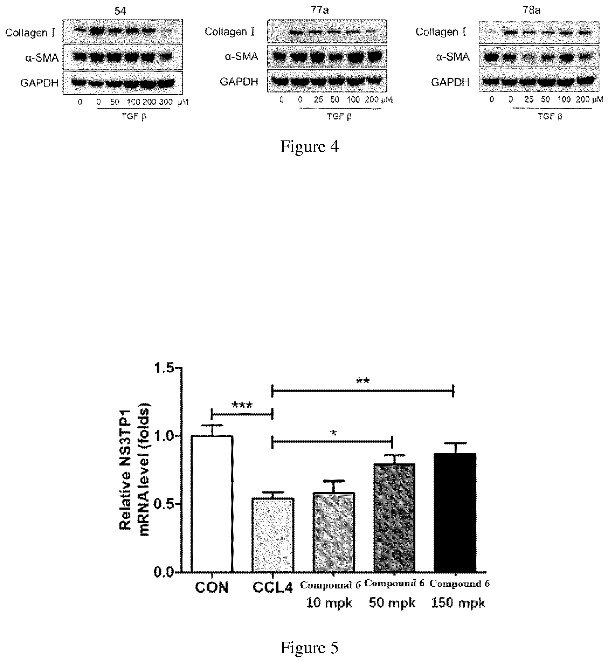

The results showed that after stimulation of LX2 cells by TGFβ, the expression of collagen I in the cells increased. After the administration of Compounds **54, 77a,** and **78a,** the expression of Collagen I and α-SMA in the cells significantly decreased (Figure 4).

### (f) Regulatory effect of compounds on NS3TP1 gene in animal models of liver fibrosis

After CCl4 induction, the expression of NS3TP1 in mouse liver tissue decreased significantly, indicating that there could be a correlation between mouse liver fibrosis and abnormal expression of NS3TP1. After treatment with different doses of Compound 6, compared with the untreated group, the expression of NS3TP1 in the liver tissue of mice gradually increased in a dose-dependent manner (Figure 5).

### (g) Efficacy of compounds on liver fibrosis model animals

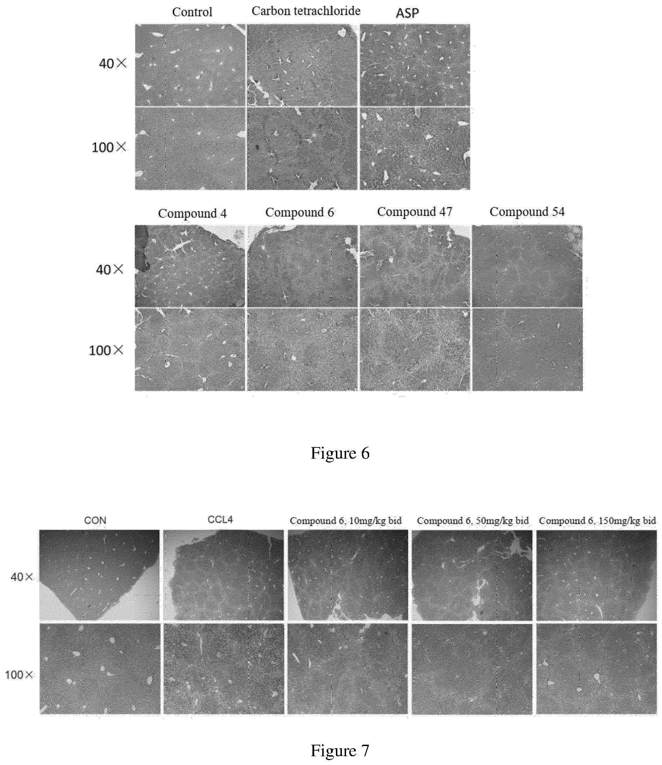

Observation and computer analysis of Masson-stained sections showed that collagen (blue) was obviously deposited in mouse liver tissue after CCl₄ induction. After treatment with Compound **4** (20 mg/kg/d), Compound **6** (25 mg/kg/d), Compound **47** (20 mg/kg/d), and Compound **54** (40 mg/kg/d), compared with the untreated group and the aspartic acid group (30 mg/kg/d), collagen deposition in the mouse liver tissue was reduced, indicating that the compounds of the present invention (e.g., Compound **4,** Compound **6,** Compound **47,** Compound **54**) could have the ability to reduce collagen deposition and inhibit liver fibrosis (Figure 6).

Observation and computer analysis of Masson-stained sections showed that collagen (blue) was obviously deposited in mouse liver tissue after CCl₄ induction. After treatment with Compound 6 (10 mg/kg/bid, 50 mg/kg/bid, 150 mg/kg/bid), compared with the untreated group, collagen deposition in the mouse liver tissue decreased, indicating that the compounds of the present invention (e.g., Compound **6**) could have the ability to reduce collagen deposition and inhibit liver fibrosis (Figure 7).

After CCl₄ induction, the levels of aminotransferases ALT and AST in mice significantly increased, and obvious liver damage occurred. After administration of the compounds of the present invention, compared with the untreated group, the ALT in the plasma of mice decreased, indicating that the compounds of the present application had protective effects on liver cells, and could reduce liver damage and improve liver function in mice.

It could be seen from the activity results that,
1. in the mouse model of liver fibrosis induced by CCl₄, the expression level of NS3TP1 in liver tissue decreased, indicating that there could be a correlation between liver fibrosis and abnormal expression of NS3TP1 in mice.
2. from the activity characterization results, it could be seen that the compounds of the present invention had the effect of up-regulating the expression of NS3TP1 in the cell experiment and the in vivo experiment.
3. from the activity characterization results, it could be seen that the compounds of the present invention also showed the effect of alleviating the symptoms of liver fibrosis in liver fibrosis models at the cellular level and animal level.

### (h) Efficacy of compounds on NAFLD model animals

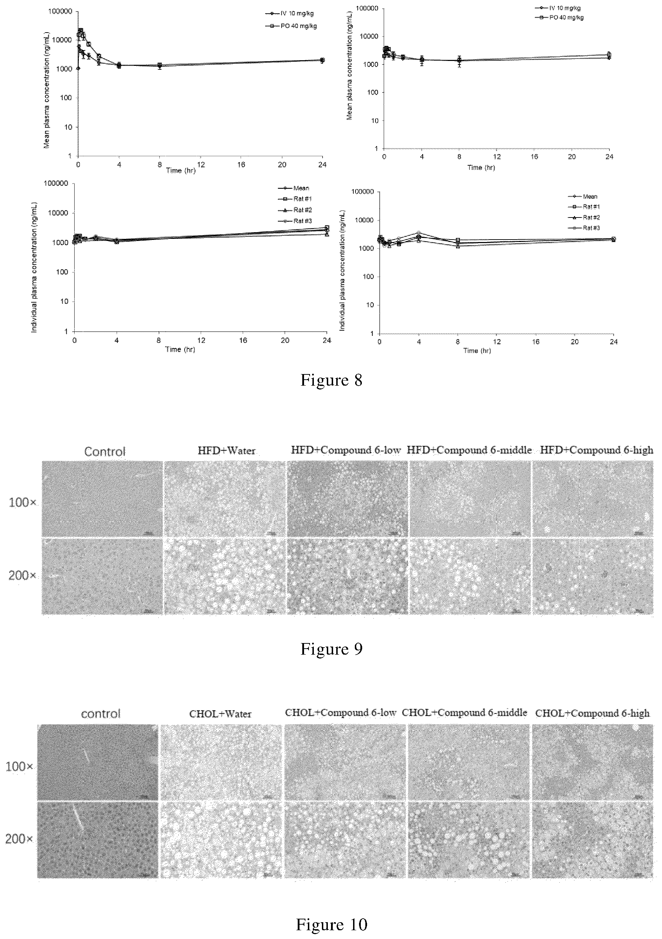

Observation and computer analysis of H&E stained sections showed that after being fed with HFD diet, a large number of lipid droplets accumulated in the liver tissue of mice, and the liver tissue showed soapy diffuse fatty change. After treatment with Compound **6,** compared with the untreated group, lipid droplet aggregation in the liver tissue of mice was reduced in a dose-dependent manner, indicating that the compounds of the present invention (e.g., Compound **6**) could have the effect of inhibiting the formation of fatty liver (Figure 9).

Observation and computer analysis of H&E stained sections showed that after being fed CHOL diet, a large number of lipid droplets accumulated in the liver tissue of mice, and the liver tissue showed soapy diffuse fatty change. After treatment with Compound **6,** compared with the untreated group, lipid droplet aggregation in the liver tissue of mice was reduced in a dose-dependent manner, indicating that the compounds of the present invention (e.g., Compound **6**) could have the effect of inhibiting the formation of fatty liver (Figure 10).

### (i) Solubility testing

Some compounds of the present invention showed good solubility, which was obviously better than that of aspartic acid.

### (j) Pharmacokinetic testing of compounds

The results showed that after the rats were administered with physiological saline, the concentrations of Compound **6** and aspartic acid in the rats fluctuated between 1000 and 3000 ng/mL within 24 hours, suggesting that Compound **6** of the present invention and aspartic acid are endogenous substances of rats;

After administration of Compound **6** to rats (IV 10 mg/kg, PO 40 mg/kg), the level of Compound **6** in the body was significantly increased (the Cmax of IV 10 mg/kg was 6100 ng/mL, and the Cmax of PO 40 mg/kg was 22500 ng/mL), but no significant increase in level of aspartic acid was observed (the Cmax of IV 10 mg/kg was 3017 ng/mL, and the Cmax of PO 40 mg/kg was 4020 ng/mL), suggesting that after the administration of Compound **6** in rats, it mainly exerted its pharmacological effects in the prototype form rather than metabolizing into aspartic acid to exert its pharmacological effects.

After aspartic acid and Compound **6** were administered to rats respectively (PO 20 mg/kg), it could be seen that the Cmax and AUC of the rats administrated with Compound 6 were significantly higher than those of rats administrated with aspartic acid, suggesting that Compound **6** had a significant increase in in vivo exposure amount as compared with aspartic acid. At the same time, it could be seen that the Tmax of rats administered with Compound **6** was significantly smaller than that of rats administered with aspartic acid, suggesting that Compound **6** could reach the highest concentration faster.

**Table 4: Pharmacokinetic parameters of compounds in rats**

| | Aspartic acid | Compound 6 |
|---|---|---|
| Tmax (hr) | 24 | 0.5 |
| Cmax (ng/mL) | 1960 | 14800 |
| AUClast (hr*ng/mL) | 26671.815 | 71261.16 |

The results showed that the compounds of the present invention had good pharmacokinetic parameters, performed better than aspartic acid in multiple pharmacokinetic parameters such as AUC, Cmax and/or bioavailability, and had better druggability.

## Claims

1. Use of a compound represented by the following Formula I, or pharmaceutically acceptable salt, ester, prodrug, stereoisomer, hydrate, solvate, isotopic compound, crystalline form, metabolite form thereof, or any combination or mixture thereof, in the manufacture of a medicament or agent, wherein the medicament is used for at least one of the following:
1) treating, improving, or preventing a liver disease (e.g., NAFLD, NASH, liver fibrosis);
2) alleviating a mammalian liver fibrosis, liver steatosis, or inflammation;
3) inhibiting the activation of stellate cells;
4) regulating the expression of NS3TP1; A-(L₁)ₓ-A' (Formula I)
wherein, A is 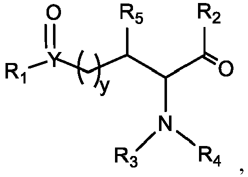 A' is 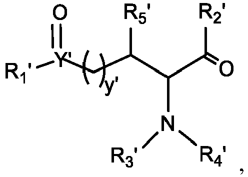 A and A' are the same or different;
wherein:
x is selected from the group consisting of 0, 1 and 2;
1) when x is 0, A' is absent; for A, wherein:
R₁ and R₂ are each independently selected from the group consisting of: C1-C6 alkyl, -O-R^{a1}, and -NR^{a2}R^{a3};
R₃ and R₄ are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, -C(R^{h})₂-R^{d1}, -(CO)-(L)ₙ-R^{d2}, -(CO)O-(L)ₙ-R^{d3}, -(SO₂)-(L)ₙR^{d4}, and -P(O)(OR^{e})₂, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), and heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-O-(CO)-O-R^{b4}, -(C1-C6 alkyl)ₙ-(CO)-NR^{b5}R^{b6}, -(C1-C6 alkyl)ₙ-NR^{b7}-(CO)-R^{b8}, and -(C1-C6 alkyl)ₙ-NR^{b9}-(CO)-O-R^{b10}, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
R^{b1}, R^{b2}, R^{b3}, R^{b4}, R^{b5}, R^{b6}, R^{b7}, R^{b8}, R^{b9}, R^{b10} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-O-R^{c1}, -(C1-C6 alkyl)ₙ-O-(CO)-R^{c2}, and -(C1-C6 alkyl)ₙ-O-(CO)-O-R^{c3}, the alkyl, cycloalkyl, aryl, heterocyclyl are optionally substituted with one or more groups independently selected from the group consisting of halogen, amino, hydroxyl, carboxyl, C1-C6 alkyl, and C6-C10 aryl;
R^{c1}, R^{c2}, and R^{c3} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, and 3- to 15-membered heterocyclyl;
L is each independently selected from the group consisting of: absence, O, NR^{d5}, C1-C6 alkyl, C2-C6 alkenyl, and C2-C6 alkynyl;
R^{e} is each independently selected from the group consisting of hydrogen, deuterium, Na, K, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl and heterocyclyl are optionally substituted with one or more groups independently selected from the group consisting of halogen, C1-C6 alkyl, and halogenated C1-C6 alkyl;
R^{h} is each independently selected from the group consisting of: C1-C6 alkyl, -NHC(O)R^{g1}, -OC(O)R^{g2}, -OP(O)(ONa)₂, -NHC(O)OR^{d6}, -OC(O)OR^{d7}, and -OC(O)NHR^{d8};
R^{d1}, R^{d2}, R^{d3}, R^{d4}, R^{d5}, R^{d6}, R^{d7}, R^{d8} are each independently selected from the group consisting of: hydrogen, deuterium, amino, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, heterocyclyl are optionally substituted with one or more R^{f} groups;
R^{f} is each independently selected from the group consisting of halogen, C1-C6 alkyl, halogenated C1-C6 alkyl, C2-C6 alkenyl, C2-C6 alkynyl, C6-C10 aryl, halogenated C6-C10 aryl, C3-C7 cycloalkyl, 3- to 15-membered heterocyclyl, -NR^{g3}R^{g4}, -OR^{g5}, -NHC(O)R^{g6}, and -OC(O)R^{g7};
R^{g1}, R^{g2}, R^{g3}, R^{g4}, R^{g5}, R^{g6}, and R^{g7} are each independently selected from the group consisting of: hydrogen, C1-C6 alkyl, C2-C6 alkenyl, C2-C6 alkynyl, C3-C7 cycloalkyl, C6-C10 aryl, and 3-to 15-membered heterocyclyl;
R₅ is selected from the group consisting of: hydrogen, deuterium, C1-C6 alkyl, halogen, and amino; or, R₅ is selected from the group consisting of hydrogen and amino;
Y is C or S;
y is an integer of 0 or above; or y is selected from the group consisting of 0, 1, 2, and 3; or y is 0 or 1;
n is 0 or 1;
2) when x is 1 or 2, A and A' are the same or different, wherein:
any one of R₁, R₂, R₃, and R₄ of a unit A is connected to any one of R₁', R₂', R₃', and R₄' of an adjacent unit A' through Li;
each Li is independently a unit-linking group selected from the group consisting of: absence, O, S, carbonyl, alkyl (or C1-C6 alkyl), alkenyl (or C2-C6 alkenyl), alkynyl (or C2-C6 alkynyl), -O-alkenyl-O- (or -O-C2-C6 alkenyl-O-), -O-alkynyl-O- (or -O-C2-C6 alkynyl-O-), cycloalkyl (or C3-C7 cycloalkyl), and heteroalkyl (or 3- to 7-membered heteroalkyl; for example, -O-alkyl-O- (or -O-C1-C6 alkyl-O-)), wherein the alkyl, cycloalkyl, heteroalkyl are optionally substituted with one or more groups independently selected from the group consisting of Z;
Z is independently selected from the group consisting of: halogen, amino, alkyl (or C1-C6 alkyl), alkenyl (or C2-C6 alkenyl), alkynyl (or C2-C6 alkynyl), haloalkyl (or halogenated C1-C6 alkyl), cycloalkyl (or C3-C7 cycloalkyl), aryl (or C6-C12 aryl), and heterocyclyl;
or, Li is selected from the group consisting of: absence and -CR'R", wherein R' and R" are each independently H, C1-C6 alkyl (or C1-C3 alkyl);
or, Li is selected from the group consisting of: absence, -CH₂-, -CH(CH₃)-, and -C-(CH₃)₂;
for A, wherein:
R₁ and R₂ are each independently selected from the group consisting of: C1-C6 alkyl, -O-, -O-R^{a1}, and -NR^{a2}R^{a3};
R₃ and R₄ are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, -C(R^{h})₂-R^{d1}, -(CO)-(L)ₙ-R^{d2}, -(CO)O-(L)ₙ-R^{d3}, -(SO₂)-(L)ₙR^{d4}, and -P(O)(OR^{e})₂, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), and heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-O-(CO)-O-R^{b4}, -(C1-C6 alkyl)ₙ-(CO)-NR^{b5}R^{b6}, -(C1-C6 alkyl)ₙ-NR^{b7}-(CO)-R^{b8}, and -(C1-C6 alkyl)ₙ-NR^{b9}-(CO)-O-R^{b10}, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
R^{b1}, R^{b2}, R^{b3}, R^{b4}, R^{b5}, R^{b6}, R^{b7}, R^{b8}, R^{b9}, R^{b10} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-O-R^{c1}, -(C1-C6 alkyl)ₙ-O-(CO)-R^{c2}, and -(C1-C6 alkyl)ₙ-O-(CO)-O-R^{c3}, the alkyl, cycloalkyl, aryl, heterocyclyl are optionally substituted with one or more groups independently selected from the group consisting of halogen, amino, hydroxyl, carboxyl, C1-C6 alkyl, and C6-C10 aryl;
R^{c1}, R^{c2}, and R^{c3} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, and 3- to 15-membered heterocyclyl;
L is each independently selected from the group consisting of: absence, O, NR^{d5}, C1-C6 alkyl, C2-C6 alkenyl, and C2-C6 alkynyl;
R^{e} is each independently selected from the group consisting of hydrogen, deuterium, Na, K, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, and halogenated C1-C6 alkyl;
R^{h} is each independently selected from the group consisting of: C1-C6 alkyl, -NHC(O)R^{g1}, -OC(O)R^{g2}, -OP(O)(ONa)₂, -NHC(O)OR^{d6}, -OC(O)OR^{d7}, and -OC(O)NHR^{d8};
R^{d1}, R^{d2}, R^{d3}, R^{d4}, R^{d5}, R^{d6}, R^{d7}, R^{d8} are each independently selected from the group consisting of: hydrogen, deuterium, amino, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, heterocyclyl are optionally substituted with one or more R^{f} groups;
R^{f} is each independently selected from the group consisting of halogen, C1-C6 alkyl, halogenated C1-C6 alkyl, C2-C6 alkenyl, C2-C6 alkynyl, C6-C10 aryl, halogenated C6-C10 aryl, C3-C7 cycloalkyl, 3- to 15-membered heterocyclyl, -NR^{g3}R^{g4}, -OR^{g5}, -NHC(O)R^{g6}, and -OC(O)R^{g7};
R^{g1}, R^{g2}, R^{g3}, R^{g4}, R^{g5}, R^{g6}, and R^{g7} are each independently selected from the group consisting of: hydrogen, C1-C6 alkyl, C2-C6 alkenyl, C2-C6 alkynyl, C3-C7 cycloalkyl, C6-C10 aryl, and 3-to 15-membered heterocyclyl;
R₅ is selected from the group consisting of: hydrogen, deuterium, C1-C6 alkyl, halogen, and amino; or, R₅ is selected from the group consisting of hydrogen and amino;
Y is C or S;
y is an integer of 0 or above; or y is selected from the group consisting of 0, 1, 2 and 3; or y is 0 or 1;
n is 0 or 1;
for A', wherein:
R₁' and R₂' are each independently selected from the group consisting of: C1-C6 alkyl, -O-, -O-R^{a1}, and -NR^{a2}R^{a3};
R₃' and R₄' are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, 3- to 15-membered heterocyclyl, -C(R^{h})₂-R^{d1}, -(CO)-(L)ₙ-R^{d2}, -(CO)O-(L)ₙ-R^{d3}, -(SO₂)-(L)ₙ-R^{d4}, and -P(O)(OR^{e})₂, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), and heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-O-(CO)-O-R^{b4}, -(C1-C6 alkyl)ₙ-(CO)-NR^{b5}R^{b6}, -(C1-C6 alkyl)ₙ-NR^{b7}-(CO)-R^{b8}, and -(C1-C6 alkyl)ₙ-NR^{b9}-(CO)-O-R^{b10}, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
R^{b1}, R^{b2}, R^{b3}, R^{b4}, R^{b5}, R^{b6}, R^{b7}, R^{b8}, R^{b9}, R^{b10} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-O-R^{c1}, -(C1-C6 alkyl)ₙ-O-(CO)-R^{c2}, and -(C1-C6 alkyl)ₙ-O-(CO)-O-R^{c3}, the alkyl, cycloalkyl, aryl, heterocyclyl are optionally substituted with one or more groups independently selected from the group consisting of halogen, amino, hydroxyl, carboxyl, C1-C6 alkyl, and C6-C10 aryl;
R^{c1}, R^{c2}, and R^{c3} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, and 3- to 15-membered heterocyclyl;
L is each independently selected from the group consisting of: absence, O, NR^{d5}, C1-C6 alkyl, C2-C6 alkenyl, and C2-C6 alkynyl;
R^{e} is each independently selected from the group consisting of hydrogen, deuterium, Na, K, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, and heterocyclyl are optionally substituted with one or more groups independently selected from the group consisting of halogen, C1-C6 alkyl, and halogenated C1-C6 alkyl;
R^{h} is each independently selected from the group consisting of: C1-C6 alkyl, -NHC(O)R^{g1}, -OC(O)R^{g2}, -OP(O)(ONa)₂, -NHC(O)OR^{d6}, -OC(O)OR^{d7}, and -OC(O)NHR^{d8};
R^{d1}, R^{d2}, R^{d3}, R^{d4}, R^{d5}, R^{d6}, R^{d7}, R^{d8} are each independently selected from the group consisting of: hydrogen, deuterium, amino, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, heterocyclyl are optionally substituted with one or more R^{f} groups;
R^{f} is each independently selected from the group consisting of halogen, C1-C6 alkyl, halogenated C1-C6 alkyl, C2-C6 alkenyl, C2-C6 alkynyl, C6-C10 aryl, halogenated C6-C10 aryl, C3-C7 cycloalkyl, 3- to 15-membered heterocyclyl, -NR^{g3}R^{g4}, -OR^{g5}, -NHC(O)R^{g6}, and -OC(O)R^{g7};
R^{g1}, R^{g2}, R^{g3}, R^{g4}, R^{g5}, R^{g6}, and R^{g7} are each independently selected from the group consisting of: hydrogen, C1-C6 alkyl, C2-C6 alkenyl, C2-C6 alkynyl, C3-C7 cycloalkyl, C6-C10 aryl, and 3-to 15-membered heterocyclyl;
R₅' is selected from the group consisting of: hydrogen, deuterium, C1-C6 alkyl, halogen, and amino; or, R₅ is selected from the group consisting of hydrogen and amino;
Y' is C or S;
y' is an integer of 0 and above; or y' is selected from the group consisting of 0, 1, 2, and 3; or y' is 0 or 1;
n is 0 or 1;
or, Formula I is:

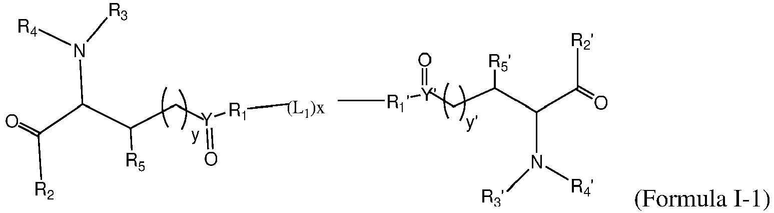

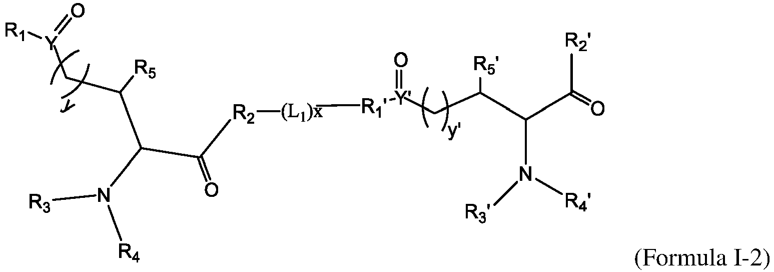

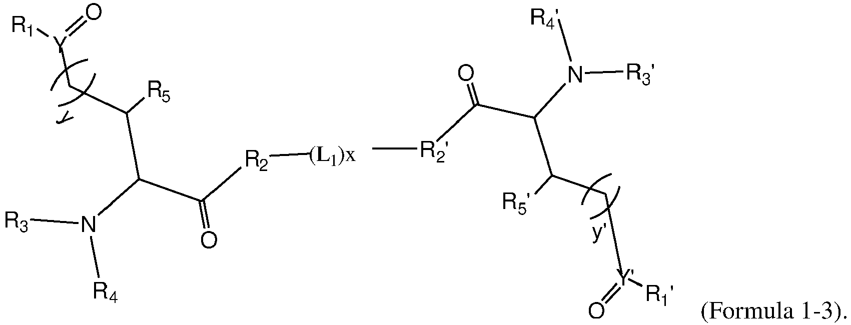

2. The use according to claim 1, wherein,
1) when x is 0, A' is absent; for A, wherein:
R₁ and R₂ are each independently selected from the group consisting of:
C1-C6 alkyl,
-O-R^{a1}, wherein R^{a1} is selected from the group consisting of: hydrogen, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, and -(C1-C6 alkyl)ₙ-O-(CO)-O-R^{b4}, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl); and
-NR^{a2}R^{a3}, wherein R^{a2} and R^{a3} are each independently selected from the group consisting of: hydrogen, C1-C6 alkyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-O-(CO)-O-R^{b4}, and -(C1-C6 alkyl)ₙ-(CO)-NR^{b5}R^{b6}, the alkyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
R^{b1}, R^{b2}, R^{b3}, R^{b4}, R^{b5} and R^{b6} are each independently selected from the group consisting of: hydrogen, hydroxyl, amino, C1-C6 alkyl, C3-C7 cycloalkyl, C6-C10 aryl, 3- to 15-membered heterocyclyl, -(C1-C6 alkyl)ₙ-(CO)-O-R^{c1}, -(C1-C6 alkyl)ₙ-O-(CO)-R^{c2}, and -(C1-C6 alkyl)ₙ-O-(CO)-O-R^{c3}, the alkyl, cycloalkyl, aryl, heterocyclyl are optionally substituted with one or more groups independently selected from the group consisting of halogen, amino, hydroxyl, carboxyl, C1-C6 alkyl, and C6-C10 aryl;
R^{c1}, R^{c2} and R^{c3} are each independently selected from the group consisting of: hydrogen, hydroxyl, amino, C1-C6 alkyl, and C3-C7 cycloalkyl;
or, R₁ and R₂ are each independently selected from the group consisting of:
hydroxyl,
C1-C6 alkyl,
-O-R^{a1}, wherein, R^{a1} is selected from the group consisting of: C1-C6 alkyl, C3-C7 cycloalkyl, C2-C6 alkenyl, and -(C1-C6 alkyl)ₙ-O-(CO)-O-R^{b4}, which are optionally further substituted with one or more C6-C10 aryl groups, wherein R^{b4} is selected from the group consisting of C1-C6 alkyl, n is 1; and
-NR^{a2}R^{a3}, wherein R^{a2} and R^{a3} are each independently selected from the group consisting of:
hydrogen,
C1-C6 alkyl, wherein C1-C6 alkyl is optionally further substituted with one or more C6-C10 aryl groups;
-(C1-C6 alkyl)ₙ-O-(CO)-R^{b3}, wherein R^{b3} is C1-C6 alkyl, which is further substituted with C6-C10 aryl;
-(C1-C6 alkyl)ₙ-(CO)-R^{b1}, wherein R^{b1} is hydroxyl, n or 1;
-(C1-C6 alkyl)ₙ-(CO)-NR^{b5}R^{b6}, wherein n is 1, R^{b5} and R^{b6} are each independently hydrogen, or a 3- to 15-membered heterocyclyl, and the heterocyclic ring is further substituted with one or more C1-C6 alkyl groups; and
-(C1-C6 alkyl)ₙ-(CO)-O-R^{b2}, wherein the alkyl is substituted with one or more C6-C10 aryl groups, n is 1, R^{b2} is C1-C6 alkyl or -(C1-C6 alkyl)ₙ-O-(CO)-O-R^{c}, R^{c} is C1-C6 alkyl;
R₃ and R₄ are each independently selected from the group consisting of: hydrogen, hydroxyl, C1-C6 alkyl, -(CO)-(L)ₙ-R^{d2}, and -(CO)O-(L)ₙ-R^{d3}, the alkyl, aryl and heterocyclyl are optionally substituted with one or more groups independently selected from the group consisting of halogen, C1-C6 alkyl, halogenated C1-C6 alkyl, aryl (or C6-C12 aryl), and heterocyclyl;
L is each independently selected from the group consisting of: absence, O, NR^{d5}, and C1-C6 alkyl;
R^{d5}, R^{d2} and R^{d3} are each independently selected from the group consisting of: hydrogen, amino, C1-C6 alkyl, C3-C7 cycloalkyl, C6-C10 aryl, and 3- to 15-membered heterocyclyl, the alkyl, cycloalkyl, aryl, heterocyclyl are optionally substituted with one or more R^{f} groups;
R^{f} is each independently selected from the group consisting of amino, halogen, C1-C6 alkyl, halogenated C1-C6 alkyl, C6-C10 aryl, halogenated C6-C10 aryl, C3-C7 cycloalkyl, and 3- to 15-membered heterocyclyl;
or, R₃ and R₄ are each independently selected from the group consisting of:
hydrogen,
hydroxyl,
C1-C6 alkyl,
-(CO)-(L)ₙ-R^{d2}: wherein, the combination option of (L)ₙ and R^{d2} is selected from the group consisting of the following combinations:
1) L is absent, R^{d2} is C1-C6 alkyl that is further substituted with one or more R^{f} groups, R^{f} is amino;
2) L is absent, R^{d2} is H;
3) L is absent, R^{d2} is C1-C6 alkyl;
4) L is absent, R^{d2} is C1-C6 alkyl that is further substituted with one or more R^{f} groups, R^{f} is selected from the group consisting of C1-C6 alkyl and amino;
5) L is absent, R^{d2} is 3- to 15-membered heterocyclyl; and
6) L is NR^{d5}, R^{d5} is C1-C6 alkyl, n is 1, R^{d2} is H; and
-(CO)O-(L)ₙ-R^{d3}: wherein, the combination option of (L)ₙ and R^{d3} is selected from the group consisting of: L is absent, R^{d3} is C1-C6 alkyl, which is optionally further substituted with one or more R^{f} groups, and R^{f} is selected from the group consisting of C1-C6 alkyl, C6-C10 aryl, and polycyclic aromatic hydrocarbonyl (e.g., fluorenyl);
2) when x is 1 or 2, A and A' are the same or different, wherein
for A, wherein:
R₁ and R₂ are each independently selected from the group consisting of: C1-C6 alkyl, -O-, -O-R^{a1}, and -NR^{a2}R^{a3};
R₃ and R₄ are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), and heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, and 3-to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
R₅ is selected from the group consisting of: hydrogen, deuterium, C1-C6 alkyl, halogen, and amino; or, R₅ is selected from the group consisting of hydrogen and amino; or, R₅ is selected from the group consisting of hydrogen;
Y is C;
y is selected from the group consisting of 0, 1, 2, and 3; or y is 0 or 1;
for A', wherein:
R₁' and R₂' are each independently selected from the group consisting of: C1-C6 alkyl, -O-, -O-R^{a1}, and -NR^{a2}R^{a3};
R₃' and R₄' are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogen, C1-C6 alkyl, aryl (or C6-C12 aryl) and heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, and 3-to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
R₅' is selected from the group consisting of: hydrogen, deuterium, C1-C6 alkyl, halogen, and amino; or, R₅ is selected from the group consisting of hydrogen and amino; or, R₅ is selected from the group consisting of hydrogen;
Y' is C;
y' is selected from the group consisting of 0, 1, 2, and 3; or y is 0 or 1.

3. The use according to claim 1 or 2, wherein,
1) when x is 0, A' is absent; for A, wherein:
R₁ is selected from the group consisting of:
C1-C6 alkyl,
-O-R^{a1}, wherein, R^{a1} is selected from the group consisting of: hydrogen, C1-C6 alkyl, C2-C6 alkenyl, C3-C7 cycloalkyl, C6-C10 aryl, 3- to 15-membered heterocyclyl, and -(C1-C6 alkyl)ₙ-O-(CO)-R^{b3}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl); and
-NR^{a2}R^{a3}, wherein, R^{a2} and R^{a3} are each independently selected from the group consisting of: hydrogen, C1-C6 alkyl, C3-C7 cycloalkyl, C6-C10 aryl, 3- to 15-membered heterocyclyl, -(C1-C6 alkyl)ₙ-(CO)-R^{b1}, and -(C1-C6 alkyl)ₙ-O-(CO)-R^{b3}, the alkyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl) -(3- to 15-membered heterocyclyl);
R^{b1} and R^{b3} are each independently selected from the group consisting of: hydrogen, hydroxyl, amino, C1-C6 alkyl, and C6-C10 aryl, the alkyl, aryl, heterocyclyl are optionally substituted with one or more groups independently selected from the group consisting of halogen, amino, hydroxyl, carboxyl, C1-C6 alkyl, and C6-C10 aryl;
or, R₁ is selected from the group consisting of:
hydroxyl,
C1-C6 alkyl,
-O-R^{a}, wherein R^{a1} is selected from the group consisting of: C1-C6 alkyl, C3-C7 cycloalkyl, C2-C6 alkenyl, and -(C1-C6 alkyl)ₙ-O-(CO)-O-R^{b4}, which are optionally further substituted with one or more C6-C10 aryl groups, wherein R^{b4} is selected from the group consisting of C1-C6 alkyl, n is 1; and
-NR^{a2}R^{a3}, wherein R^{a2} and R^{a3} are each independently selected from the group consisting of: hydrogen; C1-C6 alkyl, which is optionally further substituted with one or more C6-C10 aryl groups; -(C1-C6 alkyl)ₙ-O-(CO)-R^{b3}, R^{b3} is C1-C6 alkyl, which is further substituted with C6-C10 aryl; and -(C1-C6 alkyl)ₙ-(CO)-R^{b1}, R^{b1} is hydroxyl, n is 1;
or, R₁ is selected from the group consisting of: hydroxyl, methoxy, -OCH₂CH=CH₂, ethoxy, -OC(CH₃)₃, -OCH₂C₆H₅, -OC₆H₁₁, Trt-NH-, 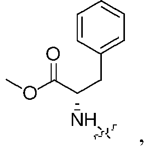 CH₃-NH-, HOCOCH₂NH-, -OCH(CH₃)₂, -CH₃, and 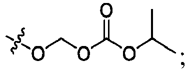 wherein, Trt represents trityl;
R₂ is selected from the group consisting of:
C1-C6 alkyl,
-O-R^{a1}, wherein, R^{a1} is selected from the group consisting of: hydrogen, C1-C6 alkyl, C2-C6 alkenyl, C6-C10 aryl, and -(C1-C6 alkyl)ₙ-O-(CO)-O-R^{b4}, and the alkyl, alkenyl, aryl are optionally substituted with one or more groups independently selected from the group consisting of halogen, amino, hydroxyl, C1-C6 alkyl, C6-C10 aryl, and -(C1-C6 alkyl)-(C6-C10 aryl); and
-NR^{a2}R^{a3}, wherein, R^{a2} and R^{a3} are each independently selected from the group consisting of: hydrogen, C1-C6 alkyl, -(C1-C6 alkyl)ₙ-(CO)-R^{b1}, -(C1-C6 alkyl)ₙ-(CO)-O-R^{b2}, -(C1-C6 alkyl)ₙ-(CO)-NR^{b5}R^{b6}, and 3- to 15-membered heterocyclyl, the alkyl and heterocyclyl are optionally substituted with one or more groups independently selected from the group consisting of halogen, amino, hydroxyl, and C1-C6 alkyl;
R^{b1}, R^{b2}, R^{b4}, R^{b5} and R^{b6} are each independently selected from the group consisting of: hydrogen, hydroxyl, C1-C6 alkyl, C3-C7 cycloalkyl, and -(C1-C6 alkyl)ₙ-O-(CO)-O-R^{c3}, the cycloalkyl is optionally substituted with one or more groups independently selected from the group consisting of halogen, amino, hydroxyl, carboxyl, C1-C6 alkyl, and C6-C10 aryl;
R^{c3} is each independently selected from the group consisting of: hydrogen, hydroxyl, amino, C1-C6 alkyl, and C3-C7 cycloalkyl;
or, R₂ is selected from the group consisting of:
hydroxyl,
-O-R^{a1}: wherein, R^{a1} is selected from the group consisting of: C1-C6 alkyl, C2-C6 alkenyl, and -(C1-C6 alkyl)ₙ-O-(CO)-O-R^{b4}, which are optionally further substituted with one or more C6-C10 aryl groups, wherein R^{b4} is selected from the group consisting of C1-C6 alkyl, n is 1; and
-NR^{a2}R^{a3}: R^{a2} and R^{a3} are each independently selected from the group consisting of: hydrogen; -(C1-C6 alkyl)ₙ-(CO)-NR^{b5}R^{b6}, n is 1, R^{b5} and R^{b6} are each independently hydrogen, or 3- to 15-membered heterocyclyl, the heterocyclyl is further substituted with one or more C1-C6 alkyl groups; -(C1-C6 alkyl)ₙ-(CO)-R^{b1}, n is 1, R^{b1} is hydroxyl; and -(C1-C6 alkyl)ₙ-(CO)-O-R^{b2}, the alkyl is substituted with one or more C6-C10 aryl groups, n is 1, R^{b2} is C1-C6 alkyl or -(C1-C6 alkyl)ₙ-O-(CO) -O-R^{c3}, R^{c3} is C1-C6 alkyl;
or, R₂ is selected from the group consisting of: hydroxyl, methoxy, -OCH₂CH=CH₂, ethoxy, -OC(CH₃)₃, -OCH₂C₆H₅, 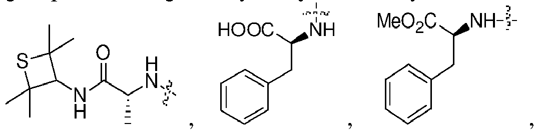 -OCH(CH₃)₂, 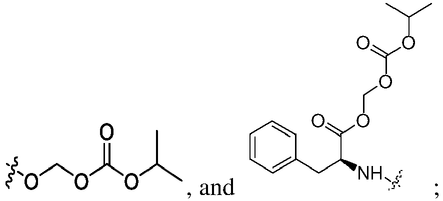
R₃ and R₄ are each independently selected from the group consisting of: hydrogen, hydroxyl, Boc, Cbz, -CH₃, Fmoc, -COOCH₂C₆H₅, -COCH₂NH₂, -CHO, -COCH₃, 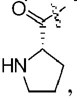 , -COCH₂CH₃, -COCH(CH₃)₂, -COCH(CH₂)₂, -COOCH₃, -CON(CH₃)₂, and -COCH(CH₃)(NH₂); wherein, Boc represents tert-butoxycarbonyl, Cbz represents benzyloxycarbonyl, and Fmoc represents fluorenylmethoxycarbonyl;
2) when x is 1, A and A' are the same or different, wherein
any one of R₁, R₂, R₃, and R₄ of a unit A is connected to any one of R₁', R₂', R₃', and R₄' of an adjacent unit A' through Li;
Li is each independently a unit-linking group selected from the group consisting of: absence, O, S, carbonyl, alkyl (or C1-C6 alkyl), alkenyl (or C2-C6 alkenyl), alkynyl (or C2-C6 alkynyl), -O-alkenyl-O- (or -O-C2-C6 alkenyl-O-), -O-alkynyl-O- (or -O-C2-C6 alkynyl-O-), cycloalkyl (or C3-C7 cycloalkyl), and heteroalkyl (or 3- to 7-membered heteroalkyl; for example, -O-alkyl-O- (or -O-C1-C6 alkyl-O-)), wherein the alkyl, cycloalkyl, heteroalkyl are optionally substituted with one or more groups independently selected from the group consisting of Z;
Z is independently selected from the group consisting of: halogen, amino, alkyl (or C1-C6 alkyl), alkenyl (or C2-C6 alkenyl), alkynyl (or C2-C6 alkynyl), haloalkyl (or halogenated C1-C6 alkyl), cycloalkyl (or C3-C7 cycloalkyl), aryl (or C6-C12 aryl), and heterocyclyl;
or, Li is selected from the group consisting of: absence and -CR'R", wherein R' and R" are each independently H, C1-C6 alkyl (or C1-C3 alkyl);
or, Li is selected from the group consisting of: absence, -CH₂-, -CH(CH₃)-, and -C-(CH₃)₂;
for A, wherein:
R₁ is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, and 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁ is selected from the group consisting of: -O-, hydroxyl, and -O-R^{a}: R^{a} is C1-C6 alkyl;
or, R₁ is selected from the group consisting of: -O-, hydroxyl, methoxy, ethoxy, and -OCH(CH₃)₂;
Y is C;
y is 0;
R₂ is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, and 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₂ is selected from the group consisting of: hydroxyl, -O-, and -O-R^{a}: R^{a} is C1-C6 alkyl;
or, R₂ is selected from the group consisting of: hydroxyl, -O-, and methoxy;
R₃ and R₄ are each independently selected from the group consisting of: hydrogen, hydroxyl, and C1-C6 alkyl;
or, R₃ and R₄ are each independently hydrogen;
R₅ is selected from the group consisting of: hydrogen, deuterium, C1-C6 alkyl, halogen, and amino; or, R₅ is selected from the group consisting of hydrogen and amino; or, R₅ is selected from the group consisting of hydrogen;
for A', wherein:
R₁' is selected from the group consisting of: -O-, C1-C6 alkyl, and -O-R^{a1}, wherein, R^{a1} is selected from the group consisting of: hydrogen, C1-C6 alkyl, C2-C6 alkenyl, C3-C7 cycloalkyl, C6-C10 aryl, and 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁ is selected from the group consisting of: -O-, hydroxyl, and -O-R^{a}: R^{a} is C1-C6 alkyl;
or, R₁' is selected from the group consisting of: -O-, hydroxyl, methoxy, ethoxy, and -OCH(CH₃)₂;
Y' is C;
y' is 0;
R₂' is selected from the group consisting of: -O-, C1-C6 alkyl, and -O-R^{a1}, wherein, R^{a1} is selected from the group consisting of: hydrogen, C1-C6 alkyl, C2-C6 alkenyl, C3-C7 cycloalkyl, C6-C10 aryl, and 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₂' is selected from the group consisting of: hydroxyl, -O-, and -O-R^{a}: R^{a} is C1-C6 alkyl;
or, R₂' is selected from the group consisting of: hydroxyl, -O-, and methoxy;
R₃' and R₄' are each independently selected from the group consisting of: hydrogen, hydroxyl, and C1-C6 alkyl;
or, R₃' and R₄' are each independently hydrogen;
R₅' is selected from the group consisting of: hydrogen, deuterium, C1-C6 alkyl, halogen, and amino;
or, R₅' is selected from the group consisting of hydrogen and amino;
or, R₅' is selected from the group consisting of hydrogen;
3) when x is 2, A and A' are the same or different, wherein
any one of R₁, R₂, R₃, and R₄ of a unit A is connected to any one of R₁', R₂', R₃', and R₄' of an adjacent unit A' through Li;
each Li is independently a unit-linking group selected from the group consisting of: absence, O, S, carbonyl, alkyl (or C1-C6 alkyl), alkenyl (or C2-C6 alkenyl), alkynyl (or C2-C6 alkynyl), -O-alkenyl-O- (or -O-C2-C6 alkenyl-O-), -O-alkynyl-O- (or -O-C2-C6 alkynyl-O-), cycloalkyl (or C3-C7 cycloalkyl), and heteroalkyl (or 3- to 7-membered heteroalkyl; for example, -O-alkyl-O- (or -O-C1-C6 alkyl-O-)), wherein the alkyl, cycloalkyl, heteroalkyl are optionally substituted with one or more groups independently selected from the group consisting of Z;
Z is independently selected from the group consisting of: halogen, amino, alkyl (or C1-C6 alkyl), alkenyl (or C2-C6 alkenyl), alkynyl (or C2-C6 alkynyl), haloalkyl (or halogenated C1-C6 alkyl), cycloalkyl (or C3-C7 cycloalkyl), aryl (or C6-C12 aryl), and heterocyclyl;
or, Li is selected from the group consisting of: absence and -CR'R", wherein R' and R" are each independently H, C1-C6 alkyl (or C1-C3 alkyl);
or, Li is selected from the group consisting of: absence, -CH₂-, -CH(CH₃)-, and -C-(CH₃)₂;
or, Li is selected from the group consisting of: -CH₂-;
for A, wherein:
R₁ is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, and 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁ is selected from the group consisting of: -O-, hydroxyl, and -O-R^{a}: R^{a} is C1-C6 alkyl;
or, R₁ is selected from the group consisting of: -O-, hydroxyl, methoxy, ethoxy, and -OCH(CH₃)₂;
or, R₁ is selected from the group consisting of: methoxy;
Y is C;
y is 0;
R₂ is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, and 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₂ is selected from the group consisting of: hydroxyl, -O-, and -O-R^{a}: R^{a} is C1-C6 alkyl;
or, R₂ is selected from the group consisting of: hydroxyl, -O-, and methoxy;
or, R₂ is selected from the group consisting of: -O-;
R₃ and R₄ are each independently selected from the group consisting of: hydrogen, hydroxyl, and C1-C6 alkyl;
or, R₃ and R₄ are each independently hydrogen;
R₅ is selected from the group consisting of: hydrogen, deuterium, C1-C6 alkyl, halogen, and amino; or, R₅ is selected from the group consisting of hydrogen and amino; or, R₅ is selected from the group consisting of hydrogen;
for A', wherein:
R₁' is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, and 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁ is selected from the group consisting of: -O-, hydroxyl, and -O-R^{a}: R^{a} is C1-C6 alkyl;
or, R₁' is selected from the group consisting of: -O-, hydroxyl, methoxy, ethoxy, and -OCH(CH₃)₂;
or, R₁' is selected from the group consisting of: methoxy;
Y' is C;
y' is 0;
R₂' is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, and 3- to 15-membered heterocyclyl, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₂' is selected from the group consisting of: hydroxyl, -O-, and -O-R^{a}: R^{a} is C1-C6 alkyl;
or, R₂' is selected from the group consisting of: hydroxyl, -O-, and methoxy;
more furthermore, R₂' is selected from the group consisting of: -O-;
R₃' and R₄' are each independently selected from the group consisting of: hydrogen, hydroxyl, and C1-C6 alkyl;
or, R₃' and R₄' are each independently hydrogen;
R₅' is selected from the group consisting of: hydrogen, deuterium, C1-C6 alkyl, halogen, and amino;
or, R₅' is selected from the group consisting of hydrogen and amino;
or, R₅' is selected from the group consisting of hydrogen.

4. Use of a compound represented by the following Formula I-4, or pharmaceutically acceptable salt, ester, prodrug, stereoisomer, hydrate, solvate, isotopic compound, crystalline form, metabolite form thereof, or any combination or mixture thereof, in the manufacture of a medicament or agent, wherein the medicament is used for at least one of the following:
1) treating, improving, or preventing a liver disease (e.g., NAFLD, NASH, liver fibrosis);
2) alleviating a mammalian liver fibrosis, liver steatosis, or inflammation;
3) inhibiting the activation of a stellate cell;
4) regulating the expression of NS3TP1; 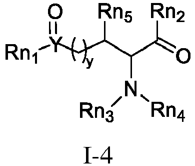
wherein:
Rn₁ and Rn₂ are each independently selected from the group consisting of: C1-C6 alkyl, -O-R^{a4}, -O-L₂-O-R^{a4}, and -NR^{a5}R^{a6};
each R^{a4} is independently selected from the group consisting of: hydrogen, deuterium, C1-C6 alkyl, C2-C6 alkenyl, C2-C6 alkynyl, C3-C7 cycloalkyl, C3-C7 cycloalkyl-C1-C6 alkyl, C6-C10 aryl, C6-C10 aryl-C1-C6 alkyl, 3- to 15-membered heterocyclyl, 3- to 15-membered heterocyclyl-C1-C6 alkyl, C1-C6 alkoxyacyl, -(O=)C-CH₂-CH(NH₂)-COORₘ, -(O=)C-CH(NH₂)-CH₂-COORₘ, Ph-CH2-CH(NH2)-C(=O)-, NH₂-CH₂-C(=O)-, CH3-CH(NH2)-C(=O)-, RₘOOC-(CH₂)₂-CH(NH₂)-C(=O)-, -C(=O)-(CH₂)₂-CH(NH₂)-COORₘ, RₘOOC-CH(NH₂)-CH₂-S-S-CH₂-CH(NH₂)-C(=O)-, H₂N-CO-(CH₂)₂-CH(NH₂)-C(=O)-, *N=CH-NH-CH=*C-CH₂-CH(NH₂)-C(=O)- (or 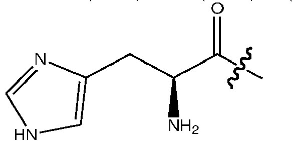 ), HO-p-Ph-CH₂-CH(NH₂)-C(=O)-, HO-CH₂-CH(NH₂)-C(=O)-, CH₃-S-(CH₂)₂-CH(NH₂)-C(=O)-, HN=C(NH₂)-NH-(CH₂)₃-CH(NH₂)-C(=O)-, and (CH₃)₂CH-CH₂-CH(NH₂)-C(=O)-; wherein Ph represents phenyl, HO-p-Ph-CH₂-CH(NH₂)-C(=O)- represents p-hydroxybenzylaminomethylcarbonyl;
R^{a5} and R^{a6} are each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, 3- to 15-membered heterocyclylaminoacyl-C1-C6 alkyl, Ph-CH₂-CH(COORₘ)-, -CH₂-COORₘ, -CH(COORₘ)-CH₂-COORₘ, -CH(CH₃)-COORₘ, RₘOOC-(CH₂)₂-CH(COORₘ)-, -CH(COORₘ)-CH₂-S-S-CH₂-CH(NH₂)-COORₘ, H₂N-CO-(CH₂)₂-CH(COORₘ)-, *N=CH-NH-CH=*C-CH₂-CH(COORₘ)- (or 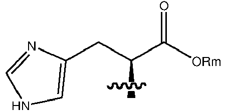 ), HO-p-Ph-CH₂-CH(COORₘ)-, HO-CH₂-CH(COORₘ)-, CH₃-S-(CH₂)₂-CH(COORₘ)-, HN=C(NH₂)-NH-(CH₂)₃-CH(COORₘ)-, and (CH₃)₂CH-CH₂-CH(COORₘ)-;
each Rₘ is independently selected from the group consisting of: hydrogen, C1-C6 alkyl, and C1-C6 alkoxyacyl-O-L₃-;
L₂ and L₃ are each independently selected from the group consisting of: C1-C6 alkyl, C2-C6 alkenyl, C2-C6 alkynyl, C3-C7 cycloalkyl, and 3- to 7-membered heteroalkyl, L₂ and L₃ are optionally substituted with one or more groups independently selected from the group consisting of halogen, amino, and hydroxyl;
Rn₃ and Rn₄ are each independently selected from the group consisting of: hydrogen, deuterium, hydroxyl, H(C=O)-, C1-C6 alkyl, C2-C6 alkenyl, C2-C6 alkynyl, C3-C7 cycloalkyl, C6-C10 aryl, 3- to 15-membered heterocyclyl, aminoacyl, C1-C6 alkylacyl, C1-C6 alkoxyacyl, C1-C6 alkylaminoacyl, amino-C1-C6 alkanoyl, 3- to 15-membered heterocyclylacyl, C3-C7 cycloalkylacyl, and C6-C10 aryl-C1-C6 alkoxyacyl;
Y is C or S;
y is 0, 1, 2 or 3;
or
Rn₁ and Rn₂ are each independently selected from the group consisting of: C1-C6 alkyl, -O-R^{a4}, -O-L₂-O-R^{a4}, and -NR^{a5}R^{a6};
each R^{a4} is independently selected from the group consisting of: hydrogen, C1-C6 alkyl, C2-C6 alkenyl, C3-C7 cycloalkyl, C3-C7 cycloalkyl-C1-C6 alkyl, C6-C10 aryl, C6-C10 aryl-C1-C6 alkyl, 3- to 15-membered heterocyclyl, 3- to 15-membered heterocyclyl-C1-C6 alkyl, C1-C6 alkoxyacyl, -(O=)C-CH₂-CH(NH₂)-COORₘ, Ph-CH₂-CH(NH₂)-C(=O)-, NH₂-CH₂-C(=O)-, CH3-CH(NH2)-C(=O)-, RₘOOC-(CH₂)₂-CH(NH₂)-C(=O)-, RₘOOC-CH(NH₂)-CH₂-S-S-CH₂-CH(NH₂)-C(=O)-, H2N-CO-(CH2)2-CH(NH2)-C(=O)-, *N=CH-NH-CH=*C-CH₂-CH(NH₂)-C(=O)- (or 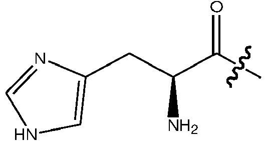 ), HO-p-Ph-CH₂-CH(NH₂)-C(=O)-, HO-CH₂-CH(NH₂)-C(=O)-, CH₃-S-(CH₂)₂-CH(NH₂)-C(=O)-, HN=C(NH₂)-NH-(CH₂)₃-CH(NH₂)-C(=O)-, and (CH₃)₂CH-CH₂-CH(NH₂)-C(=O)-;
R^{a5} and R^{a6} are each independently selected from the group consisting of: hydrogen, C1-C6 alkyl, C2-C6 alkenyl, C3-C7 cycloalkyl, C6-C10 aryl, 3- to 15-membered heterocyclyl, 3- to 15-membered heterocyclylaminoacyl-C1-C6 alkyl, Ph-CH₂-CH(COORₘ)-, -CH₂-COORₘ, -CH(COORₘ)-CH₂-COORₘ, -CH(CH₃)-COORₘ, RₘOOC-(CH₂)₂-CH(COORₘ)-, -CH(COORₘ)-CH₂-S-S-CH₂-CH(NH₂)-COORₘ, H₂N-CO-(CH₂)₂-CH(COORₘ)-, *N=CH-NH-CH=*C-CH₂-CH(COORₘ)- (or 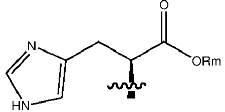 ), HO-p-Ph-CH₂-CH(COORₘ)-, HO-CH₂-CH(COORₘ)-, CH₃-S-(CH₂)₂-CH(COORₘ)-, HN=C(NH₂)-NH-(CH₂)₃-CH(COORₘ)-, and (CH₃)₂CH-CH₂-CH(COORₘ)-;
each Rₘ is independently selected from the group consisting of: hydrogen, C1-C6 alkyl, and C1-C6 alkoxyacyl-O-L₃-;
L₂ and L₃ are each independently selected from the group consisting of: C1-C6 alkyl, C2-C6 alkenyl, C3-C7 cycloalkyl, and 3- to 7-membered heteroalkyl, L₂ and L₃ are each optionally substituted with one or more groups independently selected from the group consisting of halogen, amino, and hydroxyl;
Rn₃ and Rn₄ are each independently selected from the group consisting of: hydrogen, hydroxyl, H(C=O)-, C1-C6 alkyl, C2-C6 alkenyl, C3-C7 cycloalkyl, C6-C10 aryl, 3- to 15-membered heterocyclyl, aminoacyl, C1-C6 alkylacyl, C1-C6 alkoxyacyl, C1-C6 alkylaminoacyl,
amino-C1-C6 alkanoyl, 3- to 15-membered heterocyclylacyl, C3-C7 cycloalkylacyl, and C6-C10 aryl-C1-C6 alkoxyacyl;
Y is C;
y is 0, 1 or 2;
or
Rn₁ and Rn₂ are each independently selected from the group consisting of: C1-C6 alkyl, -O-R^{a4}, -O-L₂-O-R^{a4}, and -NR^{a5}R^{a6};
each R^{a4} is independently selected from the group consisting of: hydrogen, C1-C6 alkyl, C2-C6 alkenyl, C3-C7 cycloalkyl, C6-C10 aryl, C6-C10 aryl-C1-C6 alkyl, 3- 15-membered heterocyclyl, 3- to 15-membered heterocyclyl-C1-C6 alkyl, -(O=)C-CH₂-CH(NH₂)-COORₘ, Ph-CH₂-CH(NH₂)-C(=O)-, NH₂-CH₂-C(=O)-, CH₃-CH(NH₂)-C(=O)-, RₘOOC-(CH₂)₂-CH(NH₂)-C(=O)-, RₘOOC-CH(NH₂)-CH₂-S-S-CH₂-CH(NH₂)-C(=O)-, H₂N-CO-(CH₂)₂-CH(NH₂)-C(=O)-, *N=CH-NH-CH=*C-CH₂-CH(NH₂)-C(=O)- (or 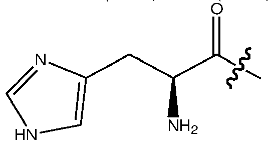 ), HO-p-Ph-CH₂-CH(NH₂)-C(=O)-, HO-CH₂-CH(NH₂)-C(=O)-, CH₃-S-(CH₂)₂-CH(NH₂)-C(=O)-, HN=C(NH₂)-NH-(CH₂)₃-CH(NH₂)-C(=O)-, and (CH₃)₂CH-CH₂-CH(NH₂)-C(=O)-;
R^{a5} and R^{a6} are each independently selected from the group consisting of: hydrogen, C1-C6 alkyl, C3-C7 cycloalkyl, C6-C10 aryl, 3- to 15-membered heterocyclyl, 3- to 15-membered heterocyclylaminoacyl-C1-C6 alkyl, Ph-CH₂-CH(COORₘ)-, -CH₂-COORₘ, -CH(COORₘ)-CH₂-COORₘ, -CH(CH₃)-COORₘ, RₘOOC-(CH₂)₂-CH(COORₘ)-, -CH(COORₘ)-CH₂-S-S-CH₂-CH(NH₂)-COORₘ, H₂N-CO-(CH₂)₂-CH(COORₘ)-, *N=CH-NH-CH=*C-CH₂-CH(COORₘ)- (or 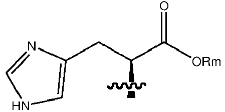 ), HO-p-Ph-CH₂-CH(COORₘ)-, HO-CH₂-CH(COORₘ)-, CH₃-S-(CH₂)₂-CH(COORₘ)-, HN=C(NH₂)-NH-(CH₂)₃-CH(COORₘ)-, and (CH₃)₂CH-CH₂-CH(COORₘ)-;
each Rₘ is independently selected from the group consisting of: hydrogen, C1-C6 alkyl, and C1-C6 alkoxyacyl-O-L₃-;
L₂ and L₃ are each independently selected from the group consisting of: C1-C6 alkyl, L₂ and L₃ are each optionally substituted with one or more groups independently selected from the group consisting of halogen, amino, and hydroxyl;
Rn₃ and Rn₄ are each independently selected from the group consisting of: hydrogen, hydroxyl, C1-C6 alkyl, C2-C6 alkenyl, C3-C7 cycloalkyl, C6-C10 aryl, 3- to 15-membered heterocyclyl, aminoacyl, C1-C6 alkylacyl, C1-C6 alkoxyacyl, C1-C6 alkylaminoacyl, amino-C1-C6 alkanoyl,
3- to 15-membered heterocyclylacyl, C3-C7 cycloalkylacyl, and C6-C10 aryl-C1-C6 alkoxyacyl;
Y is C;
y is 0, 1 or 2.

5. The use according to any one of claims 1 to 4, wherein the compound represented by Formula I or I-1, I-2, I-3, I-4 is selected from the group consisting of the following:

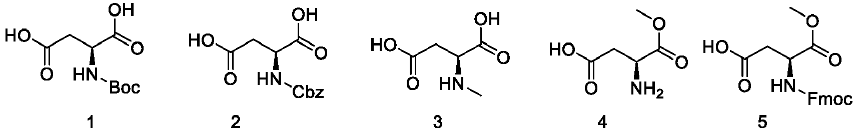

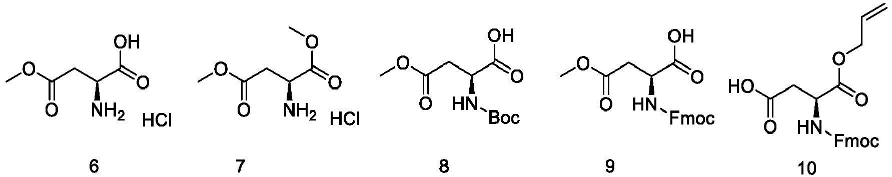

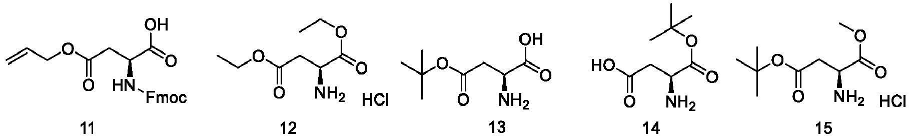

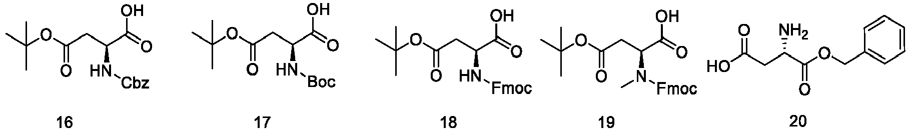

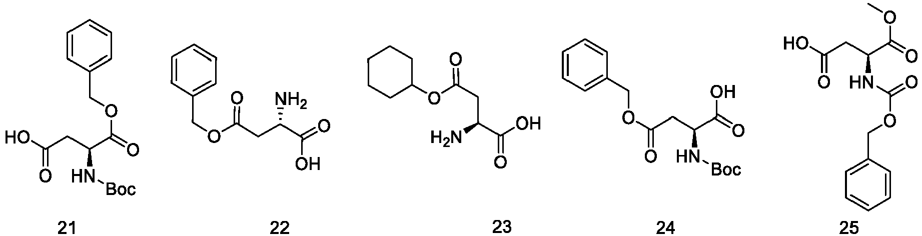

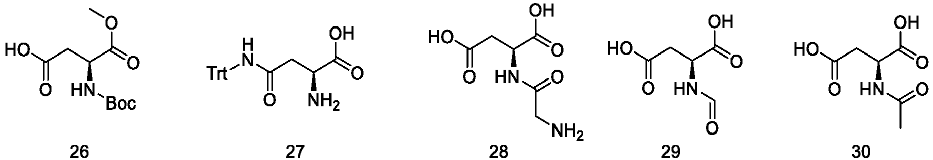

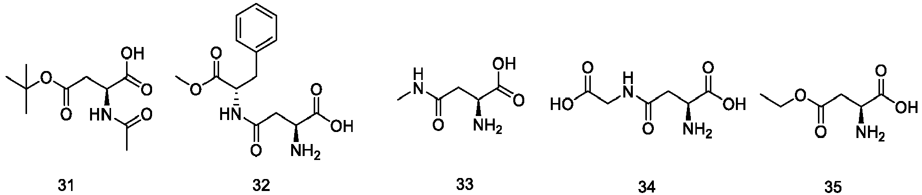

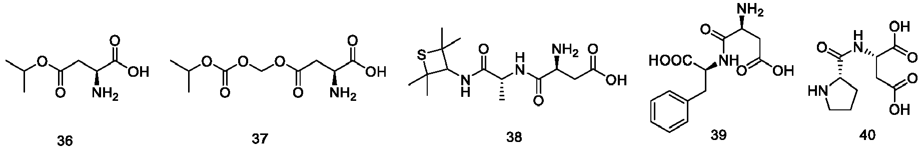

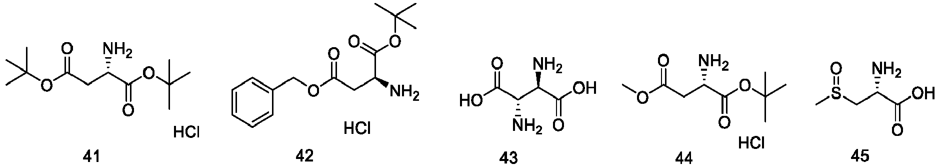

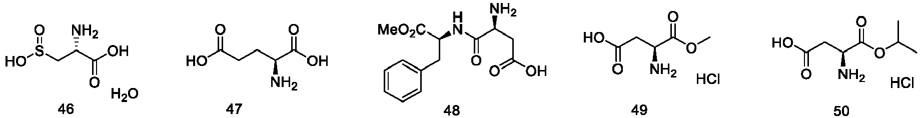

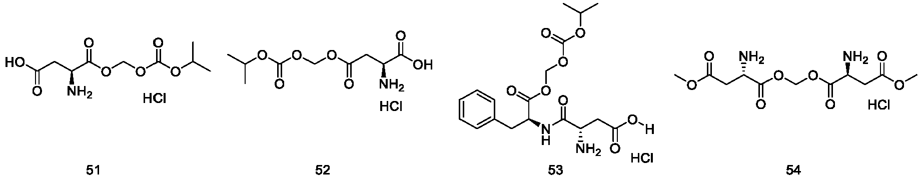

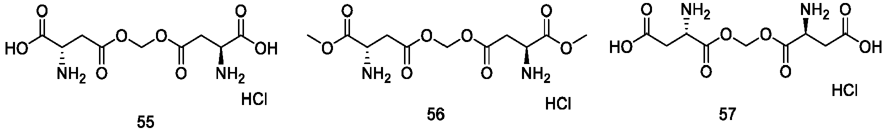

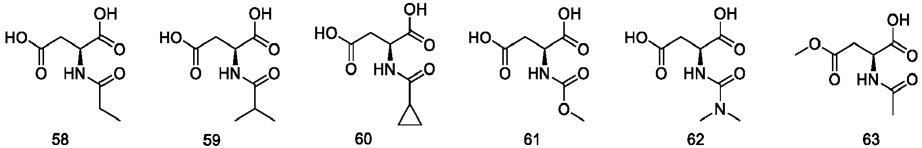

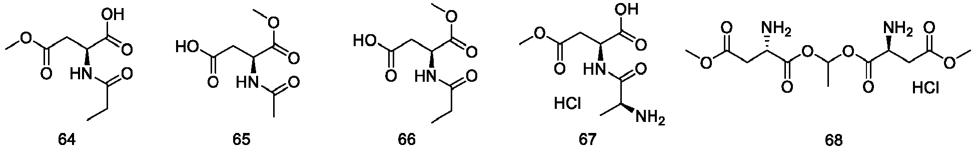

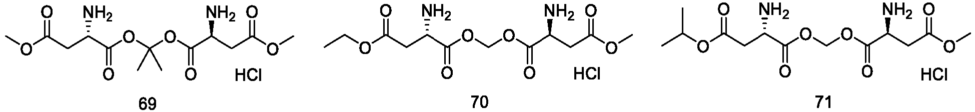

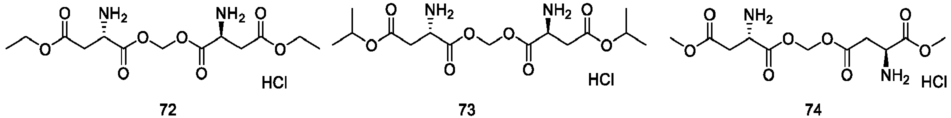

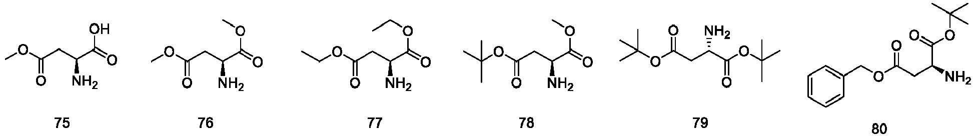

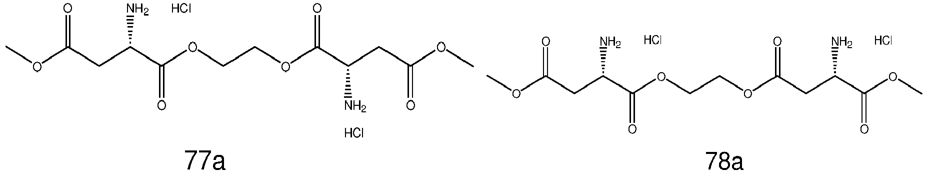

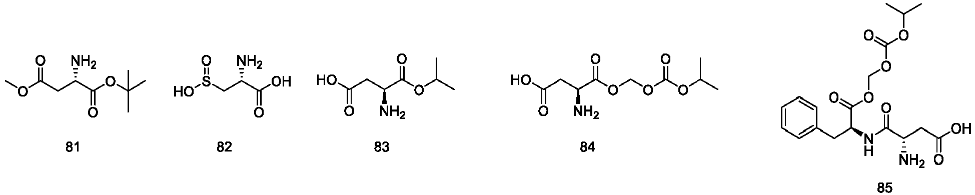

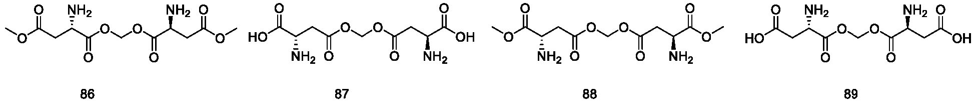

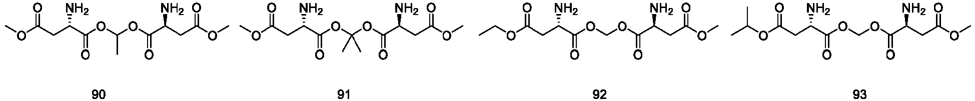

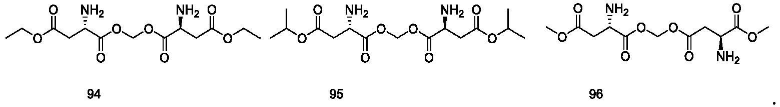

6. The use according to any one of claims 1 to 5, wherein the compound represented by Formula I or I-1, I-2, I-3, I-4 is selected from the group consisting of the following:

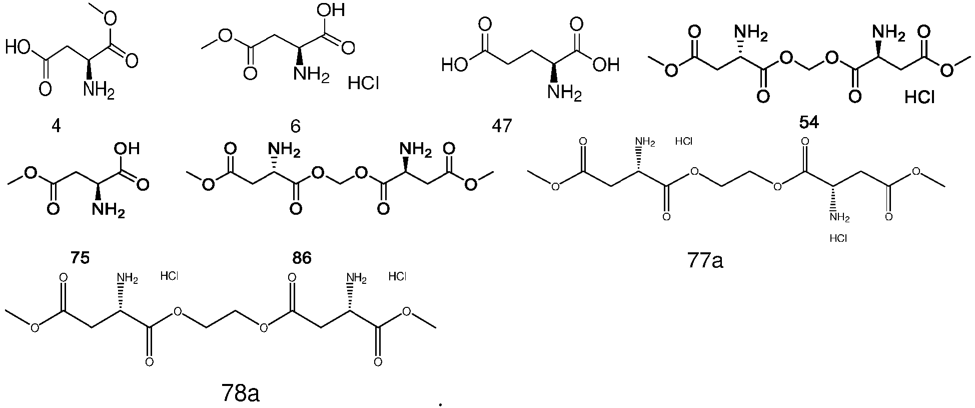

7. Use of a peptide containing 2 to 10 or 2 to 3 amino acids, or pharmaceutically acceptable salt, ester, prodrug, stereoisomer, hydrate, solvate, isotopic compound, crystalline form, metabolite form thereof, or any combination or mixture thereof, in the manufacture of a medicament or agent,
wherein the drug or agent is used for at least one of the following:
1) treating, improving, or preventing a liver disease (e.g., NAFLD, NASH, liver fibrosis);
2) alleviating a mammalian liver fibrosis, liver steatosis, or inflammation;
3) inhibiting the activation of a stellate cell;
4) regulating the expression of NS3TP1;
in the peptide, at least one amino acid is aspartic acid, and when aspartic acid has a free carboxyl group, the free carboxyl group is optionally esterified with C1-C6 alkyl-OH;
optionally, the amino acid is aspartic acid, phenylalanine, glycine, alanine, glutamic acid, cystine, glutamine, histidine, tyrosine, serine, methionine, arginine acid or leucine;
or, the free amino acid located on one side of the peptide is aspartic acid, and optionally, the free carboxyl group of the free aspartic acid is optionally esterified with C1-C6 alkyl-OH;
optionally, the remaining amino acids are aspartic acid, phenylalanine, glycine, alanine, glutamic acid, cystine, glutamine, histidine, tyrosine, serine, methionine, arginine or leucine.

8. The use according to any one of claims 1 to 7, wherein the liver fibrosis includes, but is not limited to: liver fibrosis caused by chronic viral liver diseases, liver fibrosis caused by alcoholism or long-term drinking, liver fibrosis caused by non-alcoholic factor such as obesity, portal liver fibrosis caused by repeated schistosomiasis infection, biliary liver fibrosis caused by chronic cholestasis, metabolic liver fibrosis caused by hepatolenticular degeneration and hemoglobin deposition, toxic liver fibrosis caused by various toxic substances, malnutrition-induced liver fibrosis caused by a preference for low-protein diets and fatty fried foods, and cardiogenic liver fibrosis caused by chronic congestive heart failure.

9. Use of a pharmaceutical composition in the manufacture of a medicament, the pharmaceutical composition comprising the compound or pharmaceutically acceptable salt, ester, prodrug, stereoisomer, hydrate, solvate, isotope compound, crystalline form, metabolite form thereof, or any combination or mixture thereof, as defined in any one of claims 1 to 6, or the peptide or pharmaceutically acceptable salt, ester, prodrug, stereoisomer, hydrate, solvate, isotopic compound, crystalline form, metabolite form thereof, or any combination or mixture thereof, as defined in claim 7, wherein the medicament is used for at least one of the following:
1) treating, improving, or preventing a liver disease (e.g., NAFLD, NASH, liver fibrosis);
2) alleviating a mammalian liver fibrosis, liver steatosis or inflammation;
3) inhibiting the activation of stellate cells;
4) regulating the expression of NS3TP1.

10. The use according to claim 9, wherein the liver fibrosis includes, but is not limited to: liver fibrosis caused by chronic viral liver diseases, liver fibrosis caused by alcoholism or long-term drinking, liver fibrosis caused by non-alcoholic factor such as obesity, portal liver fibrosis caused by repeated schistosomiasis infection, biliary liver fibrosis caused by chronic cholestasis, metabolic liver fibrosis caused by hepatolenticular degeneration and hemoglobin deposition, toxic liver fibrosis caused by various toxic substances, malnutrition-induced liver fibrosis caused by a preference for low-protein diets and fatty fried foods, and cardiogenic liver fibrosis caused by chronic congestive heart failure.

11. The use according to claim 9, wherein the pharmaceutical composition further comprises an additional active ingredient: other amino acids for improving liver function (including but not limited to alanine, glutamic acid, cystine, glutamine, glycine, histidine, tyrosine, serine, methionine, arginine, leucine), cholesterol absorption inhibitors (e.g., Ezetimibe), HSC activation and proliferation inhibitors (e.g., Pirfenidone, Fluorofenidone, Pegbelfermin), PCSK9 inhibitors, PPAR agonists (e.g., Gemfibrozil, Fenofibrate, Clofibrate, Bezafibrate, Pemafibrate, Elafibranor), ACE inhibitors, CCR2/5 inhibitors, TLR4 inhibition agents, LOXL2 inhibitors, TIMP-1 inhibitors, FXR agonists, AT1R blockers, NOX inhibitors, calcium channel blockers, ARBs, diuretics, renin, GLP-1 or synthetic variants thereof, insulin or synthetic variants thereof, metformin, sulfonylurea compounds, thiazolidinediones (TZD), SGLT2 inhibitors, DPP-IV inhibitors, inhibitors of HMGCoA reductase, inhibitors of proprotein convertase subtilisin/kexin type 9 (PCSK9), Gemcabene (CI-1027), ACC inhibitors, ApoC-III inhibitors, ACL-inhibitors (e.g., bepedic acid), prescription fish oil, CETP inhibitors, ursodeoxycholic acid, obeticholic acid, polyene phosphatidylcholine, glucocorticoids, silymarin, glycyrrhizic acid preparations (e.g., magnesium isoglycyrrhizinate injection and diammonium glycyrrhizinate enteric-coated capsules), and combinations thereof;
and further comprises a pharmaceutically acceptable carrier or excipient;
or, the pharmaceutical composition is a solid preparation, an injection, a topical preparation, a spray, a liquid preparation or a compound preparation.

12. A compound represented by Formula II, or pharmaceutically acceptable salt or ester, prodrug, stereoisomer, hydrate, solvate, crystalline form, and metabolism form thereof,
A₁-(L₁)ₓ-A₁' Formula II
wherein, A₁ is 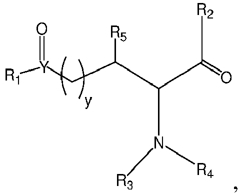 A₁' is 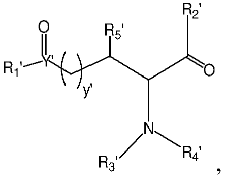 A₁ and A₁' are the same or different;
x is selected from the group consisting of 0, 1 and 2;
1) when x is 0, A' is absent; for A₁, wherein:
R₁ and R₂ are independently selected from the group consisting of: 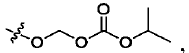 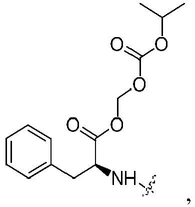 and -O-R^{a},
R^{a} is each independently selected from the group consisting of: hydrogen, deuterium, C1-C6 alkyl, C2-C6 alkenyl, C2-C6 alkynyl, C3-C7 cycloalkyl, C6-C10 aryl, 3- to 15-membered heterocyclyl, -(C1-C6 alkyl)ₙ-(CO)-R^{b1}, -(C1-C6 alkyl)ₙ-(CO)-O-R^{b2}, -(C1-C6 alkyl)ₙ-O-(CO)-R^{b3}, -(C1-C6 alkyl)ₙ-NR^{b4}-(CO)-R^{b5}, and -(C1-C6 alkyl)ₙ-NR^{b6}-(CO)-O-R^{b7}, the alkyl, alkenyl, alkynyl, cycloalkyl, aryl, and heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
R^{b1}, R^{b2}, R^{b3}, R^{b4}, R^{b5}, R^{b6}, R^{b7} are each independently selected from the group consisting of: hydrogen, deuterium, hydroxyl, amino, C1-C6 alkyl, C2-C6 alkenyl, C2-C6 alkynyl, C3-C7 cycloalkyl, C6-C10 aryl, and 3- to 15-membered heterocyclyl;
R₃ and R₄ are independently selected from the group consisting of: -COCH(CH₃)(NH₂), hydrogen, -COCH₂CH₃, deuterium, and C1-C6 alkyl, the alkyl is optionally substituted with one or more groups independently selected from the group consisting of halogen, C1-C6 alkyl, halogenated C1-C6 alkyl, aryl, and heterocyclyl;
and for "x is 0, A' is absent", A₁ meets one of the following conditions, (1), (2), (3) or (4):
(1) at least one of R₁ and R₂ is 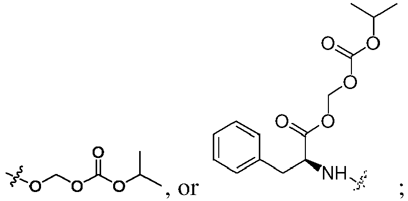
(2) at least one of R₃ and R₄ is -COCH(CH₃)(NH₂) or -COCH₂CH₃;
(3) when the situation is: R₁ and R₂ are independently selected from the group consisting of: 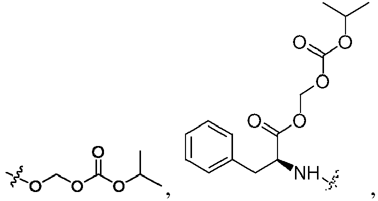 hydroxyl, methoxy, and at the same time, R₃ and R₄ are independently selected from the group consisting of: hydrogen, -COCH₂CH₃ and -COCH(CH₃)(NH₂),
A₁ meets the following conditions:
when one of R₁ and R₂ is hydroxyl or methoxy, the other of R₁ and R₂ is 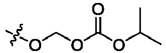 or 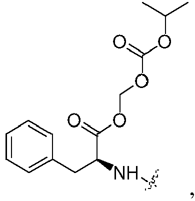 or one of R₃ and R₄ is -COCH(CH₃)(NH₂);
(4) when the situation is: R₁ is selected from the group consisting of: methoxy, 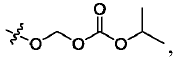 hydroxyl,
R₂ is selected from the group consisting of: 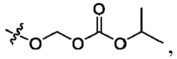 hydroxyl, 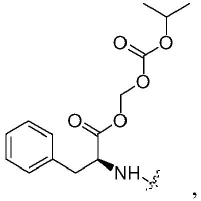 methoxy,
and at the same time, R₃ and R₄ are independently selected from the group consisting of: hydrogen, -COCH₂CH₃ and -COCH(CH₃)(NH₂),
A₁ meets the following conditions:
when R₁ is hydroxyl or methoxy, R₂ is 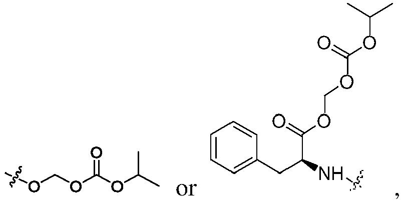 or one of R₃ and R₄ is -COCH(CH₃)(NH₂), or
when R₂ is hydroxyl or methoxy, R₁ is 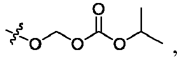 or one of R₃ and R₄ is -COCH(CH₃)(NH₂);
2) when x is 1, A₁ and A₁' are the same or different, wherein
any one of R₁, R₂, R₃, and R₄ of a unit A₁ is connected to any one of R₁', R₂', R₃' and R₄' of an adjacent unit A₁' through L₁;
each Li is independently a unit-linking group selected from the group consisting of: absence, O, S, carbonyl, alkyl (or C1-C6 alkyl), alkenyl (or C2-C6 alkenyl), alkynyl (or C2-C6 alkynyl), -O-alkyl-O- (or -O-C1-C6 alkyl-O-), -O-alkenyl-O- (or -O-C2-C6 alkenyl-O-), -O-alkynyl-O-(or -O-C2-C6 alkynyl-O-), cycloalkyl (or C3-C7 cycloalkyl), and heteroalkyl (or 3- to 7-membered heteroalkyl), wherein the alkyl, cycloalkyl, heteroalkyl are optionally substituted with one or more groups independently selected from the group consisting of Z;
Z is independently selected from the group consisting of: halogen, amino, alkyl (or C1-C6 alkyl), alkenyl (or C2-C6 alkenyl), alkynyl (or C2-C6 alkynyl), haloalkyl (or halogenated C1-C6 alkyl), cycloalkyl (or C3-C7 cycloalkyl), aryl (or C6-C12 aryl), and heterocyclyl;
or, Li is selected from the group consisting of: absence and -CR'R", wherein R' and R" are each independently H, C1-C6 alkyl (or C1-C3 alkyl);
or, Li is selected from the group consisting of: absence, -CH₂-, -CH(CH₃)-, and -C-(CH₃)₂;
for A₁, wherein:
R₁ and R₂ are each independently selected from the group consisting of: C1-C6 alkyl, -O-, -O-R^{a1}, and -NR^{a2}R^{a3};
R₃ and R₄ are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), and heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, and 3-to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁ is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl,
C6-C10 aryl, 3- to 15-membered heterocyclyl, and -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl,
C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁ is selected from the group consisting of: -O-, hydroxyl, and -O-R^{a}: R^{a} is C1-C6 alkyl;
or, R₁ is selected from the group consisting of: -O-, hydroxyl, methoxy, ethoxy, and -OCH(CH₃)₂;
or, R₂ is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl,
C6-C10 aryl, 3- to 15-membered heterocyclyl, and -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl,
C6-C10 aryl, 3- to 15-membered heterocyclyl, -(C1-C6 alkyl)-(C6-C10 aryl), and -(C1-C6 alkyl)-(3- to 15-membered heterocyclyl);
or, R₂ is selected from the group consisting of: hydroxyl, -O-, and -O-R^{a}: R^{a} is C1-C6 alkyl;
or, R₂ is selected from the group consisting of: hydroxyl, -O-, and methoxy,
R₃ and R₄ are each independently selected from the group consisting of: hydrogen, hydroxyl, and C1-C6 alkyl;
or, R₃ and R₄ are each independently hydrogen;
R₅ is selected from the group consisting of: hydrogen, deuterium, C1-C6 alkyl, halogen, and amino;
or, R₅ is selected from the group consisting of hydrogen and amino;
or, R₅ is selected from the group consisting of hydrogen;
Y is C;
y is selected from the group consisting of 0, 1, 2, and 3; or y is 0 or 1; or y is 0;
for A₁', wherein:
R₁' and R₂' are each independently selected from the group consisting of: C1-C6 alkyl, -O-, -O-R^{a1}, and -NR^{a2}R^{a3};
R₃' and R₄' are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl) and heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, and 3-to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁' is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, and -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁' is selected from the group consisting of: -O-, hydroxyl, and -O-R^{a}: R^{a} is C1-C6 alkyl;
or, R₁' is selected from the group consisting of: -O-, hydroxyl, methoxy, ethoxy, and -OCH(CH₃)₂;
or, R₂' is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, and -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₂' is selected from the group consisting of: hydroxyl, -O-, and -O-R^{a}: R^{a} is C1-C6 alkyl;
or, R₂' is selected from the group consisting of: hydroxyl, -O-, and methoxy;
R₃' and R₄' are each independently selected from the group consisting of: hydrogen, hydroxyl, and C1-C6 alkyl;
or, R₃' and R₄' are each independently hydrogen;
R₅' is selected from the group consisting of: hydrogen, deuterium, C1-C6 alkyl, halogen, and amino;
or, R₅' is selected from the group consisting of hydrogen and amino;
or, R₅' is selected from the group consisting of hydrogen;
Y' is C;
y' is selected from the group consisting of 0, 1, 2, and 3; or y is 0 or 1; or y' is 0;
3) when x is 2, A₁ and A₁' are the same or different, wherein
any one of R₁, R₂, R₃ and R₄ of a unit A₁ is connected to any one of R₁', R₂', R₃' and R₄' of an adjacent unit A₁' through L₁;
each L₁ is independently a unit-linking group selected from the group consisting of: absence, O, S, carbonyl, alkyl (or C1-C6 alkyl), alkenyl (or C2-C6 alkenyl), alkynyl (or C2-C6 alkynyl), -O-alkyl-O- (or -O-C1-C6 alkyl-O-), -O-alkenyl-O- (or -O-C2-C6 alkenyl-O-), -O-alkynyl-O-(or -O-C2-C6 alkynyl-O-), cycloalkyl (or C3-C7 cycloalkyl), and heteroalkyl (or 3- to 7-membered heteroalkyl), wherein the alkyl, cycloalkyl, heteroalkyl are optionally substituted with one or more groups independently selected from the group consisting of Z;
Z is independently selected from the group consisting of: halogen, amino, alkyl (or C1-C6 alkyl), alkenyl (or C2-C6 alkenyl), alkynyl (or C2-C6 alkynyl), haloalkyl (or halogenated C1-C6 alkyl), cycloalkyl (or C3-C7 cycloalkyl), aryl (or C6-C12 aryl), and heterocyclyl;
or, Li is selected from the group consisting of: absence and -CR'R", wherein R' and R" are each independently H, C1-C6 alkyl (or C1-C3 alkyl);
or, Li is selected from the group consisting of: absence, -CH₂-, -CH(CH₃)-, and -C-(CH₃)₂;
or, Li is selected from the group consisting of: -CH₂-;
for A₁, wherein:
R₁ and R₂ are each independently selected from the group consisting of: C1-C6 alkyl, -O-, -O-R^{a1}, and -NR^{a2}R^{a3};
R₃ and R₄ are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl), and heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, and 3-to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁ is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl,
C6-C10 aryl, 3- to 15-membered heterocyclyl, and -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl,
C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁ is selected from the group consisting of: -O-, hydroxyl, and -O-R^{a}; R^{a} is C1-C6 alkyl;
or, R₁ is selected from the group consisting of: -O-, hydroxyl, methoxy, ethoxy, and -OCH(CH₃)₂;
or, R₁ is selected from the group consisting of: methoxy;
or, R₂ is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, and -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₂ is selected from the group consisting of: hydroxyl, -O-, and -O-R^{a}; R^{a} is C1-C6 alkyl;
or, R₂ is selected from the group consisting of: hydroxyl, -O-, and methoxy,
or, R₂ is selected from the group consisting of: -O-;
R₃ and R₄ are each independently selected from the group consisting of: hydrogen, hydroxyl, and C1-C6 alkyl;
or, R₃ and R₄ are each independently hydrogen;
R₅ is selected from the group consisting of: hydrogen, deuterium, C1-C6 alkyl, halogen, and amino;
or, R₅ is selected from the group consisting of hydrogen and amino;
or, R₅ is selected from the group consisting of hydrogen;
Y is C;
y is selected from the group consisting of 0, 1, 2, and 3; or y is 0 or 1; or y is 0;
for A₁', wherein:
R₁' and R₂' are each independently selected from the group consisting of: C1-C6 alkyl, -O-, -O-R^{a1}, and -NR^{a2}R^{a3};
R₃' and R₄' are each independently selected from the group consisting of: hydrogen, deuterium, hydroxyl, C1-C6 alkyl, C2-C6 alkenyl, C2-C6 alkynyl, C3-C7 cycloalkyl, C6-C10 aryl, and 3- to 15-membered heterocyclyl, the alkyl, alkenyl, alkynyl, cycloalkyl, aryl, heterocyclyl are optionally substituted with one or more groups independently selected from the group consisting of halogen, C1-C6 alkyl, halogenated C1-C6 alkyl, aryl (or C6-C12 aryl) and heterocyclyl;
R^{a1}, R^{a2}, and R^{a3} are each independently selected from the group consisting of: hydrogen, deuterium, C1-C6 alkyl, C2-C6 alkenyl, C2-C6 alkynyl, C3-C7 cycloalkyl, C6-C10 aryl, and 3-to 15-membered heterocyclyl, the alkyl, alkenyl, alkynyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁' is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, and -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₁' is selected from the group consisting of: -O-, hydroxyl, and -O-R^{a}: R^{a} is C1-C6 alkyl;
or, R₁' is selected from the group consisting of: -O-, hydroxyl, methoxy, ethoxy, and -OCH(CH₃)₂;
or, R₁' is selected from the group consisting of: methoxy;
or, R₂' is selected from the group consisting of: -O-, C1-C6 alkyl, and -O-R^{a1}, wherein R^{a1} is selected from the group consisting of: hydrogen, C1-C6 alkyl, C2-C6 alkenyl, C3-C7 cycloalkyl, C6-C10 aryl, 3- to 15-membered heterocyclyl, and -(C1-C6 alkyl)ₙ-O-(CO)-R^{b1}, the alkyl, alkenyl, cycloalkyl, aryl, heterocyclyl are optionally substituted with one or more groups independently selected from the group consisting of halogen, amino, hydroxyl, C1-C6 alkyl, C6-C10 aryl, 3- to 15-membered heterocyclyl, -(C1-C6 alkyl)-(C6-C10 aryl), and -(C1-C6 alkyl)-(3- to 15-membered heterocyclyl);
or, R₂' is selected from the group consisting of: hydroxyl, -O-, and -O-R^{a}: R^{a} is C1-C6 alkyl;
or, R₂' is selected from the group consisting of: hydroxyl, -O-, and methoxy;
or, R₂' is selected from the group consisting of: -O-;
R₃' and R₄' are each independently selected from the group consisting of: hydrogen, hydroxyl, and C1-C6 alkyl;
or, R₃' and R₄' are each independently hydrogen;
R₅' is selected from the group consisting of: hydrogen, deuterium, C1-C6 alkyl, halogen, and amino;
or, R₅' is selected from the group consisting of hydrogen and amino;
or, R₅' is selected from the group consisting of hydrogen;
Y' is C;
y' is selected from the group consisting of 0, 1, 2, and 3; or y is 0 or 1; or y' is 0;
or, Formula II is:

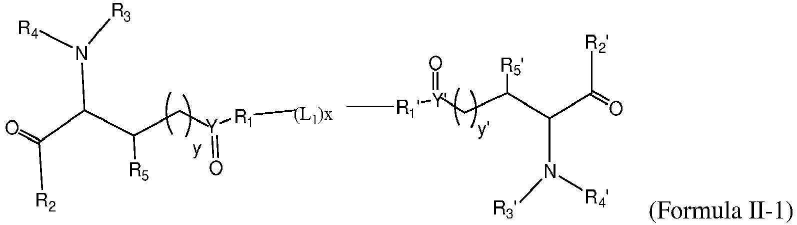

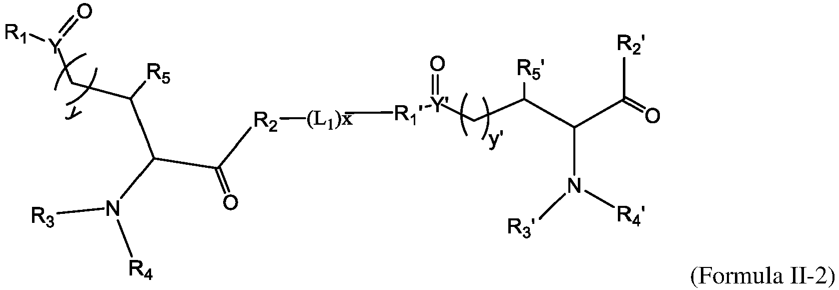

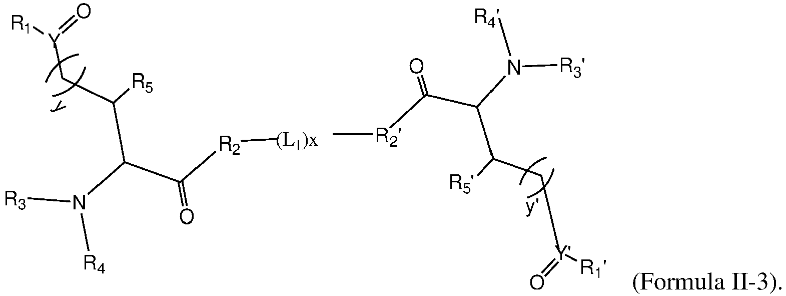

13. The compound of claim 12, wherein the compound represented by Formula II or II-1, II-2, II-3 is selected from the group consisting of the following:

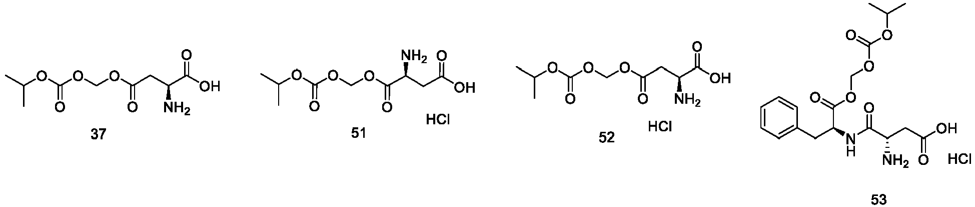

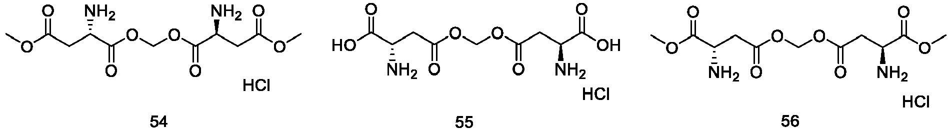

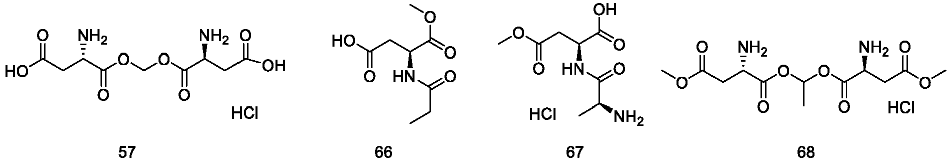

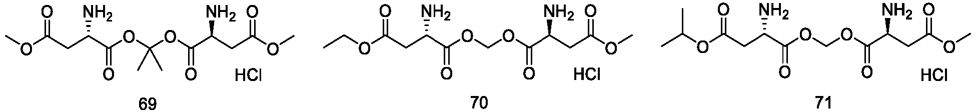

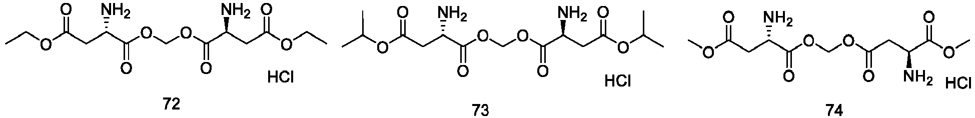

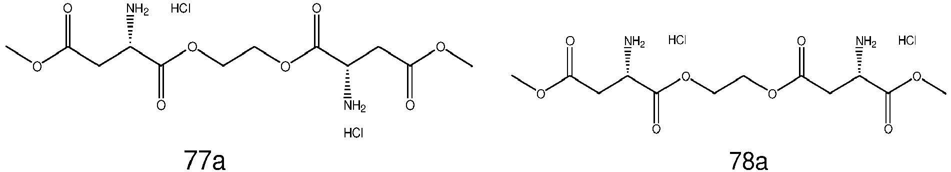

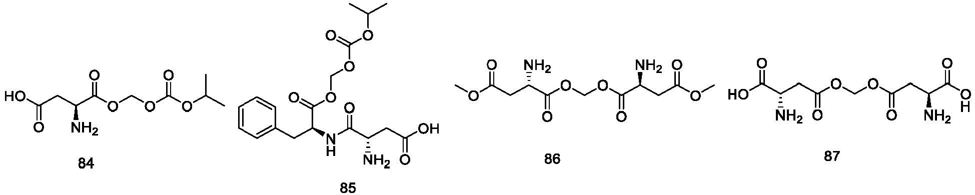

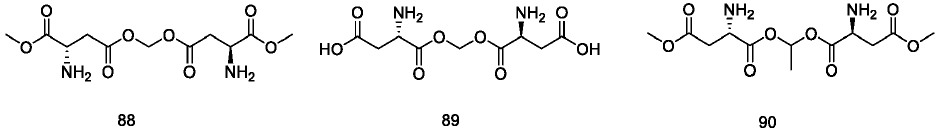

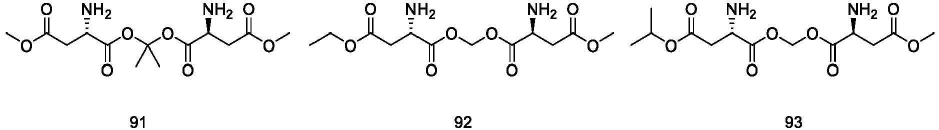

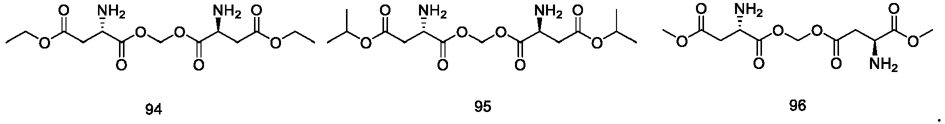

14. A pharmaceutical composition, which comprises the compound represented by Formula II, or pharmaceutically acceptable salt or ester, prodrug, stereoisomer, hydrate, solvate, isotopic compound, crystalline form, metabolite form thereof, or any combination or mixture thereof, according to any one of claims 12 to 13,
optionally, the pharmaceutical composition further comprises a pharmaceutically acceptable carrier or excipient;
optionally, the pharmaceutical composition further comprises an additional active ingredient: other amino acids for improving liver function (including but not limited to alanine, glutamic acid, cystine, glutamine, glycine, histidine, tyrosine, serine, methionine, arginine, leucine), cholesterol absorption inhibitors (e.g., Ezetimibe), HSC activation and proliferation inhibitors (e.g., Pirfenidone, Fluorofenidone, Pegbelfermin), PCSK9 inhibitors, PPAR agonists (e.g., Gemfibrozil, Fenofibrate, Clofibrate, Bezafibrate, Pemafibrate, Elafibranor), ACE inhibitors, CCR2/5 inhibitors, TLR4 inhibition agents, LOXL2 inhibitors, TIMP-1 inhibitors, FXR agonists, AT1R blockers, NOX inhibitors, calcium channel blockers, ARBs, diuretics, renin, GLP-1 or synthetic variants thereof, insulin or synthetic variants thereof, metformin, sulfonylurea compounds, thiazolidinediones (TZD), SGLT2 inhibitors, DPP-IV inhibitors, inhibitors of HMGCoA reductase, inhibitors of proprotein convertase subtilisin/kexin type 9 (PCSK9), Gemcabene (CI-1027), ACC inhibitors, ApoC-III inhibitors, ACL-inhibitors (e.g., bepedic acid), prescription fish oil, CETP inhibitors, ursodeoxycholic acid, obeticholic acid, polyene phosphatidylcholine, glucocorticoids, silymarin, glycyrrhizic acid preparations (e.g., magnesium isoglycyrrhizinate injection and diammonium glycyrrhizinate enteric-coated capsules), and combinations thereof;
or, the pharmaceutical composition is a solid preparation, an injection, a topical preparation, a spray, a liquid preparation or a compound preparation.
